(19) Europäisches Patentamt / European Patent Office / Office européen des brevets

(11) EP 4 691 497 A1

(12) EUROPEAN PATENT APPLICATION
published in accordance with Art. 153(4) EPC

(43) Date of publication:
**11.02.2026 Bulletin 2026/07**

(21) Application number: **24780774.6**

(22) Date of filing: **29.03.2024**

(51) International Patent Classification (IPC):
***A61K 47/68*** (2017.01) ***A61K 31/277*** (2006.01)
***A61K 31/4745*** (2006.01) ***A61K 39/395*** (2006.01)
***A61K 45/00*** (2006.01) ***A61P 1/00*** (2006.01)
***A61P 1/16*** (2006.01) ***A61P 1/18*** (2006.01)
***A61P 5/14*** (2006.01) ***A61P 11/00*** (2006.01)
***A61P 13/08*** (2006.01) ***A61P 13/12*** (2006.01)
***A61P 15/00*** (2006.01) ***A61P 17/00*** (2006.01)
***A61P 19/08*** (2006.01) ***A61P 25/00*** (2006.01)
***A61P 35/00*** (2006.01) ***C07K 1/107*** (2006.01)
***C07K 7/06*** (2006.01) ***C07K 16/28*** (2006.01)
***C12N 15/13*** (2006.01)

(52) Cooperative Patent Classification (CPC):
**A61K 31/277; A61K 31/4745; A61K 39/395; A61K 45/00; A61K 47/68; A61P 1/00; A61P 1/16; A61P 1/18; A61P 5/14; A61P 11/00; A61P 13/08; A61P 13/12; A61P 15/00; A61P 17/00; A61P 19/08;**
(Cont.)

(86) International application number:
**PCT/JP2024/012972**

(87) International publication number:
**WO 2024/204679 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.03.2023 JP 2023058171**

(71) Applicant: **Daiichi Sankyo Company, Limited**
**Tokyo 103-8426 (JP)**

(72) Inventor: **SUZUKI Hirokazu**
**Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

# (54) COMBINATION OF ANTI-CDH6 ANTIBODY-DRUG CONJUGATE AND HIF-2ALPHA INHIBITOR

(57) The present invention provides a pharmaceutical composition and a method of treatment, wherein a specific anti-CDH6 antibody-drug conjugate and an HIF-2α inhibitor are administered in combination. The present invention provides a pharmaceutical composition and a method of treatment, wherein an antibody-drug conjugate and an HIF-2α inhibitor are administered in combination, the antibody-drug conjugate being an antibody-drug conjugate in which a drug linker represented by the following formula (wherein A represents a connecting position to an anti-CDH6 antibody or a functional fragment of the antibody) and an anti-CDH6 antibody or a functional fragment of the antibody are conjugated via a thioether bond.

EP 4 691 497 A1

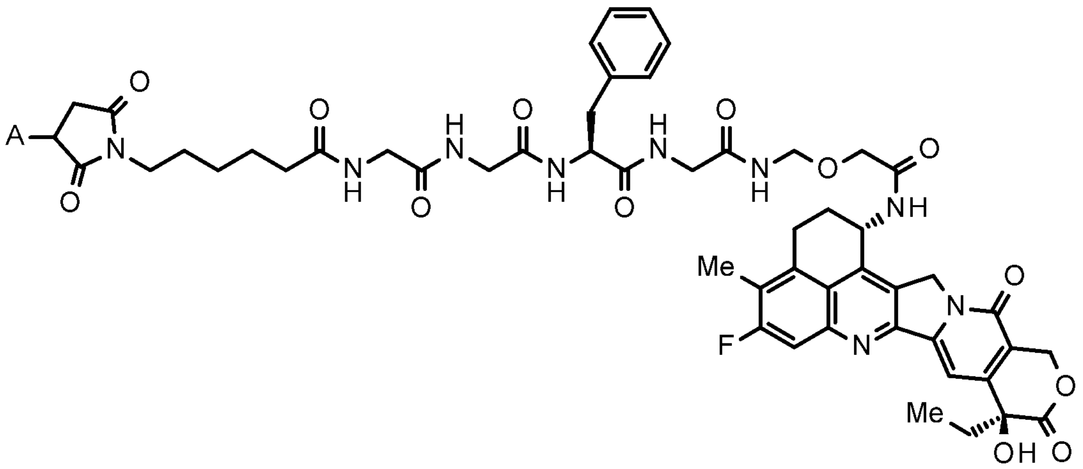

(52) Cooperative Patent Classification (CPC): (Cont.)
**A61P 25/00; A61P 35/00; C07K 1/107; C07K 7/06; C07K 16/00; C07K 16/28**

## Description

[Technical Field]

The present invention relates to a pharmaceutical composition wherein a specific anti-CDH6 antibody-drug conjugate and a HIF-2α inhibitor are administered in combination, and/or a method of treatment comprising administering a specific anti-CDH6 antibody-drug conjugate and a HIF-2α inhibitor in combination to a subject.

[Background Art]

The intratumoral hypoxic environment promotes the progression of cancer, and is closely related to poor prognosis of patients and resistance to chemotherapy. Hypoxia-inducible factor 2α (HIF-2α) is a transcriptional factor that plays a central role in the hypoxic response pathway, and controls the expression of genes that regulate angiogenesis, cell growth, cell survival, extracellular matrix modeling, pH homeostasis, and the like (Non Patent Literature 1).

HIF-2α inhibitors are known as, for example, agents that exhibit an antitumor effect by inhibiting the transcriptional activity of HIF-2α. For example, belzutifan, PT2385, and PT2399 are known HIF-2α inhibitors (Non Patent Literatures 2 and 3).

An antibody-drug conjugate (ADC), in which an antibody that binds to an antigen expressed on the surface of cancer cells and can internalize the antigen into the cell is conjugated to a drug having cytotoxic activity, can selectively deliver the drug to cancer cells, and can thereby kill the cancer cells by accumulating the drug in the cancer cells. An antibody-drug conjugate comprising an anti-CDH6 antibody and a topoisomerase I inhibitor exatecan derivative as components is a known antibody-drug conjugate (Patent Literature 1).

However, effects have not been known when an anti-CDH6 drug conjugate (including the anti-CDH6 antibody-drug conjugate described above) and a HIF-2α inhibitor are used in combination or when an anti-CDH6 antibody and a HIF-2α inhibitor are used in combination.

[Citation List]

[Patent Literature]

[Patent Literature 1] International Publication No. WO 2018/212136

[Non Patent Literature]

[Non Patent Literature 1] Keith et al., Nat. Rev. Cancer. (2011) 12(1): 9-22.
[Non Patent Literature 2] Martinez-Saez et al. Crit Rev Oncol Hematol. (2017) 111: 117-123.
[Non Patent Literature 3] Xu et al., J. Med. Chem. (2019) 62(15): 6876-6893.

[Summary of Invention]

[Technical Problem]

The anti-CDH6 antibody-drug conjugate (anti-CDH6 antibody-drug conjugate comprising an exatecan derivative as a component) used in the present invention has been confirmed to exhibit an excellent antitumor effect even when used alone. However, there has been a need to obtain a method of treatment which can suppress growth of cancer cells in multiple manners and exert an even more superior antitumor effect by using the antibody-drug conjugate in combination with another anticancer agent having a different mechanism of action.

An object of the present invention is to provide a pharmaceutical composition wherein a specific anti-CDH6 antibody-drug conjugate and a HIF-2α inhibitor are administered in combination, and/or a method of treatment comprising administering a specific anti-CDH6 antibody-drug conjugate and a HIF-2α inhibitor in combination to a subject.

[Solution to Problem]

The present inventors have conducted intensive studies directed towards achieving the above-described object, and consequently completed the present invention by finding that a specific anti-CDH6 antibody-drug conjugate and a HIF-2α inhibitor administered in combination exhibit an excellent combinatorial effect (a highly safe and marked antitumor

effect).

Thus, the present invention provides the following [1] to [221].

[1] A pharmaceutical composition comprising an anti-CDH6 antibody-drug conjugate, wherein the anti-CDH6 antibody-drug conjugate and a HIF-2α inhibitor are administered in combination, and
the anti-CDH6 antibody-drug conjugate is an antibody-drug conjugate in which a drug linker represented by the formula:

[Formula 1]

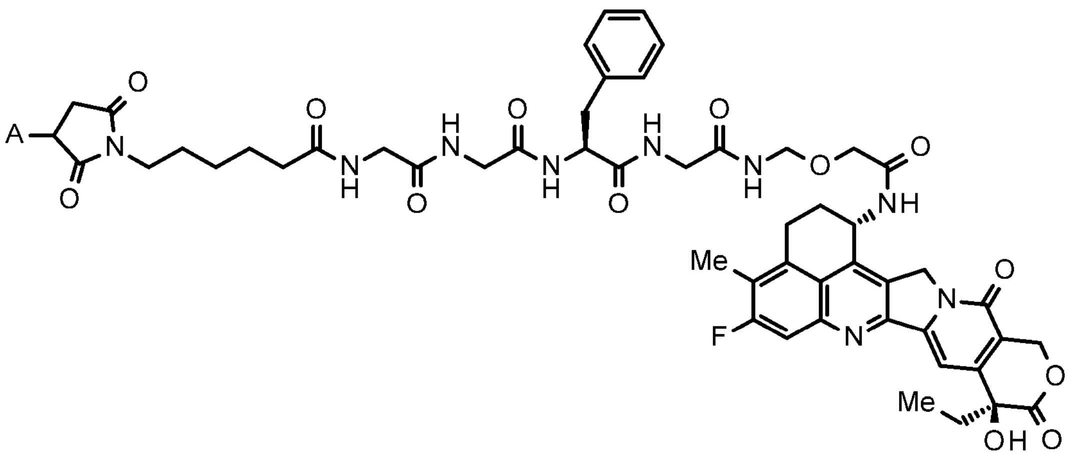

wherein A represents a connecting position to an anti-CDH6 antibody or a functional fragment of the antibody, is conjugated to the anti-CDH6 antibody or the functional fragment of the antibody via a thioether bond.

[2] The pharmaceutical composition according [1], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.
[3] The pharmaceutical composition according to [1] or [2], wherein the anti-CDH6 antibody or the functional fragment of the antibody is an antibody comprising

CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23; and

CDRL1, CDRL2 and CDRL3 in any one combination selected from the group consisting of the following combinations (6) to (9):

(6) CDRL1 consisting of the amino acid sequence shown in SEQ ID NO: 5, CDRL2 consisting of the amino

acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[4] The pharmaceutical composition according to any one of [1] to [3], wherein the anti-CDH6 antibody or the functional fragment of the antibody is an antibody comprising CDRH1, CDRH2 and CDRH3, and CDRL1, CDRL2 and CDRL3, in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid sequence shown in SEQ ID NO: 8, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, CDRH3 consisting of the amino acid sequence shown in SEQ ID NO: 11, CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, CDRH3 consisting of the amino acid sequence shown in SEQ ID NO: 17, CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid sequence shown in SEQ ID NO: 22, CDRH3 consisting of the amino acid sequence shown in SEQ ID NO: 23, CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.
[5] The pharmaceutical composition according to any one of [1] to [4], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.
[6] The pharmaceutical composition according to any one of [1] to [5], wherein the anti-CDH6 antibody or the functional fragment of the antibody is a humanized antibody or functional fragment of the antibody.
[7] The pharmaceutical composition according to any one of [1] to [6], wherein the anti-CDH6 antibody or the functional fragment of the antibody is an antibody comprising

any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the

framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4); and

any one light chain variable region selected from the group consisting of the following variable regions (6) to (9):

(6) a light chain variable region consisting of the amino acid sequence shown in SEQ ID NO: 37,
(7) a light chain variable region consisting of the amino acid sequence shown in SEQ ID NO: 40,
(8) an amino acid sequence having a sequence identity of at least 95% or more to the sequence of the framework regions other than at each CDR sequence in the amino acid sequences of any one of (6) and (7), and
(9) an amino acid sequence comprising a deletion, substitution or addition of one or several amino acids in the sequence of the framework regions other than at each CDR sequence in the amino acid sequences of any one of (6) to (8),

or a functional fragment of the antibody.

[8] The pharmaceutical composition according to any one of [1] to [7], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,
(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.
[9] The pharmaceutical composition according to any one of [1] to [8], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.
[10] The pharmaceutical composition according to any one of [1] to [9], wherein the anti-CDH6 antibody or the functional fragment of the antibody is an antibody comprising any of the following combinations (1) to (4):

(1) a heavy chain consisting of the amino acid sequence shown in SEQ ID NO: 29 and a light chain consisting of the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38,

or a functional fragment of the antibody.
[11] The pharmaceutical composition according to any one of [1] to [10], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.
[12] The pharmaceutical composition according to any one of [1] to [11], wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal

glutamic acid to pyroglutamic acid, and a deletion of one or two amino acids from the carboxyl terminus, or a functional fragment of the antibody.

[13] The pharmaceutical composition according to [12], wherein the anti-CDH6 antibody or the functional fragment of the antibody is an antibody in which one or two amino acids are deleted from the carboxyl terminus of a heavy chain thereof, or a functional fragment of the antibody.

[14] The pharmaceutical composition according to [13], wherein the anti-CDH6 antibody or the functional fragment of the antibody is an antibody in which one amino acid is deleted from the carboxyl terminus of each of the two heavy chains thereof, or a functional fragment of the antibody.

[15] The pharmaceutical composition according to any one of [12] to [14], wherein the deleted amino acid is lysine.

[16] The pharmaceutical composition according to any one of [12] to [15], wherein the anti-CDH6 antibody or the functional fragment of the antibody is an antibody in which a proline residue at the carboxyl terminus of a heavy chain thereof is further amidated, or a functional fragment of the antibody.

[17] The pharmaceutical composition according to any one of [1] to [16], wherein the average number of units of the drug-linker conjugated per antibody in the anti-CDH6 antibody-drug conjugate is in the range of from 1 to 10.

[18] The pharmaceutical composition according to any one of [1] to [16], wherein the average number of units of the drug-linker conjugated per antibody in the anti-CDH6 antibody-drug conjugate is in the range of from 2 to 8.

[19] The pharmaceutical composition according to any one of [1] to [16], wherein the average number of units of the drug-linker conjugated per antibody in the anti-CDH6 antibody-drug conjugate is in the range of from 5 to 8.

[20] The pharmaceutical composition according to any one of [1] to [16], wherein the average number of units of the drug-linker conjugated per antibody in the anti-CDH6 antibody-drug conjugate is in the range of from 7 to 8.

[21] The pharmaceutical composition according to any one of [1] to [16], wherein the average number of units of the drug-linker conjugated per antibody in the anti-CDH6 antibody-drug conjugate is in the range of from 7.5 to 8.

[22] The pharmaceutical composition according to any one of [1] to [16], wherein the average number of units of the drug-linker conjugated per antibody in the anti-CDH6 antibody-drug conjugate is approximately 8.

[22-2] The pharmaceutical composition according to any one of [1] to [16], wherein the number of the drug-linker structures conjugated per antibody in the anti-CDH6 antibody-drug conjugate is an integer in the range of from 1 to 10.

[22-3] The pharmaceutical composition according to any one of [1] to [16], wherein the number of the drug-linker structures conjugated per antibody in the anti-CDH6 antibody-drug conjugate is an integer in the range of from 2 to 8.

[22-4] The pharmaceutical composition according to any one of [1] to [16], wherein the number of the drug-linker structures conjugated per antibody in the anti-CDH6 antibody-drug conjugate is 2, 4, 6 or 8.

[22-5] The pharmaceutical composition according to any one of [1] to [16], wherein the number of the drug-linker structures conjugated per antibody in the anti-CDH6 antibody-drug conjugate is 8.

[23] The pharmaceutical composition according to any one of [1] to [22-5], wherein the HIF-2$\alpha$ inhibitor is belzutifan, NKT2152, DFF332, AB521, or BPI-452080, or a pharmaceutically acceptable salt thereof.

[24] The pharmaceutical composition according to any one of [1] to [22-5], wherein the HIF-2$\alpha$ inhibitor is belzutifan or a pharmaceutically acceptable salt thereof.

[25] The pharmaceutical composition according to any one of [1] to [24], wherein the anti-CDH6 antibody-drug conjugate and the HIF-2$\alpha$ inhibitor are separately contained (as active components) in different formulations, and are administered at the same time (at approximately the same time) or at different times.

[26] The pharmaceutical composition according to any one of [1] to [24], wherein the anti-CDH6 antibody-drug conjugate and the HIF-2$\alpha$ inhibitor are contained (as active components) in a single formulation for administration.

[27] The pharmaceutical composition according to any one of [1] to [26], wherein the pharmaceutical composition is for use in treating cancer.

[28] The pharmaceutical composition according to any one of [1] to [26], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

[29] The pharmaceutical composition according to any one of [1] to [26], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[30] The pharmaceutical composition according to any one of [1] to [26], wherein the pharmaceutical composition is for

use in treating at least one selected from the group consisting of renal cell carcinoma, renal clear cell carcinoma, and papillary renal cell carcinoma.

[31] A pharmaceutical composition comprising an anti-CDH6 antibody-drug conjugate, wherein the anti-CDH6 antibody-drug conjugate and a HIF-2α inhibitor are administered in combination, and

the anti-CDH6 antibody-drug conjugate is an antibody-drug conjugate represented by the formula:

[Formula 2]

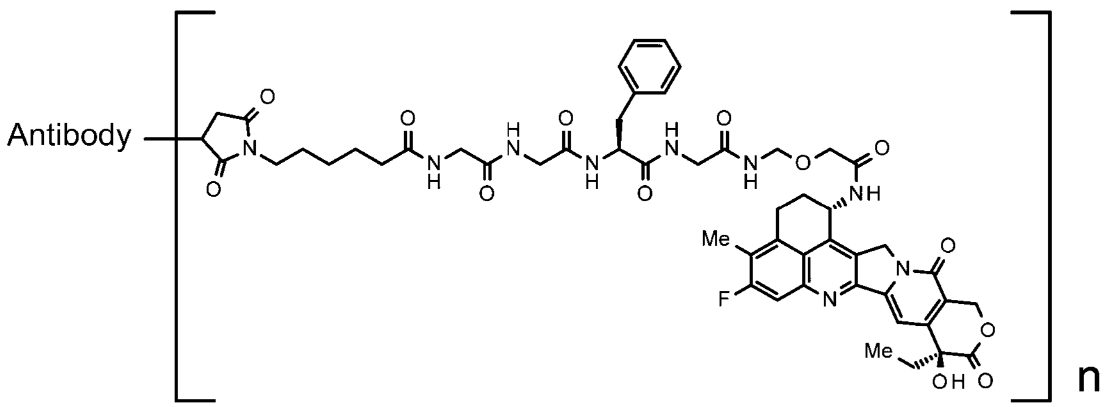

wherein Antibody is an anti-CDH6 antibody, a drug-linker is conjugated to the antibody via a thioether bond, and n represents an average number of units of the drug-linker conjugated per antibody.

[32] The pharmaceutical composition according to [31], wherein the anti-CDH6 antibody is an antibody comprising a heavy chain consisting of the amino acid sequence shown in SEQ ID NO: 29 and a light chain consisting of the amino acid sequence shown in SEQ ID NO: 38.

[33] The pharmaceutical composition according to [31] or [32], wherein a lysine residue is deleted from the carboxyl terminus of a heavy chain of the anti-CDH6 antibody.

[34] The pharmaceutical composition according to any one of [31] to [33], wherein the average number of units of the drug-linker conjugated per antibody in the anti-CDH6 antibody-drug conjugate is in the range of from 1 to 10.

[35] The pharmaceutical composition according to any one of [31] to [33], wherein the average number of units of the drug-linker conjugated per antibody in the anti-CDH6 antibody-drug conjugate is in the range of from 2 to 8.

[36] The pharmaceutical composition according to any one of [31] to [33], wherein the average number of units of the drug-linker conjugated per antibody in the anti-CDH6 antibody-drug conjugate is in the range of from 5 to 8.

[37] The pharmaceutical composition according to any one of [31] to [33], wherein the average number of units of the drug-linker conjugated per antibody in the anti-CDH6 antibody-drug conjugate is in the range of from 7 to 8.

[38] The pharmaceutical composition according to any one of [31] to [33], wherein the average number of units of the drug-linker conjugated per antibody in the anti-CDH6 antibody-drug conjugate is in the range of from 7.5 to 8.

[39] The pharmaceutical composition according to any one of [31] to [33], wherein the average number of units of the drug-linker conjugated per antibody in the anti-CDH6 antibody-drug conjugate is approximately 8.

[40] The pharmaceutical composition according to any one of [31] to [39], wherein the HIF-2α inhibitor is belzutifan, NKT2152, DFF332, AB521, or BPI-452080, or a pharmaceutically acceptable salt thereof.

[41] The pharmaceutical composition according to any one of [31] to [39], wherein the HIF-2α inhibitor is belzutifan or a pharmaceutically acceptable salt thereof.

[42] The pharmaceutical composition according to any one of [31] to [41], wherein the anti-CDH6 antibody-drug conjugate and the HIF-2α inhibitor are separately contained (as active components) in different formulations, and are administered at the same time (at approximately the same time) or at different times.

[43] The pharmaceutical composition according to any one of [31] to [41], wherein the anti-CDH6 antibody-drug conjugate and the HIF-2α inhibitor are contained (as active components) in a single formulation for administration.

[44] The pharmaceutical composition according to any one of [31] to [43], wherein the pharmaceutical composition is for use in treating cancer.

[45] The pharmaceutical composition according to any one of [31] to [43], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer,

stomach cancer, endometrial cancer, uterine body cancer, nasopharyngeal cancer, prostate cancer, and cancer related to von Hippel-Lindau disease.

[46] The pharmaceutical composition according to any one of [31] to [43], wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[47] The pharmaceutical composition according to any one of [31] to [43], wherein the pharmaceutical composition is for use in treating at least one selected from the group consisting of renal cell carcinoma, renal clear cell carcinoma, and papillary renal cell carcinoma.

[48] The pharmaceutical composition according to any one of [1] to [47], wherein the anti-CDH6 antibody-drug conjugate is raludotatug deruxtecan (DS-6000a).

[49] A method of treatment, comprising administering an anti-CDH6 antibody-drug conjugate and a HIF-2α inhibitor in combination to a subject in need of the treatment,

wherein the antibody-drug conjugate is an antibody-drug conjugate in which a drug-linker represented by the formula:

[Formula 3]

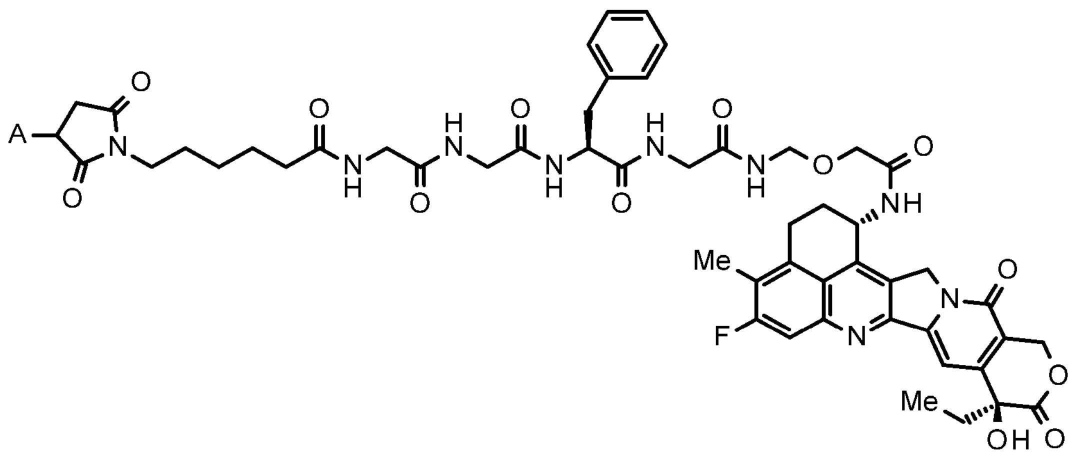

wherein A represents a connecting position to an anti-CDH6 antibody or a functional fragment of the antibody, is conjugated to the anti-CDH6 antibody or the functional fragment of the antibody via a thioether bond.

[50] The method of treatment according to [49], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

[51] The method of treatment according to [49] or [50], wherein the anti-CDH6 antibody or the functional fragment of the antibody is an antibody comprising

CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,

(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23; and

CDRL1, CDRL2 and CDRL3 in any one combination selected from the group consisting of the following combinations (6) to (9):

(6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[52] The method of treatment according to any one of [49] to [51], wherein the anti-CDH6 antibody or the functional fragment of the antibody is an antibody comprising CDRH1, CDRH2, CDRH3, CDRL1, CDRL2 and CDRL3 in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid sequence shown in SEQ ID NO: 8, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, CDRH3 consisting of the amino acid sequence shown in SEQ ID NO: 11, CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, CDRH3 consisting of the amino acid sequence shown in SEQ ID NO: 17, CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid sequence shown in SEQ ID NO: 22, CDRH3 consisting of the amino acid sequence shown in SEQ ID NO: 23, CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.
[53] The method of treatment according to any one of [49] to [52], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

[54] The method of treatment according to any one of [49] to [53], wherein the anti-CDH6 antibody or the functional fragment of the antibody is a humanized antibody or functional fragment of the antibody.
[55] The method of treatment according to any one of [49] to [54], wherein the anti-CDH6 antibody or the functional fragment of the antibody is an antibody comprising

any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4); and

any one light chain variable region selected from the group consisting of the following variable regions (6) to (9):

(6) a light chain variable region consisting of the amino acid sequence shown in SEQ ID NO: 37,
(7) a light chain variable region consisting of the amino acid sequence shown in SEQ ID NO: 40,
(8) an amino acid sequence having a sequence identity of at least 95% or more to the sequence of the framework regions other than at each CDR sequence in the amino acid sequences of any one of (6) and (7), and
(9) an amino acid sequence comprising a deletion, substitution or addition of one or several amino acids in the sequence of the framework regions other than at each CDR sequence in the amino acid sequences of any one of (6) to (8),

or a functional fragment of the antibody.

[56] The method of treatment according to any one of [49] to [55], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,
(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.
[57] The method of treatment according to any one of [49] to [56], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.
[58] The method of treatment according to any one of [49] to [57], wherein the anti-CDH6 antibody or the functional fragment of the antibody is an antibody comprising any of the following combinations (1) to (4):

(1) a heavy chain consisting of the amino acid sequence shown in SEQ ID NO: 29 and a light chain consisting of the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38,

or a functional fragment of the antibody.

[59] The method of treatment according to any one of [49] to [58], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.

[60] The method of treatment according to any one of [49] to [59], wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus, or a functional fragment of the antibody.

[61] The method of treatment according to [60], wherein the anti-CDH6 antibody or the functional fragment of the antibody is an antibody in which one or two amino acids are deleted from the carboxyl terminus of a heavy chain thereof, or a functional fragment of the antibody.

[62] The method of treatment according to [61], wherein the anti-CDH6 antibody or the functional fragment of the antibody is an antibody in which one amino acid is deleted from the carboxyl terminus of each of the two heavy chains thereof, or a functional fragment of the antibody.

[63] The method of treatment according to any one of [60] to [62], wherein the deleted amino acid is lysine.

[64] The method of treatment according to any one of [60] to [63], wherein the anti-CDH6 antibody or the functional fragment of the antibody is an antibody in which a proline residue at the carboxyl terminus of a heavy chain thereof is further amidated, or a functional fragment of the antibody.

[65] The method of treatment according to any one of [49] to [64], wherein the average number of units of the drug-linker conjugated per antibody in the anti-CDH6 antibody-drug conjugate is in the range of from 1 to 10.

[66] The method of treatment according to any one of [49] to [64], wherein the average number of units of the drug-linker conjugated per antibody in the anti-CDH6 antibody-drug conjugate is in the range of from 2 to 8.

[67] The method of treatment according to any one of [49] to [64], wherein the average number of units of the drug-linker conjugated per antibody in the anti-CDH6 antibody-drug conjugate is in the range of from 5 to 8.

[68] The method of treatment according to any one of [49] to [64], wherein the average number of units of the drug-linker conjugated per antibody in the anti-CDH6 antibody-drug conjugate is in the range of from 7 to 8.

[69] The method of treatment according to any one of [49] to [64], wherein the average number of units of the drug-linker conjugated per antibody in the anti-CDH6 antibody-drug conjugate is in the range of from 7.5 to 8.

[70] The method of treatment according to any one of [49] to [64], wherein the average number of units of the drug-linker conjugated per antibody in the anti-CDH6 antibody-drug conjugate is approximately 8.

[70-2] The method of treatment according to any one of [49] to [64], wherein the number of the drug-linker structures conjugated per antibody in the anti-CDH6 antibody-drug conjugate is an integer in the range of from 1 to 10.

[70-3] The method of treatment according to any one of [49] to [64], wherein the number of the drug-linker structures conjugated per antibody in the anti-CDH6 antibody-drug conjugate is an integer in the range of from 2 to 8.

[70-4] The method of treatment according to any one of [49] to [64], wherein the number of the drug-linker structures conjugated per antibody in the anti-CDH6 antibody-drug conjugate is 2, 4, 6 or 8.

[70-5] The method of treatment according to any one of [49] to [64], wherein the number of the drug-linker structures conjugated per antibody in the anti-CDH6 antibody-drug conjugate is 8.

[71] The method of treatment according to any one of [49] to [70-5], wherein the HIF-2$\alpha$ inhibitor is belzutifan, NKT2152, DFF332, AB521, or BPI-452080, or a pharmaceutically acceptable salt thereof.

[72] The method of treatment according to any one of [49] to [70-5], wherein the HIF-2$\alpha$ inhibitor is belzutifan or a pharmaceutically acceptable salt thereof.

[73] The method of treatment according to any one of [49] to [72], wherein the anti-CDH6 antibody-drug conjugate and the HIF-2$\alpha$ inhibitor are separately contained (as active components) in different formulations, and are administered at the same time (at approximately the same time) or at different times.

[74] The method of treatment according to any one of [49] to [72], wherein the anti-CDH6 antibody-drug conjugate and the HIF-2$\alpha$ inhibitor are contained (as active components) in a single formulation for administration.

[75] The method of treatment according to any one of [49] to [74], wherein the method of treatment is for treating cancer.

[76] The method of treatment according to any one of [49] to [74], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

[77] The method of treatment according to any one of [49] to [74], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[78] The method of treatment according to any one of [49] to [74], wherein the method of treatment is for treating at least one selected from the group consisting of renal cell carcinoma, renal clear cell carcinoma, and papillary renal cell carcinoma.

[79] A method of treatment, comprising administering an anti-CDH6 antibody-drug conjugate and a HIF-2α inhibitor in combination to a subject in need of the treatment, wherein the anti-CDH6 antibody-drug conjugate is an antibody-drug conjugate represented by the formula:

[Formula 4]

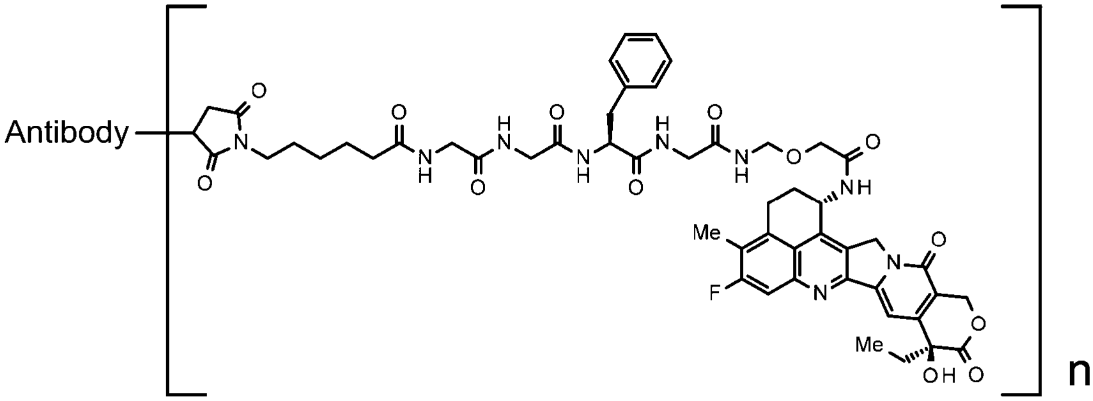

wherein Antibody is an anti-CDH6 antibody, a drug-linker is conjugated to the antibody via a thioether bond, and n represents an average number of units of the drug-linker conjugated per antibody.

[80] The method of treatment according to [79], wherein the anti-CDH6 antibody is an antibody comprising a heavy chain consisting of the amino acid sequence shown in SEQ ID NO: 29 and a light chain consisting of the amino acid sequence shown in SEQ ID NO: 38.

[81] The method of treatment according to [79] or [80], wherein a lysine residue is deleted from the carboxyl terminus of a heavy chain of the anti-CDH6 antibody.

[82] The method of treatment according to any one of [79] to [81], wherein the average number of units of the drug-linker conjugated per antibody in the anti-CDH6 antibody-drug conjugate is in the range of from 1 to 10.

[83] The method of treatment according to any one of [79] to [81], wherein the average number of units of the drug-linker conjugated per antibody in the anti-CDH6 antibody-drug conjugate is in the range of from 2 to 8.

[84] The method of treatment according to any one of [79] to [81], wherein the average number of units of the drug-linker conjugated per antibody in the anti-CDH6 antibody-drug conjugate is in the range of from 5 to 8.

[85] The method of treatment according to any one of [79] to [81], wherein the average number of units of the drug-linker conjugated per antibody in the anti-CDH6 antibody-drug conjugate is in the range of from 7 to 8.

[86] The method of treatment according to any one of [79] to [81], wherein the average number of units of the drug-linker conjugated per antibody in the anti-CDH6 antibody-drug conjugate is in the range of from 7.5 to 8.

[87] The method of treatment according to any one of [79] to [81], wherein the average number of units of the drug-linker conjugated per antibody in the anti-CDH6 antibody-drug conjugate is approximately 8.

[88] The method of treatment according to any one of [79] to [87], wherein the HIF-2α inhibitor is belzutifan, NKT2152, DFF332, AB521, or BPI-452080, or a pharmaceutically acceptable salt thereof.

[89] The method of treatment according to any one of [79] to [87], wherein the HIF-2α inhibitor is belzutifan or a pharmaceutically acceptable salt thereof.

[90] The method of treatment according to any one of [79] to [89], wherein the anti-CDH6 antibody-drug conjugate and the HIF-2α inhibitor are separately contained (as active components) in different formulations, and are administered at the same time (at approximately the same time) or at different times.

[91] The method of treatment according to any one of [79] to [89], wherein the anti-CDH6 antibody-drug conjugate and the HIF-2$\alpha$ inhibitor are contained (as active components) in a single formulation for administration.
[92] The method of treatment according to any one of [79] to [91], wherein the method of treatment is for treating cancer.
[93] The method of treatment according to any one of [79] to [91], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.
[94] The method of treatment according to any one of [79] to [91], wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.
[95] The method of treatment according to any one of [79] to [91], wherein the method of treatment is for treating at least one selected from the group consisting of renal cell carcinoma, renal clear cell carcinoma, and papillary renal cell carcinoma.
[96] The method of treatment according to any one of [49] to [95], wherein the anti-CDH6 antibody-drug conjugate is raludotatug deruxtecan (DS-6000a).
[97] An anti-CDH6 antibody-drug conjugate for use in treating a disease by administering the anti-CDH6 antibody-drug conjugate and a HIF-2$\alpha$ inhibitor in combination, wherein in the anti-CDH6 antibody-drug conjugate, a drug-linker represented by the formula:

[Formula 5]

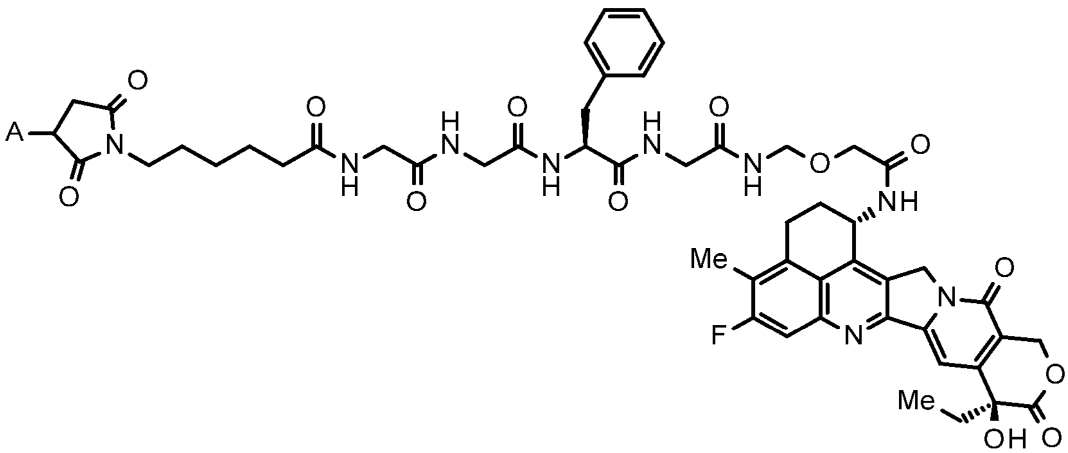

wherein A represents a connecting position to an anti-CDH6 antibody or a functional fragment of the antibody, is conjugated to the anti-CDH6 antibody or the functional fragment of the antibody via a thioether bond.
[98] The anti-CDH6 antibody-drug conjugate according to [97], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.
[99] The anti-CDH6 antibody-drug conjugate according to [97] or [98], wherein the anti-CDH6 antibody or the functional fragment of the antibody is an antibody comprising

CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID

NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23; and

CDRL1, CDRL2 and CDRL3 in any one combination selected from the group consisting of the following combinations (6) to (9):

(6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[100] The anti-CDH6 antibody-drug conjugate according to any one of [97] to [99], wherein the anti-CDH6 antibody or the functional fragment of the antibody is an antibody comprising CDRH1, CDRH2, CDRH3, CDRL1, CDRL2 and CDRL3 in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid sequence shown in SEQ ID NO: 8, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, CDRH3 consisting of the amino acid sequence shown in SEQ ID NO: 11, CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, CDRH3 consisting of the amino acid sequence shown in SEQ ID NO: 17, CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid sequence shown in SEQ ID NO: 22, CDRH3 consisting of the amino acid sequence shown in SEQ ID NO: 23, CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.

[101] The anti-CDH6 antibody-drug conjugate according to any one of [97] to [100], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

[102] The anti-CDH6 antibody-drug conjugate according to any one of [97] to [101], wherein the anti-CDH6 antibody or the functional fragment of the antibody is a humanized antibody or functional fragment of the antibody.

[103] The anti-CDH6 antibody-drug conjugate according to any one of [97] to [102], wherein the anti-CDH6 antibody or the functional fragment of the antibody is an antibody comprising

any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4); and

any one light chain variable region selected from the group consisting of the following variable regions (6) to (9):

(6) a light chain variable region consisting of the amino acid sequence shown in SEQ ID NO: 37,
(7) a light chain variable region consisting of the amino acid sequence shown in SEQ ID NO: 40,
(8) an amino acid sequence having a sequence identity of at least 95% or more to the sequence of the framework regions other than at each CDR sequence in the amino acid sequences of any one of (6) and (7), and
(9) an amino acid sequence comprising a deletion, substitution or addition of one or several amino acids in the sequence of the framework regions other than at each CDR sequence in the amino acid sequences of any one of (6) to (8),

or a functional fragment of the antibody.

[104] The anti-CDH6 antibody-drug conjugate according to any one of [97] to [103], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,
(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.

[105] The anti-CDH6 antibody-drug conjugate according to any one of [97] to [104], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.

[106] The anti-CDH6 antibody-drug conjugate according to any one of [97] to [105], wherein the anti-CDH6 antibody or the functional fragment of the antibody is an antibody comprising any of the following combinations (1) to (4):

(1) a heavy chain consisting of the amino acid sequence shown in SEQ ID NO: 29 and a light chain consisting of

the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38,

or a functional fragment of the antibody.
[107] The anti-CDH6 antibody-drug conjugate according to any one of [97] to [106], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.
[108] The anti-CDH6 antibody-drug conjugate according to any one of [97] to [107], wherein the anti-CDH6 antibody or the functional fragment of the antibody is an antibody in which

the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus,
or a functional fragment of the antibody.

[109] The anti-CDH6 antibody-drug conjugate according to [108], wherein the anti-CDH6 antibody or the functional fragment of the antibody is an antibody in which one or two amino acids are deleted from the carboxyl terminus of a heavy chain thereof, or a functional fragment of the antibody.
[110] The anti-CDH6 antibody-drug conjugate according to [109], wherein the anti-CDH6 antibody or the functional fragment of the antibody is an antibody in which one amino acid is deleted from the carboxyl terminus of each of the two heavy chains thereof, or a functional fragment of the antibody.
[111] The anti-CDH6 antibody-drug conjugate according to any one of [108] to [110], wherein the deleted amino acid is lysine.
[112] The anti-CDH6 antibody-drug conjugate according to any one of [108] to [111], wherein the anti-CDH6 antibody or the functional fragment of the antibody is an antibody in which a proline residue at the carboxyl terminus of a heavy chain thereof is further amidated, or a functional fragment of the antibody.
[113] The anti-CDH6 antibody-drug conjugate according to any one of [97] to [112], wherein the average number of units of the drug-linker conjugated per antibody in the anti-CDH6 antibody-drug conjugate is in the range of from 1 to 10.
[114] The anti-CDH6 antibody-drug conjugate according to any one of [97] to [112], wherein the average number of units of the drug-linker conjugated per antibody in the anti-CDH6 antibody-drug conjugate is in the range of from 2 to 8.
[115] The anti-CDH6 antibody-drug conjugate according to any one of [97] to [112], wherein the average number of units of the drug-linker conjugated per antibody in the anti-CDH6 antibody-drug conjugate is in the range of from 5 to 8.
[116] The anti-CDH6 antibody-drug conjugate according to any one of [97] to [112], wherein the average number of units of the drug-linker conjugated per antibody in the anti-CDH6 antibody-drug conjugate is in the range of from 7 to 8.
[117] The anti-CDH6 antibody-drug conjugate according to any one of [97] to [112], wherein the average number of units of the drug-linker conjugated per antibody in the anti-CDH6 antibody-drug conjugate is in the range of from 7.5 to 8.
[118] The anti-CDH6 antibody-drug conjugate according to any one of [97] to [112], wherein the average number of units of the drug-linker conjugated per antibody in the anti-CDH6 antibody-drug conjugate is approximately 8.
[118-2] The anti-CDH6 antibody-drug conjugate according to any one of [97] to [112], wherein the number of the drug-linker structures conjugated per antibody in the anti-CDH6 antibody-drug conjugate is an integer in the range of from 1 to 10.
[118-3] The anti-CDH6 antibody-drug conjugate according to any one of [97] to [112], wherein the number of the drug-linker structures conjugated per antibody in the anti-CDH6 antibody-drug conjugate is an integer in the range of from 2 to 8.
[118-4] The anti-CDH6 antibody-drug conjugate according to any one of [97] to [112], wherein the number of the drug-linker structures conjugated per antibody in the anti-CDH6 antibody-drug conjugate is 2, 4, 6 or 8.
[118-5] The anti-CDH6 antibody-drug conjugate according to any one of [97] to [112], wherein the number of the drug-linker structures conjugated per antibody in the anti-CDH6 antibody-drug conjugate is 8.

[119] The anti-CDH6 antibody-drug conjugate according to any one of [97] to [118-5], wherein the HIF-2α inhibitor is belzutifan, NKT2152, DFF332, AB521, or BPI-452080, or a pharmaceutically acceptable salt thereof.

[120] The anti-CDH6 antibody-drug conjugate according to any one of [97] to [118-5], wherein the HIF-2α inhibitor is belzutifan or a pharmaceutically acceptable salt thereof.

[121] The anti-CDH6 antibody-drug conjugate according to any one of [97] to [120], wherein the anti-CDH6 antibody-drug conjugate and the HIF-2α inhibitor are separately contained (as active components) in different formulations, and are administered at the same time (at approximately the same time) or at different times.

[122] The anti-CDH6 antibody-drug conjugate according to any one of [97] to [120], wherein the anti-CDH6 antibody-drug conjugate and the HIF-2α inhibitor are contained (as active components) in a single formulation for administration.

[123] The anti-CDH6 antibody-drug conjugate according to any one of [97] to [122], wherein the anti-CDH6 antibody-drug conjugate is for use in treating cancer.

[124] The anti-CDH6 antibody-drug conjugate according to any one of [97] to [122], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

[125] The anti-CDH6 antibody-drug conjugate according to any one of [97] to [122], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[126] The anti-CDH6 antibody-drug conjugate according to any one of [97] to [122], wherein the anti-CDH6 antibody-drug conjugate is for use in treating at least one selected from the group consisting of renal cell carcinoma, renal clear cell carcinoma, and papillary renal cell carcinoma.

[127] An anti-CDH6 antibody-drug conjugate for use in treating a disease by administering the anti-CDH6 antibody-drug conjugate and a HIF-2α inhibitor in combination, wherein the anti-CDH6 antibody-drug conjugate is represented by the formula:

[Formula 6]

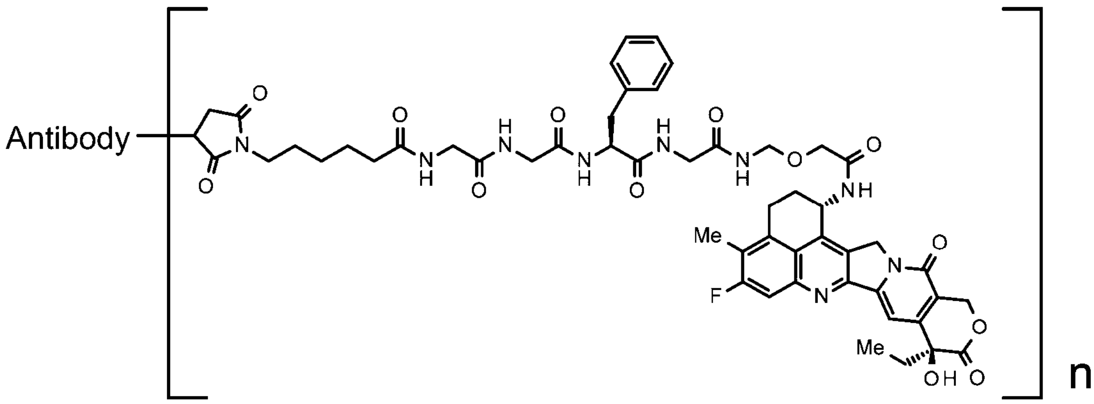

wherein Antibody is an anti-CDH6 antibody, a drug-linker is conjugated to the antibody via a thioether bond, and n represents an average number of units of the drug-linker conjugated per antibody.

[128] The anti-CDH6 antibody-drug conjugate according to [127] , wherein the anti-CDH6 antibody is an antibody comprising a heavy chain consisting of the amino acid sequence shown in SEQ ID NO: 29 and a light chain consisting of the amino acid sequence shown in SEQ ID NO: 38.

[129] The anti-CDH6 antibody-drug conjugate according to [127] or [128], wherein a lysine residue is deleted from the carboxyl terminus of a heavy chain of the anti-CDH6 antibody.

[130] The anti-CDH6 antibody-drug conjugate according to any one of [127] to [129], wherein the average number of units of the drug-linker conjugated per antibody in the anti-CDH6 antibody-drug conjugate is in the range of from 1 to

10.

[131] The anti-CDH6 antibody-drug conjugate according to any one of [127] to [129], wherein the average number of units of the drug-linker conjugated per antibody in the anti-CDH6 antibody-drug conjugate is in the range of from 2 to 8.

[132] The anti-CDH6 antibody-drug conjugate according to any one of [127] to [129], wherein the average number of units of the drug-linker conjugated per antibody in the anti-CDH6 antibody-drug conjugate is in the range of from 5 to 8.

[133] The anti-CDH6 antibody-drug conjugate according to any one of [127] to [129], wherein the average number of units of the drug-linker conjugated per antibody in the anti-CDH6 antibody-drug conjugate is in the range of from 7 to 8.

[134] The anti-CDH6 antibody-drug conjugate according to any one of [127] to [129], wherein the average number of units of the drug-linker conjugated per antibody in the anti-CDH6 antibody-drug conjugate is in the range of from 7.5 to 8.

[135] The anti-CDH6 antibody-drug conjugate according to any one of [127] to [129], wherein the average number of units of the drug-linker conjugated per antibody in the anti-CDH6 antibody-drug conjugate is approximately 8.

[136] The anti-CDH6 antibody-drug conjugate according to any one of [127] to [135], wherein the HIF-2α inhibitor is belzutifan, NKT2152, DFF332, AB521, or BPI-452080, or a pharmaceutically acceptable salt thereof.

[137] The anti-CDH6 antibody-drug conjugate according to any one of [127] to [135], wherein the HIF-2α inhibitor is belzutifan or a pharmaceutically acceptable salt thereof.

[138] The anti-CDH6 antibody-drug conjugate according to any one of [127] to [137], wherein the anti-CDH6 antibody-drug conjugate and the HIF-2α inhibitor are separately contained (as active components) in different formulations, and are administered at the same time (at approximately the same time) or at different times.

[139] The anti-CDH6 antibody-drug conjugate according to any one of [127] to [137], wherein the anti-CDH6 antibody-drug conjugate and the HIF-2α inhibitor are contained (as active components) in a single formulation for administration.

[140] The anti-CDH6 antibody-drug conjugate according to any one of [127] to [139], wherein the anti-CDH6 antibody-drug conjugate is for use in treating cancer.

[141] The anti-CDH6 antibody-drug conjugate according to any one of [127] to [139], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

[142] The anti-CDH6 antibody-drug conjugate according to any one of [127] to [139], wherein the anti-CDH6 antibody-drug conjugate is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[143] The anti-CDH6 antibody-drug conjugate according to any one of [127] to [139], wherein the anti-CDH6 antibody-drug conjugate is for use in treating at least one selected from the group consisting of renal cell carcinoma, renal clear cell carcinoma, and papillary renal cell carcinoma.

[144] The anti-CDH6 antibody-drug conjugate according to any one of [97] to [143], wherein the anti-CDH6 antibody-drug conjugate is raludotatug deruxtecan (DS-6000a).

[145] Use of an anti-CDH6 antibody-drug conjugate for the manufacture of a medicament for treating a disease through being administered in combination with a HIF-2α inhibitor, wherein in the anti-CDH6 antibody-drug conjugate, a drug-linker represented by the formula:

[Formula 7]

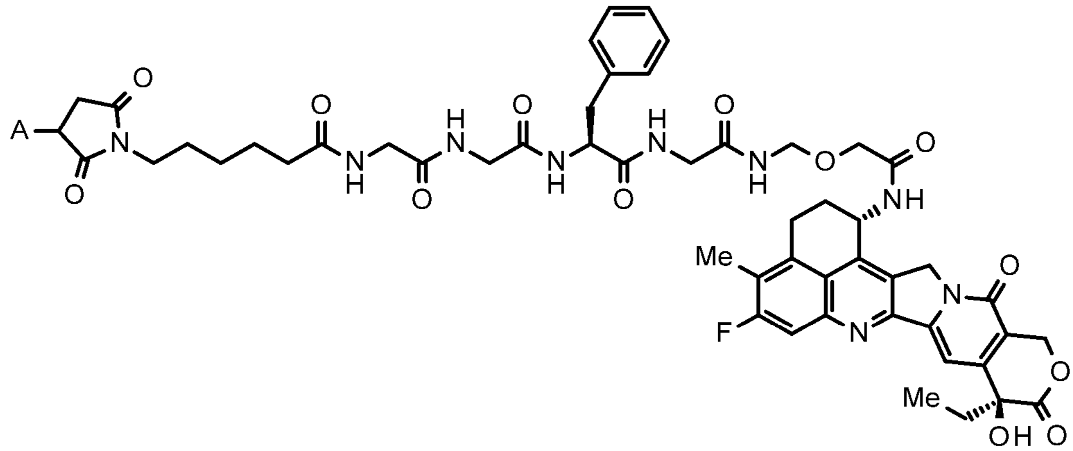

wherein A represents a connecting position to an anti-CDH6 antibody or a functional fragment of the antibody, is conjugated to the anti-CDH6 antibody or the functional fragment of the antibody via a thioether bond.
[146] The use according to [145], wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.
[147] The use according to [145] or [146], wherein the anti-CDH6 antibody or the functional fragment of the antibody is an antibody comprising

CDRH1, CDRH2 and CDRH3 in any one combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4,
(2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4,
(3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11,
(4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and
(5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23; and

CDRL1, CDRL2 and CDRL3 in any one combination selected from the group consisting of the following combinations (6) to (9):

(6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(9) CDRL1 consisting of the amino acid sequence shown in SEQ ID NO: 24, CDRL2 consisting of the amino

acid sequence shown in SEQ ID NO: 25, and CDRL3 consisting of the amino acid sequence shown in SEQ ID NO: 26,

or a functional fragment of the antibody.

[148] The use according to any one of [145] to [147], wherein the anti-CDH6 antibody or the functional fragment of the antibody is an antibody comprising CDRH1, CDRH2, CDRH3, CDRL1, CDRL2 and CDRL3 in any combination selected from the group consisting of the following combinations (1) to (5):

(1) CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(2) CDRH1 consisting of the amino acid sequence shown in SEQ ID NO: 2, CDRH2 consisting of the amino acid sequence shown in SEQ ID NO: 8, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7,
(3) CDRH1 consisting of the amino acid sequence shown in SEQ ID NO: 9, CDRH2 consisting of the amino acid sequence shown in SEQ ID NO: 10, CDRH3 consisting of the amino acid sequence shown in SEQ ID NO: 11, CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14,
(4) CDRH1 consisting of the amino acid sequence shown in SEQ ID NO: 15, CDRH2 consisting of the amino acid sequence shown in SEQ ID NO: 16, CDRH3 consisting of the amino acid sequence shown in SEQ ID NO: 17, CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and
(5) CDRH1 consisting of the amino acid sequence shown in SEQ ID NO: 21, CDRH2 consisting of the amino acid sequence shown in SEQ ID NO: 22, CDRH3 consisting of the amino acid sequence shown in SEQ ID NO: 23, CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26,

or a functional fragment of the antibody.
[149] The use according to any one of [145] to [148], wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.
[150] The use according to any one of [145] to [149], wherein the anti-CDH6 antibody or the functional fragment of the antibody is a humanized antibody or functional fragment of the antibody.
[151] The use according to any one of [145] to [150], wherein the anti-CDH6 antibody or the functional fragment of the antibody is an antibody comprising

any one heavy chain variable region selected from the group consisting of the following variable regions (1) to (5):

(1) a heavy chain variable region consisting of the amino acid sequence shown in SEQ ID NO: 28,
(2) a heavy chain variable region consisting of the amino acid sequence shown in SEQ ID NO: 31,
(3) a heavy chain variable region consisting of the amino acid sequence shown in SEQ ID NO: 34,
(4) an amino acid sequence having a sequence identity of at least 95% or more to the sequence of the framework regions other than at each CDR sequence in the amino acid sequences of any one of (1) to (3), and
(5) an amino acid sequence comprising a deletion, substitution or addition of one or several amino acids in the sequence of the framework regions other than at each CDR sequence in the amino acid sequences of any one of (1) to (4); and

any one light chain variable region selected from the group consisting of the following variable regions (6) to (9):

(6) a light chain variable region consisting of the amino acid sequence shown in SEQ ID NO: 37,
(7) a light chain variable region consisting of the amino acid sequence shown in SEQ ID NO: 40,

(8) an amino acid sequence having a sequence identity of at least 95% or more to the sequence of the framework regions other than at each CDR sequence in the amino acid sequences of any one of (6) and (7), and

(9) an amino acid sequence comprising a deletion, substitution or addition of one or several amino acids in the sequence of the framework regions other than at each CDR sequence in the amino acid sequences of any one of (6) to (8),

or a functional fragment of the antibody.

[152] The use according to any one of [145] to [151], wherein the anti-CDH6 antibody or the functional fragment of the antibody is an antibody comprising a heavy chain variable region and a light chain variable region in any of the following combinations (1) to (4):

(1) a heavy chain variable region consisting of the amino acid sequence shown in SEQ ID NO: 28 and a light chain variable region consisting of the amino acid sequence shown in SEQ ID NO: 37,
(2) a heavy chain variable region consisting of the amino acid sequence shown in SEQ ID NO: 31 and a light chain variable region consisting of the amino acid sequence shown in SEQ ID NO: 37,
(3) a heavy chain variable region consisting of the amino acid sequence shown in SEQ ID NO: 31 and a light chain variable region consisting of the amino acid sequence shown in SEQ ID NO: 40, and
(4) a heavy chain variable region consisting of the amino acid sequence shown in SEQ ID NO: 34 and a light chain variable region consisting of the amino acid sequence shown in SEQ ID NO: 37,

or a functional fragment of the antibody.
[153] The use according to any one of [145] to [152], wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.
[154] The use according to any one of [145] to [153], wherein the anti-CDH6 antibody or the functional fragment of the antibody is an antibody comprising any of the following combinations (1) to (4):

(1) a heavy chain consisting of the amino acid sequence shown in SEQ ID NO: 29 and a light chain consisting of the amino acid sequence shown in SEQ ID NO: 38,
(2) a heavy chain consisting of the amino acid sequence shown in SEQ ID NO: 32 and a light chain consisting of the amino acid sequence shown in SEQ ID NO: 38,
(3) a heavy chain consisting of the amino acid sequence shown in SEQ ID NO: 32 and a light chain consisting of the amino acid sequence shown in SEQ ID NO: 41, and
(4) a heavy chain consisting of the amino acid sequence shown in SEQ ID NO: 35 and a light chain consisting of the amino acid sequence shown in SEQ ID NO: 38,

or a functional fragment of the antibody.
[155] The use according to any one of [145] to [154], wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.
[156] The use according to any one of [145] to [155], wherein the anti-CDH6 antibody or the functional fragment of the antibody is an antibody in which

the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus,
or a functional fragment of the antibody.

[157] The use according to [156], wherein the anti-CDH6 antibody or the functional fragment of the antibody is an antibody in which one or two amino acids are deleted from the carboxyl terminus of a heavy chain thereof, or a functional fragment of the antibody.
[158] The use according to [157], wherein the anti-CDH6 antibody or the functional fragment of the antibody is an antibody in which one amino acid is deleted from the carboxyl terminus of each of the two heavy chains thereof, or a

functional fragment of the antibody.

[159] The use according to any one of [156] to [158], wherein the deleted amino acid is lysine.

[160] The use according to any one of [156] to [159], wherein the anti-CDH6 antibody or the functional fragment of the antibody is an antibody in which a proline residue at the carboxyl terminus of a heavy chain thereof is further amidated, or a functional fragment of the antibody.

[161] The use according to any one of [145] to [160], wherein the average number of units of the drug-linker conjugated per antibody in the anti-CDH6 antibody-drug conjugate is in the range of from 1 to 10.

[162] The use according to any one of [145] to [160], wherein the average number of units of the drug-linker conjugated per antibody in the anti-CDH6 antibody-drug conjugate is in the range of from 2 to 8.

[163] The use according to any one of [145] to [160], wherein the average number of units of the drug-linker conjugated per antibody in the anti-CDH6 antibody-drug conjugate is in the range of from 5 to 8.

[164] The use according to any one of [145] to [160], wherein the average number of units of the drug-linker conjugated per antibody in the anti-CDH6 antibody-drug conjugate is in the range of from 7 to 8.

[165] The use according to any one of [145] to [160], wherein the average number of units of the drug-linker conjugated per antibody in the anti-CDH6 antibody-drug conjugate is in the range of from 7.5 to 8.

[166] The use according to any one of [145] to [160], wherein the average number of units of the drug-linker conjugated per antibody in the anti-CDH6 antibody-drug conjugate is approximately 8.

[166-2] The use according to any one of [145] to [160], wherein the number of the drug-linker structures conjugated per antibody in the anti-CDH6 antibody-drug conjugate is an integer in the range of from 1 to 10.

[166-3] The use according to any one of [145] to [160], wherein the number of the drug-linker structures conjugated per antibody in the anti-CDH6 antibody-drug conjugate is an integer in the range of from 2 to 8.

[166-4] The use according to any one of [145] to [160], wherein the number of the drug-linker structures conjugated per antibody in the anti-CDH6 antibody-drug conjugate is 2, 4, 6 or 8.

[166-5] The use according to any one of [145] to [160], wherein the number of the drug-linker structures conjugated per antibody in the anti-CDH6 antibody-drug conjugate is 8.

[167] The use according to any one of [145] to [166-5], wherein the HIF-2$\alpha$ inhibitor is belzutifan, NKT2152, DFF332, AB521, or BPI-452080, or a pharmaceutically acceptable salt thereof.

[168] The use according to any one of [145] to [166-5], wherein the HIF-2$\alpha$ inhibitor is belzutifan or a pharmaceutically acceptable salt thereof.

[169] The use according to any one of [145] to [168], wherein the anti-CDH6 antibody-drug conjugate and the HIF-2$\alpha$ inhibitor are separately contained (as active components) in different formulations, and are administered at the same time (at approximately the same time) or at different times.

[170] The use according to any one of [145] to [168], wherein the anti-CDH6 antibody-drug conjugate and the HIF-2$\alpha$ inhibitor are contained (as active components) in a single formulation for administration.

[171] The use according to any one of [145] to [170], wherein the use is for treating cancer.

[172] The use according to any one of [145] to [170], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

[173] The use according to any one of [145] to [170], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[174] The use according to any one of [145] to [170], wherein the use is for treating at least one selected from the group consisting of renal cell carcinoma, renal clear cell carcinoma, and papillary renal cell carcinoma.

[175] Use of an anti-CDH6 antibody-drug conjugate for the manufacture of a medicament for treating a disease through being administered in combination with a HIF-2$\alpha$ inhibitor, wherein the anti-CDH6 antibody-drug conjugate is represented by the formula:

[Formula 8]

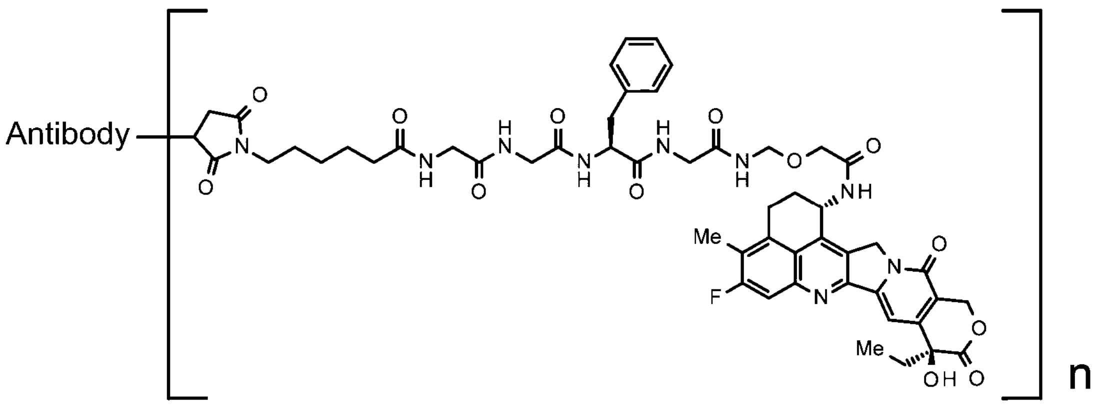

wherein Antibody is an anti-CDH6 antibody, a drug-linker is conjugated to the antibody via a thioether bond, and n represents an average number of units of the drug-linker conjugated per antibody.

[176] The use according to [175], wherein the anti-CDH6 antibody is an antibody comprising a heavy chain consisting of the amino acid sequence shown in SEQ ID NO: 29 and a light chain consisting of the amino acid sequence shown in SEQ ID NO: 38.

[177] The use according to [175] or [176], wherein a lysine residue is deleted from the carboxyl terminus of a heavy chain of the anti-CDH6 antibody.

[178] The use according to any one of [175] to [177], wherein the average number of units of the drug-linker conjugated per antibody in the anti-CDH6 antibody-drug conjugate is in the range of from 1 to 10.

[179] The use according to any one of [175] to [177], wherein the average number of units of the drug-linker conjugated per antibody in the anti-CDH6 antibody-drug conjugate is in the range of from 2 to 8.

[180] The use according to any one of [175] to [177], wherein the average number of units of the drug-linker conjugated per antibody in the anti-CDH6 antibody-drug conjugate is in the range of from 5 to 8.

[181] The use according to any one of [175] to [177], wherein the average number of units of the drug-linker conjugated per antibody in the anti-CDH6 antibody-drug conjugate is in the range of from 7 to 8.

[182] The use according to any one of [175] to [177], wherein the average number of units of the drug-linker conjugated per antibody in the anti-CDH6 antibody-drug conjugate is in the range of from 7.5 to 8.

[183] The use according to any one of [175] to [177], wherein the average number of units of the drug-linker conjugated per antibody in the anti-CDH6 antibody-drug conjugate is approximately 8.

[184] The use according to any one of [175] to [183], wherein the HIF-2α inhibitor is belzutifan, NKT2152, DFF332, AB521, or BPI-452080, or a pharmaceutically acceptable salt thereof.

[185] The use according to any one of [175] to [183], wherein the HIF-2α inhibitor is belzutifan or a pharmaceutically acceptable salt thereof.

[186] The use according to any one of [175] to [185], wherein the anti-CDH6 antibody-drug conjugate and the HIF-2α inhibitor are separately contained (as active components) in different formulations, and are administered at the same time (at approximately the same time) or at different times.

[187] The use according to any one of [175] to [185], wherein the anti-CDH6 antibody-drug conjugate and the HIF-2α inhibitor are contained (as active components) in a single formulation for administration.

[188] The use according to any one of [175] to [187], wherein the use is for treating cancer.

[189] The use according to any one of [175] to [187], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

[190] The use according to any one of [175] to [187], wherein the use is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma.

[191] The use according to any one of [175] to [187], wherein the use is for treating at least one selected from the group consisting of renal cell carcinoma, renal clear cell carcinoma, and papillary renal cell carcinoma.

[192] The use according to any one of [145] to [191], wherein the anti-CDH6 antibody-drug conjugate is raludotatug deruxtecan (DS-6000a).

[193] A pharmaceutical product comprising an anti-CDH6 antibody-drug conjugate and a HIF-2$\alpha$ inhibitor for administration in combination, wherein the anti-CDH6 antibody-drug conjugate and the HIF-2$\alpha$ inhibitor are as defined in any one of [1] to [24], [31] to [41] and [48].

[194] The pharmaceutical product according to [193], wherein the pharmaceutical product is for use in treating a disease as defined in any one of [27] to [30] and [44] to [47].

[195] The pharmaceutical product according to [193] or [194], wherein the anti-CDH6 antibody-drug conjugate and the HIF-2$\alpha$ inhibitor are separately contained (as active components) in different formulations, and are administered at the same time (at approximately the same time) or at different times.

[196] The pharmaceutical product according to [193] or [194], wherein the anti-CDH6 antibody-drug conjugate and the HIF-2$\alpha$ inhibitor are contained (as active components) in a single formulation for administration.

[197] A combination medicament comprising an anti-CDH6 antibody-drug conjugate and a HIF-2$\alpha$ inhibitor, wherein the anti-CDH6 antibody-drug conjugate and the HIF-2$\alpha$ inhibitor are as defined in any one of [1] to [24], [31] to [41] and [48].

[198] The combination medicament according to [197], wherein the combination medicament is for use in treating a disease as defined in any one of [27] to [30] and [44] to [47].

[199] The combination medicament according to [197] or [198], wherein the anti-CDH6 antibody-drug conjugate and the HIF-2$\alpha$ inhibitor are separately contained (as active components) in different formulations, and are administered at the same time (at approximately the same time) or at different times.

[200] The combination medicament according to [197] or [198], wherein the anti-CDH6 antibody-drug conjugate and the HIF-2$\alpha$ inhibitor are contained (as active components) in a single formulation for administration.

[201] A pharmaceutical combination comprising an anti-CDH6 antibody-drug conjugate and a HIF-2$\alpha$ inhibitor, wherein the anti-CDH6 antibody-drug conjugate and the HIF-2$\alpha$ inhibitor are as defined in any one of [1] to [24], [31] to [41] and [48].

[202] The pharmaceutical combination according to [201], wherein the pharmaceutical combination is for use in treating a disease as defined in any one of [27] to [30] and [44] to [47].

[203] The pharmaceutical combination according to [201] or [202], wherein the anti-CDH6 antibody-drug conjugate and the HIF-2$\alpha$ inhibitor are separately contained (as active components) in different formulations, and are administered at the same time (at approximately the same time) or at different times.

[204] The pharmaceutical combination according to [201] or [202], wherein the anti-CDH6 antibody-drug conjugate and the HIF-2$\alpha$ inhibitor are contained (as active components) in a single formulation for administration.

[205] Use of an anti-CDH6 antibody-drug conjugate combined with a HIF-2$\alpha$ inhibitor for treatment of a disease, wherein the anti-CDH6 antibody-drug conjugate and the HIF-2$\alpha$ inhibitor are as defined in any one of [1] to [24], [31] to [41] and [48].

[206] The use according to [205], wherein the disease is as defined in any one of [27] to [30] and [44] to [47].

[207] The use according to [205] or [206], wherein the anti-CDH6 antibody-drug conjugate and the HIF-2$\alpha$ inhibitor are separately contained (as active components) in different formulations, and are administered at the same time (at approximately the same time) or at different times.

[208] The use according to [205] or [206], wherein the anti-CDH6 antibody-drug conjugate and the HIF-2$\alpha$ inhibitor are contained (as active components) in a single formulation for administration.

[209] A medicament comprising an anti-CDH6 antibody-drug conjugate and a HIF-2$\alpha$ inhibitor, wherein the anti-CDH6 antibody-drug conjugate and the HIF-2$\alpha$ inhibitor are as defined in any one of [1] to [24], [31] to [41] and [48].

[210] The medicament according to [209], wherein the medicament is for use in treating a disease as defined in any one of [27] to [30] and [44] to [47].

[211] The medicament according to [209] or [210], wherein the anti-CDH6 antibody-drug conjugate and the HIF-2$\alpha$ inhibitor are separately contained (as active components) in different formulations, and are administered at the same time (at approximately the same time) or at different times.

[212] The medicament according to [209] or [210], wherein the anti-CDH6 antibody-drug conjugate and the HIF-2$\alpha$ inhibitor are contained (as active components) in a single formulation for administration.

[213] A pharmaceutical composition comprising (i) an anti-CDH6 antibody-drug conjugate and (ii) a HIF-2$\alpha$ inhibitor, wherein the anti-CDH6 antibody-drug conjugate and the HIF-2$\alpha$ inhibitor are administered in combination, wherein the anti-CDH6 antibody-drug conjugate and the HIF-2$\alpha$ inhibitor are as defined in any one of [1] to [24], [31] to [41] and [48].

[214] The pharmaceutical composition according to [213], wherein the pharmaceutical composition is for use in treating a disease as defined in any one of [27] to [30] and [44] to [47].

[215] The pharmaceutical composition according to [213] or [214], wherein the anti-CDH6 antibody-drug conjugate and the HIF-2α inhibitor are separately contained (as active components) in different formulations, and are administered at the same time (at approximately the same time) or at different times.

[216] The pharmaceutical composition according to [213] or [214], wherein the anti-CDH6 antibody-drug conjugate and the HIF-2α inhibitor are contained (as active components) in a single formulation for administration.

[217] A kit comprising (i) a first composition comprising an anti-CDH6 antibody-drug conjugate and (ii) a second composition comprising a HIF-2α inhibitor, wherein the anti-CDH6 antibody-drug conjugate and the HIF-2α inhibitor are administered in combination, wherein the anti-CDH6 antibody-drug conjugate and the HIF-2α inhibitor are as defined in any one of [1] to [24], [31] to [41] and [48].

[218] The kit according to [217], wherein the kit is for use in treating a disease as defined in any one of [27] to [30] and [44] to [47].

[219] The kit according to [217] or [218], wherein the anti-CDH6 antibody-drug conjugate and the HIF-2α inhibitor are separately contained (as active components) in different formulations, and are administered at the same time (at approximately the same time) or at different times.

[220] The kit according to [217] or [218], wherein the anti-CDH6 antibody-drug conjugate and the HIF-2α inhibitor are contained (as active components) in a single formulation for administration.

[221] A method of treatment for cancer, comprising administering an anti-CDH6 antibody-drug conjugate and a HIF-2α inhibitor in combination to a subject in need of the treatment, wherein the anti-CDH6 antibody-drug conjugate and the HIF-2α inhibitor are as defined in any one of [1] to [24], [31] to [41] and [48].

[Advantageous Effects of Invention]

The present invention can provide a pharmaceutical composition wherein a specific anti-CDH6 antibody-drug conjugate and a HIF-2α inhibitor are administered in combination, and/or a method of treatment comprising administering a specific anti-CDH6 antibody-drug conjugate and a HIF-2α inhibitor in combination to a subject.

[Brief Description of Drawings]

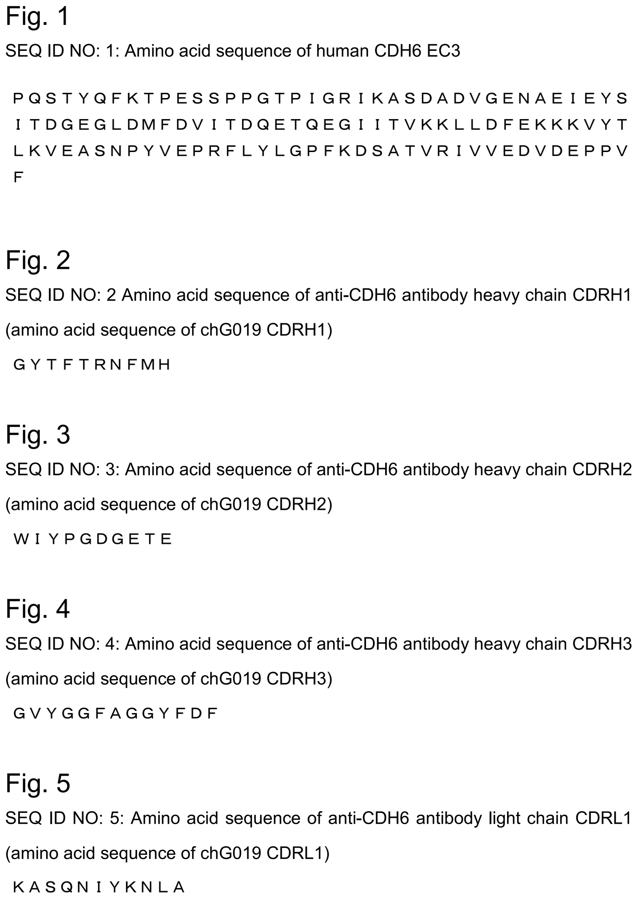

[Figure 1] Figure 1 is a diagram showing the amino acid sequence (SEQ ID NO: 1) of human CDH6 EC3.

[Figure 2] Figure 2 is a diagram showing the amino acid sequence (SEQ ID NO: 2) of an anti-CDH6 antibody heavy chain CDRH1.

[Figure 3] Figure 3 is a diagram showing the amino acid sequence (SEQ ID NO: 3) of an anti-CDH6 antibody heavy chain CDRH2.

[Figure 4] Figure 4 is a diagram showing the amino acid sequence (SEQ ID NO: 4) of an anti-CDH6 antibody heavy chain CDRH3.

[Figure 5] Figure 5 is a diagram showing the amino acid sequence (SEQ ID NO: 5) of an anti-CDH6 antibody light chain CDRL1.

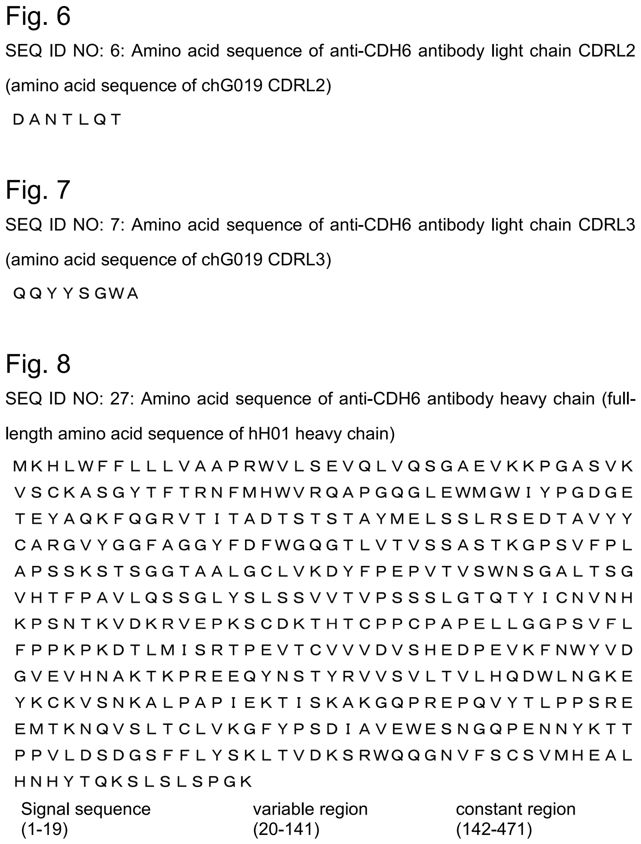

[Figure 6] Figure 6 is a diagram showing the amino acid sequence (SEQ ID NO: 6) of an anti-CDH6 antibody light chain CDRL2.

[Figure 7] Figure 7 is a diagram showing the amino acid sequence (SEQ ID NO: 7) of an anti-CDH6 antibody light chain CDRL3.

[Figure 8] Figure 8 is a diagram showing the amino acid sequence (SEQ ID NO: 27) of an anti-CDH6 antibody heavy chain.

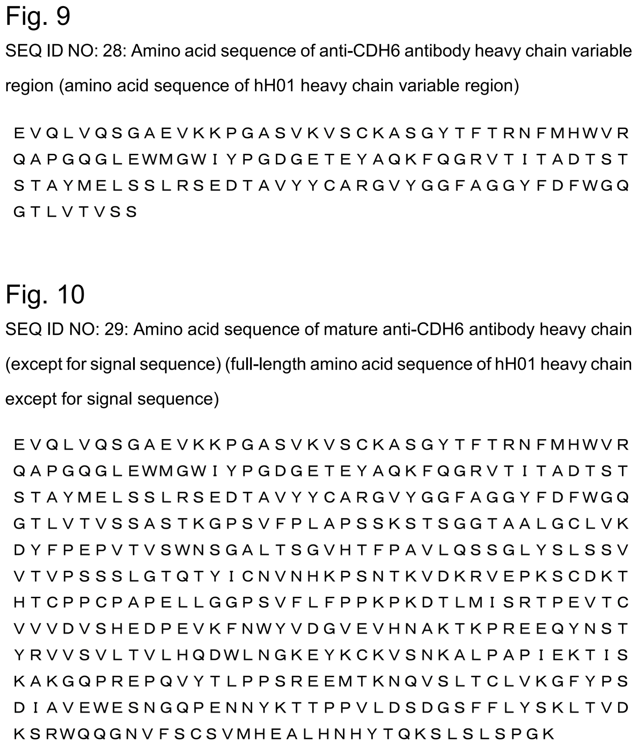

[Figure 9] Figure 9 is a diagram showing the amino acid sequence (SEQ ID NO: 28) of an anti-CDH6 antibody heavy chain variable region.

[Figure 10] Figure 10 is a diagram showing the amino acid sequence (SEQ ID NO: 29) of a mature anti-CDH6 antibody heavy chain.

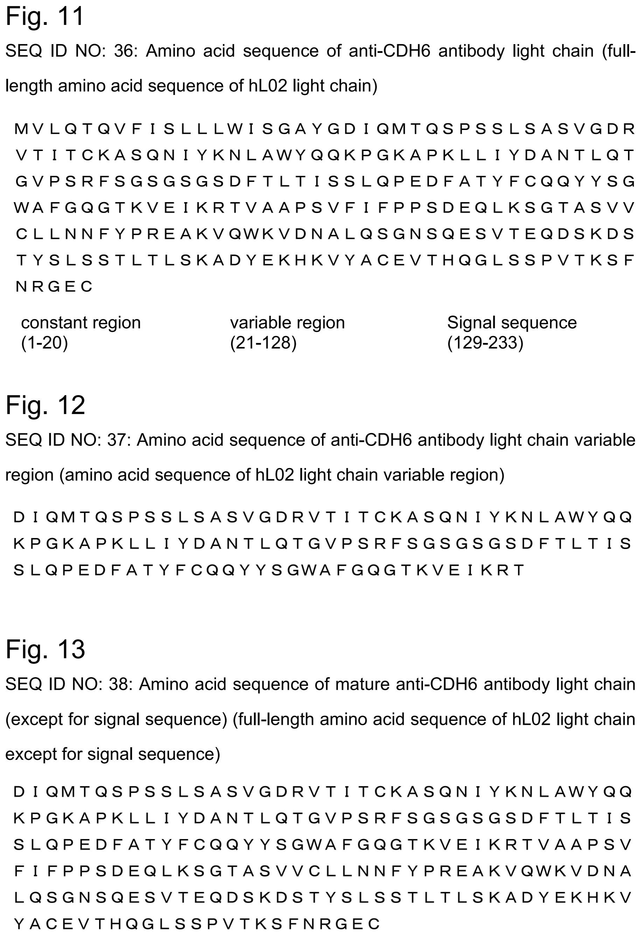

[Figure 11] Figure 11 is a diagram showing the amino acid sequence (SEQ ID NO: 36) of an anti-CDH6 antibody light chain.

[Figure 12] Figure 12 is a diagram showing the amino acid sequence (SEQ ID NO: 37) of an anti-CDH6 antibody light chain variable region.

[Figure 13] Figure 13 is a diagram showing the amino acid sequence (SEQ ID NO: 38) of a mature anti-CDH6 antibody light chain.

[Figure 14] Figure 14 is a diagram showing the tumor growth suppressing effects of respective single administration groups of an anti-CDH6 antibody-drug conjugate (1) and belzutifan (Belzutifan), and a combined administration group of the anti-CDH6 antibody-drug conjugate (1) and belzutifan (Combination), in mice with subcutaneously transplanted

786-O cells.

[Figure 15] Figure 15 is a diagram showing the percentage change in average tumor volume on Day 21, from an average tumor volume on Day 0 as a baseline, of respective single administration groups of an anti-CDH6 antibody-drug conjugate (1) and belzutifan (Belzutifan), and a combined administration group of the anti-CDH6 antibody-drug conjugate (1) and belzutifan (Combination), in mice with subcutaneously transplanted 786-O cells.

[Description of Embodiments]

Hereinafter, the preferred embodiments for carrying out the present invention will be described. It is to be noted that the embodiments described below merely illustrate representative embodiments of the present invention, and the scope of the present invention shall not be narrowly interpreted due to these examples.

1. Definition

Cadherins are glycoproteins present on the surface of cell membranes and function as cell-cell adhesion molecules through the calcium ion-dependent binding of their N-terminal extracellular domains, or as signal molecules responsible for cell-cell interactions. Classic cadherins are in the cadherin superfamily and are single-pass transmembrane proteins composed of five extracellular domains (EC domains), one transmembrane region, and an intracellular domain.

CDH6 (cadherin-6) is a single-pass transmembrane protein composed of 790 amino acids, which is classified into the type II cadherin family, and this protein has N-terminal extracellular and C-terminal intracellular domains. The human CDH6 gene was cloned for the first time in 1995 (Shimoyama Y, et al., Cancer Research, 2206-2211, 55, May 15, 1995), and its sequence can be referred to under, for example, accession Nos. NM_004932 and NP_004923 (NCBI). The amino acid sequence shown in SEQ ID NO: 1 is the amino acid sequence of extracellular domain 3 (in the present description, also referred to as EC domain 3 or EC3) of human CDH6.

In the present description, the term "cancer" is used to have the same meaning as that of the term "tumor".

In the present description, the term "gene" is used to include not only DNA but also its mRNA and cDNA, and cRNA thereof.

In the present description, the term "CDH6" can be used to have the same meaning as that of the CDH6 protein.

In the present description, the term "anti-CDH6 antibody" refers to an antibody which specifically binds to CDH6 (cadherin-6). The anti-CDH6 antibody is preferably an antibody which has the activity of being internalized into CDH6-expressing cells by binding to CDH6.

In the present description, the term "functional fragment of an antibody", also called "antigen-binding fragment of an antibody", is used to mean a partial fragment of the antibody having binding activity against an antigen, and includes Fab, F(ab')2, Fv, scFv, a diabody, a linear antibody and a multispecific antibody formed from antibody fragments, and the like. Fab', which is a monovalent fragment of antibody variable regions obtained by treating F(ab')2 under reducing conditions, is also included in the antigen-binding fragment of an antibody. However, the antigen-binding fragment of an antibody is not limited to these molecules, so long as the antigen-binding fragment has antigen-binding ability. These antigen-binding fragments include not only those obtained by treating a full-length molecule of an antibody protein with an appropriate enzyme, but proteins produced in appropriate host cells using a genetically engineered antibody gene.

In the present description, the term "CDR" is used to mean a complementarity determining region. It is known that the heavy chain and light chain of an antibody molecule each have three CDRs. Such a CDR is also referred to as a hypervariable region, and is located in the variable regions of the heavy chain and light chain of an antibody. These regions have a particularly highly variable primary structure and are separated into three sites on the primary structure of the polypeptide chain in each of the heavy chain and light chain. In the present description, with regard to the CDR of an antibody, the CDRs of a heavy chain are referred to as CDRH1, CDRH2 and CDRH3, respectively, from the amino-terminal end of the amino acid sequence of the heavy chain, whereas the CDRs of a light chain are referred to as CDRL1, CDRL2 and CDRL3, respectively, from the amino-terminal end of the amino acid sequence of the light chain. These sites are located close to one another on the three-dimensional structure, and determine the specificity of the antibody to an antigen to which the antibody binds.

In the present description, the term "approximately" refers to a value which may vary by up to plus or minus 10%, 8%, 6%, 5%, 4%, 3%, 2%, or 1% from the reference value. Preferably, the term "approximately" refers to the range of plus or minus 10%, 5%, or 1% from the reference value.

2. Anti-CDH6 antibody-drug conjugate

The anti-CDH6 antibody-drug conjugate used in the present invention is an antibody-drug conjugate in which a drug-linker represented by the formula:

[Formula 9]

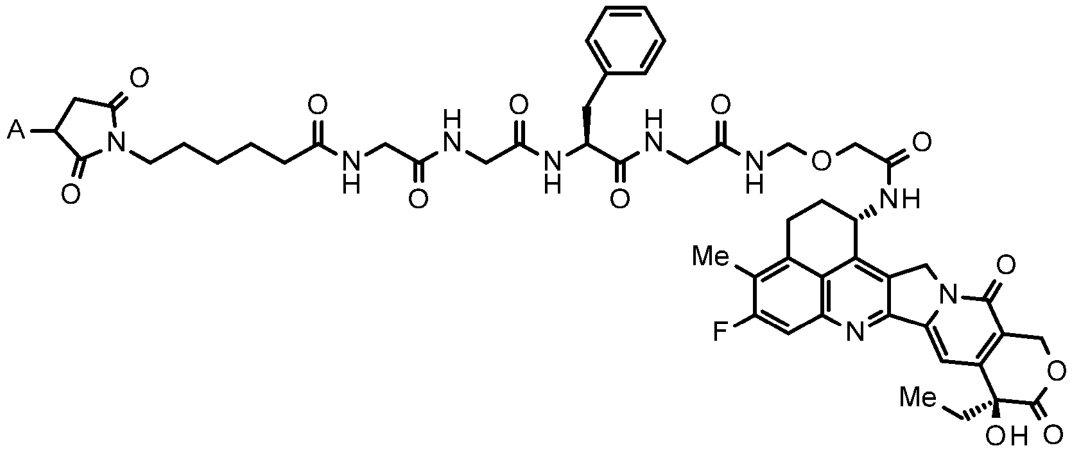

wherein A represents a connecting position to an anti-CDH6 antibody or a functional fragment of the antibody, is conjugated to the anti-CDH6 antibody or the functional fragment of the antibody via a thioether bond.

In the present invention, the partial structure consisting of a linker and a drug in the antibody-drug conjugate is referred to as a "drug-linker". This drug-linker is connected to a thiol group (in other words, the sulfur atom of a cysteine residue) formed at an interchain disulfide bond site (two sites between heavy chains and two sites between a heavy chain and a light chain) in the antibody.

The drug-linker of the present invention comprises a topoisomerase I inhibitor exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H, 12H-benzo[de]pyrano[3',4':6,7]indolizino[1,2-b]quinolin-10,13(9H,15H)-dione)) as a component. Exatecan is represented by the formula:

[Formula 10]

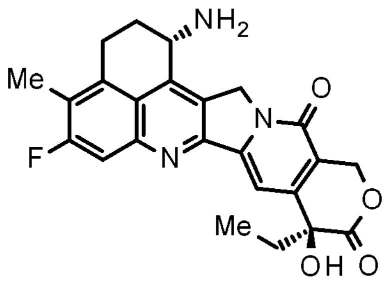

and is a camptothecin derivative having an antitumor effect.

The anti-CDH6 antibody-drug conjugate used in the present invention can also be represented by the following formula:

[Formula 11]

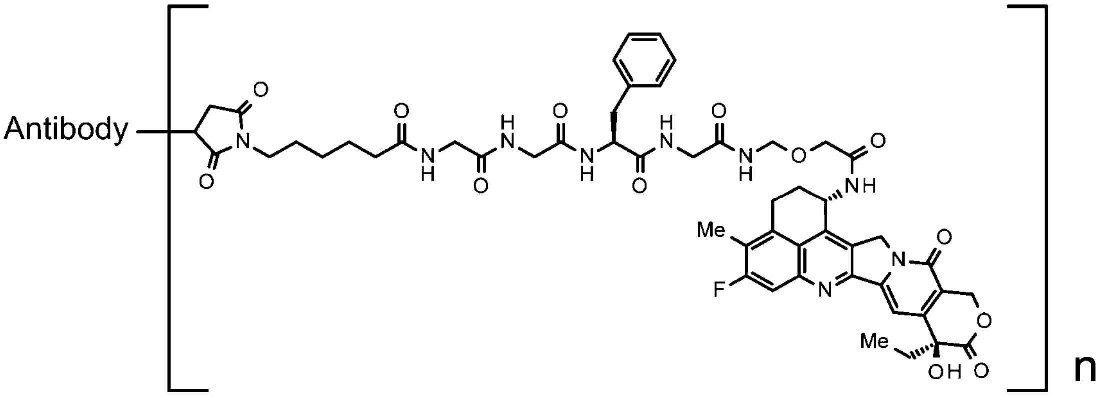

In this context, Antibody is an anti-CDH6 antibody or a functional fragment of the antibody (preferably, an anti-CDH6 antibody), and the drug-linker is conjugated to the antibody via a thioether bond (each of the drug-linkers represented by the structure shown in the parentheses in the formula is conjugated to the antibody via a thioether bond). n has the same meaning as that of the so-called average number of conjugated drug molecules (DAR; drug-to-antibody ratio), and represents the average number of units of the drug-linker conjugated per antibody.

In some embodiments, the anti-CDH6 antibody-drug conjugate is raludotatug deruxtecan (also referred to as R-DXd or DS-6000a).

After migrating into cancer cells, the antibody-drug conjugate used in the present invention is cleaved at the linker moiety to release a compound represented by the formula:

[Formula 12]

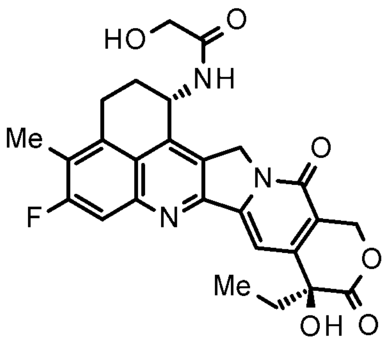

(hereinafter, referred to as a compound (A)).

The aforementioned compound is considered as the original source of the antitumor activity of the anti-CDH6 antibody-drug conjugate used in the present invention, and has been confirmed to have a topoisomerase I inhibitory effect (Ogitani Y. et al., Clinical Cancer Research, 2016, Oct 15; 22 (20): 5097-5108, Epub 2016 Mar 29).

Topoisomerase I is an enzyme that cleaves and rejoins a single strand of DNA, thereby transforming the conformation of the DNA to participate in DNA synthesis. Therefore, agents having a topoisomerase I inhibitory effect can inhibit DNA synthesis, thus arresting cell division at the S phase (DNA synthesis phase) of the cell cycle and inducing cell death by apoptosis, thereby suppressing growth of cancer cells.

The antibody-drug conjugate used in the present invention is also known to have a bystander antitumor effect (Suzuki H, et al., Molecular Cancer Therapeutics, (2024) 23(3): 257-271).

This bystander antitumor effect is exerted through a process in which the antibody-drug conjugate used in the present invention is internalized into target-expressing cancer cells where the compound is then released so as to also exert an antitumor effect on non-target-expressing cancer cells present therearound.

This bystander antitumor effect is also exerted as an excellent antitumor effect when the antibody-drug conjugate according to the present invention is used in combination with a HIF-2$\alpha$ inhibitor.

Other examples of the anti-CDH6 antibody-drug conjugate are not particularly limited so long as an antitumor compound is conjugated to an anti-CDH6 antibody via a linker structure moiety. Examples thereof can include those described in CUSP06, BSI-709, and International Publication Nos. WO 2023/102875 and WO 2023/104188.

3. Antibody in anti-CDH6 antibody-drug conjugate

The anti-CDH6 antibody in the anti-CDH6 antibody-drug conjugate used in the present invention may be derived from any species, and is preferably an anti-CDH6 antibody derived from a human, a rat, a mouse or a rabbit. When the anti-CDH6 antibody is derived from a species other than humans, it is preferred to chimerize or humanize the anti-CDH6 antibody by a well-known technique. The anti-CDH6 antibody of the present invention may be a polyclonal antibody or may be a monoclonal antibody, and a monoclonal antibody is preferred.

The anti-CDH6 antibody in the anti-CDH6 antibody-drug conjugate used in the present invention preferably has the property of being able to target cancer cells, and is preferably an antibody that possesses, for example, the property of being able to recognize cancer cells, the property of being able to bind to cancer cells, the property of being internalized into cancer cells by cellular uptake, and/or cytocidal activity against cancer cells.

The binding activity of an antibody against cancer cells can be confirmed by flow cytometry. The uptake of an antibody into cancer cells can be confirmed by (1) an assay visualizing a cellularly taken-up antibody under a fluorescent microscope using a secondary antibody (fluorescently labeled) binding to the antibody (Cell Death and Differentiation (2008) 15, 751-761), (2) an assay measuring the amount of cellularly taken-up fluorescence using a secondary antibody (fluorescently labeled) binding to the antibody (Molecular Biology of the Cell Vol. 15, 5268-5282, December 2004) or (3) a Mab-ZAP assay using an immunotoxin binding to the antibody, wherein the toxin is released upon cellular uptake, so as to suppress cell growth (Bio Techniques 28: 162-165, January 2000). A recombinant conjugated protein of a catalytic region of diphtheria toxin and protein G may be used as the immunotoxin.

The antitumor activity of the antibody can be confirmed *in vitro* by measuring inhibitory activity against cell growth. For example, a cancer cell line overexpressing the target protein of the antibody is cultured, and the antibody is added at varying concentrations into the culture system to measure inhibitory activity against focus formation, colony formation and spheroid growth. The antitumor activity can be confirmed *in vivo,* for example, by administering the antibody to a nude mouse into which a tumor cell line highly expressing the target protein has been inoculated, and then measuring a change in the cancer cells.

Since the compound conjugated in the antibody-drug conjugate exerts an antitumor effect, it is preferred, but not essential, that the antibody itself should have an antitumor effect. For the purpose of specifically and/or selectively exerting the cytotoxicity of the antitumor compound against cancer cells, it is important and preferred that the antibody should have a property of being internalized and transferred into cancer cells.

The anti-CDH6 antibody in the anti-CDH6 antibody-drug conjugate used in the present invention can be obtained by procedures known in the art. For example, the anti-CDH6 antibody can be obtained using a method usually carried out in the art, which involves immunizing an animal with an antigenic polypeptide, and then collecting and purifying an antibody produced in vivo. The origin of the antigen is not limited to a human, and, thus, an animal can also be immunized with an antigen derived from a non-human animal such as a mouse, a rat or the like. In this case, the cross-reactivity of antibodies binding to the obtained heterologous antigen with human antigens can be tested to screen for an antibody applicable to a human disease.

Alternatively, antibody-producing cells that produce an antibody against the antigen can be fused with myeloma cells according to a method known in the art (e.g., Kohler and Milstein, Nature (1975) 256, 495-497; and Kennet, R. ed., Monoclonal Antibodies, 365-367, Plenum Press, N. Y. (1980)) to establish hybridomas, so as to obtain a monoclonal antibody.

The antigen can be obtained by allowing host cells to produce a gene encoding the antigen protein according to genetic manipulation. Specifically, a vector capable of expressing the antigen gene is produced, and the vector is then introduced into host cells, so that the gene is expressed therein, and, thereafter, the expressed antigen may be purified. The antibody can also be obtained by a method of immunizing an animal with the antigen-expressing cells based on the above-described genetic manipulation, or a cell line expressing the antigen.

The anti-CDH6 antibody in the anti-CDH6 antibody-drug conjugate used in the present invention is preferably a genetically recombinant antibody that has been artificially modified for the purpose of reducing heterogenetic antigenicity to humans, such as a chimeric antibody or a humanized antibody, or is preferably an antibody having only the gene sequence of a human-derived antibody, i.e., a human antibody. These antibodies can be produced by known methods.

An example of the chimeric antibody can include antibodies in which a variable region and a constant region are heterologous to each other, such as a chimeric antibody formed by conjugating the variable region of a mouse- or rat-derived antibody to a human-derived constant region (Proc. Natl. Acad. Sci. U.S.A., 81, 6851-6855, (1984)).

Examples of the humanized antibody can include an antibody formed by incorporating only complementarity determining regions (CDRs) of a heterologous antibody into a human-derived antibody (Nature (1986) 321, p. 522-525), an antibody formed by incorporating the amino acid residues from some frameworks of a heterologous antibody, as well as CDR sequences of the heterologous antibody, into a human antibody according to a CDR grafting method (International Publication No. WO90/07861), and an antibody humanized by use of a gene conversion mutagenesis strategy (U.S. Patent No. 5821337).

Examples of the human antibody can include an antibody prepared using a human antibody-producing mouse having a human chromosomal fragment comprising the heavy chain and light chain genes of a human antibody (see Tomizuka, K. et al., Nature Genetics (1997) 16, p. 133-143; Kuroiwa, Y. et al., Nucl. Acids Res. (1998) 26, p. 3447-3448; Yoshida, H. et al., Animal Cell Technology: Basic and Applied Aspects vol. 10, p. 69-73 (Kitagawa, Y., Matsuda, T. and Iijima, S. eds.), Kluwer Academic Publishers, 1999; Tomizuka, K. et al., Proc. Natl. Acad. Sci. USA(2000) 97, p. 722-727; etc.). Another example thereof can include a phage display-derived antibody that has been selected from a human antibody library (see Wormstone, I. M. et al., Investigative Ophthalmology & Visual Science. (2002) 43 (7), p. 2301-2308; Carmen, S. et al., Briefings in Functional Genomics and Proteomics (2002), 1 (2), p. 189-203; Siriwardena, D. et al., Ophthalmology (2002) 109 (3), p. 427-431; etc.).

The anti-CDH6 antibody in the anti-CDH6 antibody-drug conjugate used in the present invention is preferably an antibody which specifically binds to the amino acid sequence shown in SEQ ID NO: 1 and has an internalization ability that permits cellular uptake. Examples of such an anti-CDH6 antibody can include antibodies described in International Publication No. WO 2018/212136 (H01L02, H02L02, H02L03, H04L02, etc.).

The phrase "specifically binds to the amino acid sequence shown in SEQ ID NO: 1" as applied to the antibody is used to mean that the antibody binds strongly to the EC3 domain of CDH6 compared with the other extracellular domains of CDH6.

The internalization activity (internalization ability) of the antibody can be evaluated using, for example, a reagent conjugated with a toxin (saporin) inhibiting protein synthesis (e.g., an anti-rat IgG reagent Rat-ZAP (Advanced Targeting Systems) and the anti-human IgG reagent Hum-ZAP (Advanced Targeting Systems)) (see International Publication No. WO 2018/212136).

The anti-CDH6 antibody in the anti-CDH6 antibody-drug conjugate used in the present invention is preferably an antibody whose survival rate (which is indicated by a ratio relative to a cell survival rate without addition of the antibody defined as 100%) of CDH6-expressing cells to which the aforementioned antibody and a saporin-labeled anti-rat IgG antibody or a saporin-labeled anti-human IgG antibody have been administered is 80% or less, more preferably 70% or less, and still more preferably 60% or less.

The anti-CDH6 antibody in the anti-CDH6 antibody-drug conjugate used in the present invention is preferably an antibody comprising

a heavy chain comprising CDRH1, CDRH2 and CDRH3 in any one combination selected from the group consisting of the following combinations (1) to (5): (1) CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4, (2) CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4, (3) CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11, (4) CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and (5) CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and

a light chain comprising CDRL1, CDRL2 and CDRL3 in any one combination selected from the group consisting of the following combinations (6) to (9): (6) CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, (7) CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14, (8) CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20, and (9) CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 2, CDRH2 consisting of the amino acid sequence shown in SEQ ID NO: 3, and CDRH3 consisting of the amino acid sequence shown in SEQ ID NO: 4, and a light chain comprising CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7.

The anti-CDH6 antibody in the anti-CDH6 antibody-drug conjugate used in the present invention is more

preferably an antibody comprising a heavy chain comprising CDRH1 consisting of the amino acid sequence shown in SEQ ID NO: 2, CDRH2 consisting of the amino acid sequence shown in SEQ ID NO: 8, and CDRH3 consisting of the amino acid sequence shown in SEQ ID NO: 4, and a light chain comprising CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 9, CDRH2 consisting of the amino acid sequence shown in SEQ ID NO: 10, and CDRH3 consisting of the amino acid sequence shown in SEQ ID NO: 11, and a light chain comprising CDRL1 consisting of the amino acid sequence shown in SEQ ID NO: 12, CDRL2 consisting of the amino acid sequence shown in SEQ ID NO: 13, and CDRL3 consisting of the amino acid sequence shown in SEQ ID NO: 14.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 15, CDRH2 consisting of the amino acid sequence shown in SEQ ID NO: 16, and CDRH3 consisting of the amino acid sequence shown in SEQ ID NO: 17, and a light chain comprising CDRL1 consisting of the amino acid sequence shown in SEQ ID NO: 18, CDRL2 consisting of the amino acid sequence shown in SEQ ID NO: 19, and CDRL3 consisting of the amino acid sequence shown in SEQ ID NO: 20.

The anti-CDH6 antibody in the anti-CDH6 antibody-drug conjugate used in the present invention is more preferably an antibody comprising a heavy chain comprising CDRH1 consisting of the amino acid sequence shown in SEQ ID NO: 21, CDRH2 consisting of the amino acid sequence shown in SEQ ID NO: 22, and CDRH3 consisting of the amino acid sequence shown in SEQ ID NO: 23, and a light chain comprising CDRL1 consisting of the amino acid sequence shown in SEQ ID NO: 24, CDRL2 consisting of the amino acid sequence shown in SEQ ID NO: 25, and CDRL3 consisting of the amino acid sequence shown in SEQ ID NO: 26.

The anti-CDH6 antibody in the anti-CDH6 antibody-drug conjugate used in the present invention can be still more preferably an antibody comprising a given combination of a heavy chain comprising any one heavy chain variable region selected from the group consisting of the following variable regions (1) to (3): (1) the amino acid sequence shown in SEQ ID NO: 28, 31 or 34, (2) an amino acid sequence having an identity of at least 95% or more to the amino acid sequence (1) (preferably an amino acid sequence having a sequence identity of at least 95% or more to the sequence of the framework regions other than at each CDR sequence), and (3) an amino acid sequence comprising a deletion, substitution or addition of one or several amino acids in the amino acid sequence (1), and a light chain comprising any one light chain variable region selected from the group consisting of the following variable regions (4) to (6): (4) the amino acid sequence shown in SEQ ID NO: 37 or 40, (5) an amino acid sequence having an identity of at least 95% or more to the amino acid sequence (4) (preferably an amino acid sequence having a sequence identity of at least 95% or more to the sequence of the framework regions other than at each CDR sequence), and (6) an amino acid sequence comprising a deletion, substitution or addition of one or several amino acids in the amino acid sequence (4).

The anti-CDH6 antibody in the anti-CDH6 antibody-drug conjugate used in the present invention is still more preferably an antibody comprising a heavy chain comprising the heavy chain variable region amino acid sequence shown in SEQ ID NO: 28, a heavy chain comprising the heavy chain variable region amino acid sequence shown in SEQ ID NO: 31, or a heavy chain comprising the heavy chain variable region amino acid sequence shown in SEQ ID NO: 34, and a light chain comprising the light chain variable region amino acid sequence shown in SEQ ID NO: 37 or the light chain having a light chain variable region amino acid sequence shown in SEQ ID NO: 40.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 28 and a light chain comprising a light chain variable region consisting of the amino acid sequence shown in SEQ ID NO: 37.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 31 and a light chain comprising a light chain variable region consisting of the amino acid sequence shown in SEQ ID NO: 37.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 34 and a light chain comprising a light chain variable region consisting of the amino acid sequence shown in SEQ ID NO: 37.

The anti-CDH6 antibody in the anti-CDH6 antibody-drug conjugate used in the present invention is still more preferably an antibody comprising a heavy chain comprising a heavy chain variable region consisting of the amino acid sequence shown in SEQ ID NO: 31 and a light chain comprising a light chain variable region consisting of the amino acid sequence shown in SEQ ID NO: 40.

The anti-CDH6 antibody in the anti-CDH6 antibody-drug conjugate used in the present invention is still more

preferably an antibody comprising a heavy chain consisting of the amino acid sequence shown in SEQ ID NO: 29 and a light chain consisting of the amino acid sequence shown in SEQ ID NO: 38, or an antibody in which a lysine residue is deleted from the carboxyl terminus of a heavy chain of the antibody.

The anti-CDH6 antibody in the anti-CDH6 antibody-drug conjugate used in the present invention is still more preferably an antibody comprising a heavy chain consisting of the amino acid sequence shown in SEQ ID NO: 32 and a light chain consisting of the amino acid sequence shown in SEQ ID NO: 38, or an antibody in which a lysine residue is deleted from the carboxyl terminus of a heavy chain of the antibody.

The anti-CDH6 antibody in the anti-CDH6 antibody-drug conjugate used in the present invention is still more preferably an antibody comprising a heavy chain consisting of the amino acid sequence shown in SEQ ID NO: 35 and a light chain consisting of the amino acid sequence shown in SEQ ID NO: 38, or an antibody in which a lysine residue is deleted from the carboxyl terminus of a heavy chain of the antibody.

The anti-CDH6 antibody in the anti-CDH6 antibody-drug conjugate used in the present invention is still more preferably an antibody comprising a heavy chain consisting of the amino acid sequence shown in SEQ ID NO: 32 and a light chain consisting of the amino acid sequence shown in SEQ ID NO: 41, or an antibody in which a lysine residue is deleted from the carboxyl terminus of a heavy chain of the antibody.

By combining together sequences showing a high identity to the above-described heavy chain amino acid sequences and light chain amino acid sequences, it is possible to select an antibody having a biological activity equivalent to that of each of the above-described antibodies. Such an identity is an identity of generally 80% or more, preferably an identity of 85% or more, more preferably an identity of 90% or more, still more preferably an identity of 95% or more, and most preferably an identity of 99% or more.

The identity between two types of amino acid sequences can be determined by aligning the sequences using the default parameters of Clustal W version 2 (Larkin MA, Blackshields G, Brown NP, Chenna R, McGettigan PA, McWilliam H, Valentin F, Wallace IM, Wilm A, Lopez R, Thompson JD, Gibson TJ and Higgins DG (2007), "Clustal W and Clustal X version 2.0", Bioinformatics. 23 (21): 2947-2948).

The antibody in the anti-CDH6 antibody-drug conjugate used in the present invention also includes a modification of an antibody. The "modification" is used to mean the antibody according to the present invention, which is chemically or biologically modified. Examples of such a chemical modification include chemical modifications such as the binding of a chemical moiety to an amino acid skeleton or the binding of a chemical moiety to an N-linked or O-linked carbohydrate chain. Examples of such a biological modification include antibodies which have undergone a posttranslational modification (e.g., N-linked or O-linked glycosylation, N-terminal or C-terminal processing, deamidation, isomerization of aspartic acid, and oxidation of methionine), and antibodies to the N-terminus of which a methionine residue is added as a result of having been allowed to be expressed using prokaryote host cells. In addition, such a modification is also meant to include labeled antibodies for enabling detection or isolation of the antibody according to the present invention or an antigen, for example, an enzymatically labeled antibody, a fluorescently labeled antibody, and an affinity-labeled antibody. Such a modification of the antibody according to the present invention is useful for the improvement of the stability and retention in blood of an antibody; a reduction in antigenicity; detection or isolation of an antibody or an antigen; etc.

Moreover, by regulating a sugar chain modification (glycosylation, de-fucosylation, etc.) that binds to the antibody according to the present invention, antibody-dependent cellular cytotoxic activity can be enhanced. As techniques of regulating the sugar chain modification of an antibody, those described in International Publication Nos. WO 99/54342, WO 00/61739, WO 02/31140, WO 2007/133855, and WO 2013/120066, etc. are known, though the techniques are not limited thereto. The antibody according to the present invention also includes antibodies in respect of which the aforementioned sugar chain modification has been regulated.

It is known that the lysine residue at the carboxyl terminus of the heavy chain of an antibody produced in cultured mammalian cells is deleted (Journal of Chromatography A, 705: 129-134 (1995)), and it is also known that the two amino acid residues at the carboxyl terminus of the heavy chain, glycine and lysine, are deleted, and that the proline residue newly positioned at the carboxyl terminus is amidated (Analytical Biochemistry, 360: 75-83 (2007)). However, such deletion and modification of these heavy chain sequences does not have an influence on the antigen-binding activity and effector function (activation of complement, antibody-dependent cellular cytotoxicity, etc.) of an antibody. Accordingly, the antibody according to the present invention also includes an antibody that has undergone the aforementioned modification, and a functional fragment of the antibody, and specific examples of such an antibody include a deletion mutant comprising a deletion of 1 or 2 amino acids at the carboxyl terminus of the heavy chain, and a deletion mutant formed by amidating the aforementioned deletion mutant (e.g., a heavy chain in which the proline residue at the carboxyl-terminal site is amidated). However, deletion mutants involving a deletion at the carboxyl terminus of the heavy chain of the antibody according to the present invention are not limited to the above-described deletion mutants, so long as they retain antigen-binding activity and effector function. The two heavy chains constituting the antibody according to the present invention may be any one type of heavy chain selected from the group consisting of a full-length antibody and the above-described deletion mutants, or may be a combination of any two types selected from the aforementioned group. The ratio of individual deletion mutants can be influenced by the types of cultured mammalian cells that produce the antibody according to the present invention,

and the culture conditions. Examples of the antibody according to the present invention can preferably include antibodies where one amino acid residue is deleted at the carboxyl terminus of each of the two heavy chains. The amino acid deleted at the carboxyl terminus of the heavy chain in the antibody according to the present invention is preferably a lysine residue.

Examples of the isotype of the antibody according to the present invention can include IgG (IgG1, IgG2, IgG3, and IgG4). Among others, IgG1, IgG2, and IgG4 are preferable.

In the full-length hH01 heavy chain amino acid sequence shown in SEQ ID NO: 27, the amino acid sequence consisting of the amino acid residues at positions 1 to 19 is the signal sequence, the amino acid sequence consisting of the amino acid residues at positions 20 to 141 is the variable region, and the amino acid sequence consisting of the amino acid residues at positions 142 to 471 is the constant region.

In the full-length hH02 heavy chain amino acid sequence shown in SEQ ID NO: 30, the amino acid sequence consisting of the amino acid residues at positions 1 to 19 is the signal sequence, the amino acid sequence consisting of the amino acid residues at positions 20 to 141 is the variable region, and the amino acid sequence consisting of the amino acid residues at positions 142 to 471 is the constant region.

In the full-length hH04 heavy chain amino acid sequence shown in SEQ ID NO: 33, the amino acid sequence consisting of the amino acid residues at positions 1 to 19 is the signal sequence, the amino acid sequence consisting of the amino acid residues at positions 20 to 141 is the variable region, and the amino acid sequence consisting of the amino acid residues at positions 142 to 471 is the constant region.

In the full-length hL02 light chain amino acid sequence shown in SEQ ID NO: 36, the amino acid sequence consisting of the amino acid residues at positions 1 to 20 is the signal sequence, the amino acid sequence consisting of the amino acid residues at positions 21 to 128 is the variable region, and the amino acid sequence consisting of the amino acid residues at positions 129 to 233 is the constant region.

In the full-length hL03 light chain amino acid sequence shown in SEQ ID NO: 39, the amino acid sequence consisting of the amino acid residues at positions 1 to 20 is the signal sequence, the amino acid sequence consisting of the amino acid residues at positions 21 to 128 is the variable region, and the amino acid sequence consisting of the amino acid residues at positions 129 to 233 is the constant region.

4. Production of anti-CDH6 antibody-drug conjugate

The drug-linker intermediate for use in the production of the anti-CDH6 antibody-drug conjugate according to the present invention is represented by the following formula:

[Formula 13]

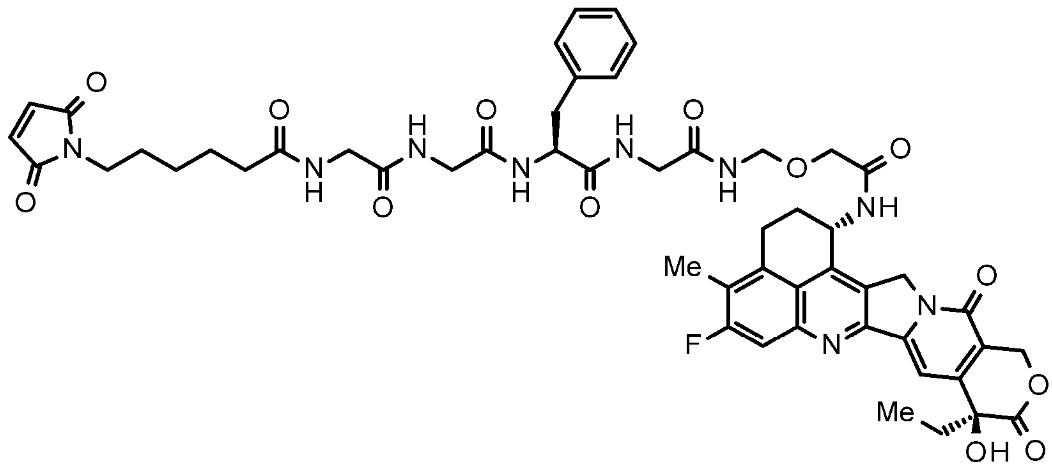

The above-described drug-linker intermediate can be represented by the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to the description of International Publication Nos. WO 2014/057687, WO 2015/098099, WO 2015/115091, WO 2015/155998, and WO 2019/044947, etc.

The anti-CDH6 antibody-drug conjugate used in the present invention can be produced by reacting the above-described drug-linker intermediate with an anti-CDH6 antibody having a thiol group (or also referred to as a sulfhydryl group).

The anti-CDH6 antibody having a sulfhydryl group can be obtained by a method well known to a person skilled in

the art (Hermanson, G.T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the anti-CDH6 antibody in a buffer solution containing a chelating agent such as ethylenediamine tetraacetic acid (EDTA), an anti-CDH6 antibody having a sulfhydryl group with partially or completely reduced interchain disulfides within the antibody can be obtained.

Further, using 2 to 20 molar equivalents of the drug-linker intermediate per anti-CDH6 antibody having a sulfhydryl group, the anti-CDH6 antibody-drug conjugate in which 2 to 8 drug molecules are conjugated per anti-CDH6 antibody can be produced.

The average number of conjugated drug molecules per antibody molecule in the produced anti-CDH6 antibody-drug conjugate can be calculated by, for example, a calculation method comprising measuring UV absorbance of the antibody-drug conjugate and a conjugation precursor thereof at two wavelengths of 280 nm and 370 nm (UV method), or a calculation method comprising treating the antibody-drug conjugate with a reducing agent and quantifying each resulting fragment by HPLC measurement (HPLC method).

The conjugation between the antibody with the drug-linker intermediate and the calculation of the average number of conjugated drug molecules per antibody molecule in the antibody-drug conjugate can be carried out with reference to the description of International Publication Nos. WO 2014/057687, WO 2015/098099, WO 2015/115091, WO 2015/155998, WO 2018/135501, and WO 2018/212136, etc.

In some embodiments, the average number of units of the drug-linker conjugated per antibody molecule in the anti-CDH6 antibody-drug conjugate according to the present invention is preferably 1 to 10, more preferably 2 to 8, still more preferably 5 to 8, still more preferably 7 to 8, still more preferably 7.5 to 8, and still more preferably approximately 8.

In other embodiments, the number of the drug or the drug-linker conjugated per antibody molecule in the anti-CDH6 antibody-drug conjugate according to the present invention is an integer in the range from preferably 2 to 8, more preferably selected from 2, 4, 6, or 8, and still more preferably 8.

The anti-CDH6 antibody-drug conjugate can be produced with reference to the description of International Publication No. WO 2018/212136, etc.

5. Hypoxia-inducible factor-2α (HIF-2α) inhibitor

In the present invention, the term "HIF-2α activity" refers to physiological and/or biological activity mediated by HIF-2α. Examples thereof can include activity of gene transcription mediated by HIF-2α.

In the present invention, the term "HIF-2α inhibitor" refers to an agent that delays, reduces, changes, completely eliminates and/or hinders HIF-2α activity (e.g., transcriptional activity of HIF-2α).

The HIF-2α inhibitor according to the present invention is not particularly limited so long as the agent has the above-described properties. The HIF-2α inhibitor may be a low-molecular-weight compound, an antibody, a functional fragment of the antibody, a fusion protein, an immunoadhesin, a nucleic acid, an oligonucleotide, an aptamer, or a polypeptide, and is preferably a low-molecular-weight compound.

In the present invention, the low-molecular-weight compound is an organic compound having a molecular weight of 1000 or less, more preferably a molecular weight of 900 or less, still more preferably a molecular weight of 800 or less, still more preferably a molecular weight of 700 or less, still more preferably a molecular weight of 600 or less, still more preferably a molecular weight of 500 or less, still more preferably a molecular weight of 50 to 500, and yet more preferably a molecular weight of 100 to 500.

The HIF-2α inhibitor according to the present invention can preferably be belzutifan (also referred to as MK-6482 or PT2977), NKT2152 (see, for example, the following literature A), DFF332 (see, for example, the following literature B), AB521 (see, for example, the following literature C), BPI-452080 (see, for example, the following literature D), PT2385, or PT2399 or a pharmaceutically acceptable salt thereof. More preferably, the HIF-2α inhibitor can be belzutifan, NKT2152, DFF332, AB521, or BPI-452080 or a pharmaceutically acceptable salt thereof. Still more preferably, the HIF-2α inhibitor can be belzutifan or NKT2152 or a pharmaceutically acceptable salt thereof. Still more preferably, the HIF-2α inhibitor can be belzutifan or a pharmaceutically acceptable salt thereof.

A: A Study of NKT2152, a HIF2α Inhibitor, in Patients With Advanced Clear Cell Renal Cell Carcinoma, [online], November 15, 2021 (First Posted), National Library of Medicine (National Institutes of Health), [retrieved on February 10, 2023], internet <URL: clinicaltrials.gov/ct2/show/NCT05119335>

B: DFF332 as a Single Agent and in Combination With Everolimus & Immuno-Oncology Agents in Advanced/Relapsed Renal Cancer & Other Malignancies, [online], May 20, 2021 (First Posted), National Library of Medicine (National Institutes of Health), [retrieved on February 10, 2023], Internet <URL: clinicaltrials.gov/ct2/-show/NCT04895748>

C: A Phase 1 Study of AB521 in Renal Cell Carcinoma and Other Solid Tumors (ARC-20), [online], September 10, 2022 (First Posted), National Library of Medicine (National Institutes of Health), [retrieved on February 10, 2023],

Internet<URL: clinicaltrials.gov/ct2/show/NCT05536141>
D: Wang et al., Cancer Res (2023) 83 (7_Supplement): 494.

Belzutifan refers to a compound represented by the following formula:

[Formula 14]

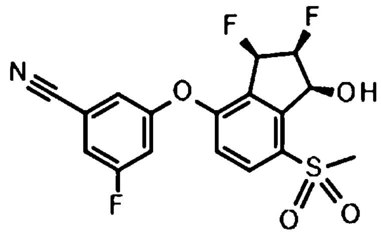

(Non Patent Literature 3).

The "pharmaceutically acceptable salt" of the HIF-2α inhibitor used in the present invention may be either an acid addition salt or a base addition salt. Examples of the acid addition salt can include lower alkanesulfonates such as camsylate (camphorsulfonate), mesylate (methanesulfonate), trifluoromethanesulfonate, and ethanesulfonate; arylsulfonates such as tosylate (p-toluenesulfonate) and benzenesulfonate; inorganic acid salts such as phosphate, nitrate, perchlorate, and sulfate; hydrogen halide salts such as hydrochloride, hydrobromide, hydroiodide, and hydrofluoride; organic acid salts such as acetate, malate, fumarate, succinate, citrate, tartrate, oxalate, and maleate; and amino acid salts such as ornithinate, glutamate, and aspartate. Examples of the base addition salt can include alkali metal salts such as sodium salt, potassium salt, and lithium salt; alkali earth metal salts such as calcium salt and magnesium salt; inorganic salts such as ammonium salt; organic amine salts such as dibenzylamine salt, morpholine salt, phenylglycine alkyl ester salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt, and tris(hydroxymethyl)aminomethane salt; and amino acid salts such as alginate.

The HIF-2α inhibitor and the pharmaceutically acceptable salt thereof used in the present invention may be present as solvates. These solvates are also included in the HIF-2α inhibitor and the pharmaceutically acceptable salt thereof used in the present invention.

The pharmaceutical composition of the present invention can be administered as a pharmaceutical composition containing a pharmaceutically acceptable carrier, diluent, solubilizer, emulsifier, preservative, aid, and the like. The "pharmaceutically acceptable carrier" and the like can be appropriately selected from a wide range depending on the type of target disease or the dosage form of the agent. The "pharmaceutically acceptable carrier" and the like include, for example, sterilized liquid. Herein, the liquid includes, for example, water and oil (petroleum oil and oil of animal origin, plant origin, or synthetic origin). The oil may be, for example, peanut oil, soybean oil, mineral oil, or sesame oil. Water is a more typical liquid when the pharmaceutical composition above is administered intravenously. Saline solution, an aqueous dextrose solution, and an aqueous glycerol solution can also be used as the liquid, in particular, for an injection solution. A suitable pharmaceutical vehicle can be selected from ones known in the art. Examples of suitable pharmaceutically acceptable carriers are disclosed in "Remington's Pharmaceutical Sciences" by E. W. Martin. The formulations correspond to the mode of administration.

An administration method can be appropriately selected. For example, administration by injection can be performed, and local injection, intraperitoneal injection, selective intravenous injection, intravenous injection, subcutaneous injection, organ perfusate injection, or the like can be adopted. The administration can be made by injection or bolus injection, for example. A solution for injection can be formulated using a carrier formed from a salt solution, a glucose solution, or a mixture of salt water and a glucose solution, various buffer solutions, and the like. Alternatively, the solution for injection may be prepared by mixing a formulation in a powder state with the liquid carrier upon use. According to a specific preferred embodiment, the administration of the pharmaceutical composition used in the present invention is performed by injection. Parenteral administration is a preferred administration route.

Other administration methods can also be appropriately selected along with the development of a formulation. In the case of oral administration, for example, an oral solution, a powder, a pill, a capsule, and a tablet are applicable. The oral solution can be produced as an oral liquid preparation such as a suspension and a syrup using, for example: water; sugars such as sucrose, sorbitol, and fructose; glycols such as polyethylene glycol; oils such as sesame oil and soybean oil; antiseptics such as alkyl p-hydroxybenzoate; and flavors such as a strawberry flavor and peppermint. The powder, the

pill, the capsule, and the tablet can be formulated using, for example: an excipient such as lactose, glucose, sucrose, or mannitol; a disintegrant such as starch or sodium alginate; a lubricant such as magnesium stearate or talc; a binder such as polyvinyl alcohol, hydroxypropylcellulose, or gelatin; a surfactant such as a fatty acid ester; and/or a plasticizer such as glycerin. The tablet and the capsule are preferred unit dosage forms because of the ease of administration. A solid pharmaceutical carrier is used in producing the tablet or the capsule.

6. Medicament

Hereinafter, the pharmaceutical composition and the method of treatment according to the present invention will be described, wherein an anti-CDH6 antibody-drug conjugate and a HIF-2α inhibitor are administered in combination. The present invention includes a pharmaceutical composition comprising an anti-CDH6 antibody-drug conjugate to be used in combination with a HIF-2α inhibitor or administered in combination with a HIF-2α inhibitor.

In the pharmaceutical composition and the method of treatment of the present invention, the anti-CDH6 antibody-drug conjugate and the HIF-2α inhibitor may be separately contained as active components in different formulations, and be administered at the same time (a person skilled in the art would naturally understand that "at the same time" may be or may not be at approximately the same time) or at different times, or the anti-CDH6 antibody-drug conjugate and the HIF-2α inhibitor may be contained as active components in a single formulation for administration.

In the pharmaceutical composition and the method of treatment of the present invention, two or more types of HIF-2α inhibitors used in the present invention may be combined for administration.

The pharmaceutical composition and the method of treatment of the present invention can be used for treating cancer, can preferably be used for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease, can more preferably be used for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, and neuroblastoma, can still more preferably be used for treating at least one cancer selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, and ovarian mucinous tumor, and can still more preferably be used for treating at least one cancer selected from the group consisting of renal cell carcinoma, renal clear cell carcinoma, and papillary renal cell carcinoma.

The cancer related to von Hippel-Lindau disease is preferably renal cell carcinoma related to von Hippel-Lindau disease, hemangioblastoma of the central nervous system related to von Hippel-Lindau disease, or pancreatic neuroendocrine tumor related to von Hippel-Lindau disease, and more preferably renal cell carcinoma related to von Hippel-Lindau disease (the renal cell carcinoma does not need immediate surgery), hemangioblastoma of the central nervous system related to von Hippel-Lindau disease (the hemangioblastoma of the central nervous system does not need immediate surgery), or pancreatic neuroendocrine tumor related to von Hippel-Lindau disease (the pancreatic neuroendocrine tumor does not need immediate surgery).

The pharmaceutical composition and the method of treatment of the present invention can be used for treating cancer having a mutation in the VHL gene, and can preferably be used for treating renal cell carcinoma, renal clear cell carcinoma, or papillary renal cell carcinoma (the renal cell carcinoma, the renal clear cell carcinoma, or the papillary renal cell carcinoma has a mutation in the VHL gene).

The pharmaceutical composition and the method of treatment of the present invention can be used for treating cancer having no mutation in the VHL gene, and can preferably be used for treating renal cell carcinoma, renal clear cell carcinoma, or papillary renal cell carcinoma (the renal cell carcinoma, the renal clear cell carcinoma, or the papillary renal cell carcinoma has no mutation in the VHL gene).

The VHL gene is a gene encoding a von Hippel-Lindau tumor suppressor (also referred to as pVHL).

The anti-CDH6 antibody-drug conjugate used in the present invention can be preferably used when CDH6 expression is confirmed in cancer.

The presence or absence of a tumor marker of CDH6 can be confirmed, for example, by collecting tumor tissues from a cancer patient, preparing formalin-fixed paraffin-embedded (FFPE) samples, and conducting a test at the gene product (protein) level by immunohistochemistry (IHC), a flow cytometer, Western blot, or the like, or a test at the gene transcription level by *in situ* hybridization (ISH), quantitative PCR (q-PCR), microarray analysis, or the like, or can also be

confirmed by collecting cell-free blood circulating tumor DNA (ctDNA) from a cancer patient, and conducting a test using a next-generation sequencer (NGS) or the like.

The pharmaceutical composition and the method of treatment of the present invention can preferably be used for a mammal, and can more preferably be used for a human.

The antitumor effect of the pharmaceutical composition and method of treatment of the present invention can be confirmed by, for example, generating a model in which cancer cells are transplanted to a test animal, and measuring reduction in tumor volume or life-prolonging effects due to applying the pharmaceutical composition and method of treatment of the present invention. Furthermore, comparison with the antitumor effect of single administrations of each of the antibody-drug conjugate and the HIF-2$\alpha$ inhibitor used in the present invention can provide confirmation of the combined effect of the antibody-drug conjugate and the HIF-2$\alpha$ inhibitor used in the present invention.

In addition, the antitumor effect of the pharmaceutical composition and method of treatment of the present invention can be confirmed, in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS).

The foregoing methods can provide confirmation of superiority in terms of the antitumor effect of the pharmaceutical composition and method of treatment of the present invention compared to existing pharmaceutical compositions and methods of treatment for cancer therapy.

The pharmaceutical composition and method of treatment of the present invention can retard growth of cancer cells, suppress their proliferation, and further can kill cancer cells. These effects can allow cancer patients to be free from symptoms caused by cancer or can achieve an improvement in the QOL of cancer patients and attain a therapeutic effect by sustaining the lives of the cancer patients. Even if the pharmaceutical composition or the method of treatment does not accomplish killing cancer cells, it can provide higher QOL of cancer patients while achieving longer-term survival, by inhibiting or controlling the growth of cancer cells.

The pharmaceutical composition of the present invention can acquire a therapeutic effect by application as systemic therapy to patients, and additionally, by local application to cancer tissues.

The pharmaceutical composition of the present invention may be administered as a composition comprising one or more pharmaceutically compatible components. The pharmaceutically compatible components can be appropriately selected, for use, from pharmaceutical additives and the like that are usually used in the art, depending on the doses, administration concentrations, etc. of the antibody-drug conjugate and the HIF-2$\alpha$ inhibitor used in the present invention. For example, the antibody-drug conjugate used in the present invention may be administered as a pharmaceutical composition containing a buffer such as a histidine buffer, an excipient such as sucrose or trehalose, and a surfactant such as polysorbate 80 or 20. The pharmaceutical composition comprising the anti-CDH6 antibody-drug conjugate used in the present invention can be preferably used as an injection, can more preferably be used as an aqueous injection or a freeze-dried injection, and can still more preferably be used as a freeze-dried injection.

In the case of an aqueous injection, the pharmaceutical composition comprising the anti-CDH6 antibody-drug conjugate used in the present invention can be preferably diluted with an appropriate diluent, and then administered by intravenous drip. Examples of the diluent can include glucose solutions and physiological saline, can preferably include glucose solutions, and can more preferably include 5% glucose solutions.

In the case of a freeze-dried injection, the pharmaceutical composition comprising the anti-CDH6 antibody-drug conjugate used in the present invention can preferably be dissolved in injectable water, then diluted in a necessary amount with an appropriate diluent, and then administered by intravenous drip. Examples of the diluent can include glucose solutions and physiological saline, can preferably include glucose solutions, and can more preferably include 5% glucose solutions.

Examples of the administration route that may be used for administering the pharmaceutical composition of the present invention can include intravenous, intradermal, subcutaneous, intramuscular, and intraperitoneal routes, and can preferably include intravenous routes.

The anti-CDH6 antibody-drug conjugate used in the present invention can be administered to a human once at intervals of 1 to 180 days, can be preferably administered once a week, once every 2 weeks, once every 3 weeks, once every 4 weeks, once every 5 weeks, once every 6 weeks, once every 7 weeks, once every 8 weeks, once every 9 weeks or once every 10 weeks, and can more preferably be administered once every 3 weeks or once every 4 weeks, and can still more preferably be once every 3 weeks.

The anti-CDH6 antibody-drug conjugate used in the present invention can be administered at a dose of approximately 0.001 to 100 mg/kg per administration, and can be administered at preferably approximately 0.1 to approximately 15 mg/kg, more preferably approximately 0.5 to approximately 12 mg/kg, still more preferably approximately 1.0 to approximately 10 mg/kg, still more preferably approximately 1.6 to approximately 9.6 mg/kg, and still more preferably approximately 4.8 to approximately 8.0 mg/kg per administration.

The anti-CDH6 antibody-drug conjugate used in the present invention can be administered at approximately 0.1,

approximately 0.2, approximately 0.3, approximately 0.4, approximately 0.5, approximately 0.6, approximately 0.7, approximately 0.8, approximately 0.9, approximately 1.0, approximately 1.1, approximately 1.2, approximately 1.3, approximately 1.4, approximately 1.5, approximately 1.6, approximately 1.7, approximately 1.8, approximately 1.9, approximately 2.0, approximately 2.1, approximately 2.2, approximately 2.3, approximately 2.4, approximately 2.5, approximately 2.6, approximately 2.7, approximately 2.8, approximately 2.9, approximately 3.0, approximately 3.1, approximately 3.2, approximately 3.3, approximately 3.4, approximately 3.5, approximately 3.6, approximately 3.7, approximately 3.8, approximately 3.9, approximately 4.0, approximately 4.1, approximately 4.2, approximately 4.3, approximately 4.4, approximately 4.5, approximately 4.6, approximately 4.7, approximately 4.8, approximately 4.9, approximately 5.0, approximately 5.1, approximately 5.2, approximately 5.3, approximately 5.4, approximately 5.5, approximately 5.6, approximately 5.7, approximately 5.8, approximately 5.9, approximately 6.0, approximately 6.1, approximately 6.2, approximately 6.3, approximately 6.4, approximately 6.5, approximately 6.6, approximately 6.7, approximately 6.8, approximately 6.9, approximately 7.0, approximately 7.1, approximately 7.2, approximately 7.3, approximately 7.4, approximately 7.5, approximately 7.6, approximately 7.7, approximately 7.8, approximately 7.9, approximately 8.0, approximately 8.1, approximately 8.2, approximately 8.3, approximately 8.4, approximately 8.5, approximately 8.6, approximately 8.7, approximately 8.8, approximately 8.9, approximately 9.0, approximately 9.1, approximately 9.2, approximately 9.3, approximately 9.4, approximately 9.5, approximately 9.6, approximately 9.7, approximately 9.8, approximately 9.9, approximately 10.0, approximately 10.1, approximately 10.2, approximately 10.3, approximately 10.4, approximately 10.5, approximately 10.6, approximately 10.7, approximately 10.8, approximately 10.9, approximately 11.0, approximately 11.1, approximately 11.2, approximately 11.3, approximately 11.4, approximately 11.5, approximately 11.6, approximately 11.7, approximately 11.8, approximately 11.9, or approximately 12.0 mg/kg or more per administration.

The anti-CDH6 antibody-drug conjugate used in the present invention can be preferably administered at approximately 1.6 mg/kg, approximately 3.2 mg/kg, approximately 4.8 mg/kg, approximately 5.4 mg/kg, approximately 5.6 mg/kg, approximately 6.4 mg/kg, approximately 8.0 mg/kg or approximately 9.6 mg/kg per administration, can more preferably be administered at approximately 3.2 mg/kg, approximately 4.8 mg/kg, approximately 5.4 mg/kg, approximately 5.6 mg/kg, approximately 6.4 mg/kg, approximately 8.0 mg/kg or approximately 9.6 mg/kg, and can still more preferably be administered at approximately 4.8 mg/kg, approximately 5.4 mg/kg, approximately 5.6 mg/kg, approximately 6.4 mg/kg or approximately 8.0 mg/kg.

The anti-CDH6 antibody-drug conjugate used in the present invention can be administered at a dose of 0.001 to 100 mg/kg per administration, and can preferably be administered at 0.1 to 15 mg/kg, more preferably 0.5 to 12 mg/kg, still more preferably 1.0 to 10 mg/kg, still more preferably 1.6 to 9.6 mg/kg, and still more preferably 4.8 to 8.0 mg/kg per administration.

The anti-CDH6 antibody-drug conjugate used in the present invention can be administered at 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, or 12.0 mg/kg or more per administration.

The anti-CDH6 antibody-drug conjugate used in the present invention can be preferably administered at 1.6 mg/kg, 3.2 mg/kg, 4.8 mg/kg, 5.4 mg/kg, 5.6 mg/kg, 6.4 mg/kg, 8.0 mg/kg or 9.6 mg/kg per administration, can more preferably be administered at 3.2 mg/kg, 4.8 mg/kg, 5.4 mg/kg, 5.6 mg/kg, 6.4 mg/kg or 8.0 mg/kg, and can still more preferably be administered at 4.8 mg, 5.4 mg/kg, 5.6 mg/kg, 6.4 mg/kg or 8.0 mg/kg.

The anti-CDH6 antibody-drug conjugate used in the present invention can be administered at a dose of approximately 5 to approximately 3000 mg per administration, can preferably be administered at a dose of approximately 10 to approximately 2000 mg, can more preferably be administered at a dose of approximately 100 to approximately 1500 mg, and can still more preferably be administered at a dose of approximately 200 to approximately 1000 mg.

The anti-CDH6 antibody-drug conjugate used in the present invention can preferably be administered at a dose of approximately 200 mg, approximately 205 mg, approximately 210 mg, approximately 215 mg, approximately 220 mg, approximately 225 mg, approximately 230 mg, approximately 235 mg, approximately 240 mg, approximately 245 mg, approximately 250 mg, approximately 255 mg, approximately 260 mg, approximately 265 mg, approximately 270 mg, approximately 275 mg, approximately 280 mg, approximately 285 mg, approximately 290 mg, approximately 295 mg, approximately 300 mg, approximately 305 mg, approximately 310 mg, approximately 315 mg, approximately 320 mg, approximately 325 mg, approximately 330 mg, approximately 335 mg, approximately 340 mg, approximately 345 mg, approximately 350 mg, approximately 355 mg, approximately 360 mg, approximately 365 mg, approximately 370 mg, approximately 375 mg, approximately 380 mg, approximately 385 mg, approximately 390 mg, approximately 395 mg, approximately 400 mg, approximately 405 mg, approximately 410 mg, approximately 415 mg, approximately 420 mg, approximately 425 mg, approximately 430 mg, approximately 435 mg, approximately 440 mg, approximately 445 mg, approximately 450 mg, approximately 455 mg, approximately 460 mg, approximately 465 mg, approximately 470 mg,

approximately 475 mg, approximately 480 mg, approximately 485 mg, approximately 490 mg, approximately 495 mg, approximately 500 mg, approximately 505 mg, approximately 510 mg, approximately 515 mg, approximately 520 mg, approximately 525 mg, approximately 530 mg, approximately 535 mg, approximately 540 mg, approximately 545 mg, approximately 550 mg, approximately 555 mg, approximately 560 mg, approximately 565 mg, approximately 570 mg, approximately 575 mg, approximately 580 mg, approximately 585 mg, approximately 590 mg, approximately 595 mg, approximately 600 mg, approximately 605 mg, approximately 610 mg, approximately 615 mg, approximately 620 mg, approximately 625 mg, approximately 630 mg, approximately 635 mg, approximately 640 mg, approximately 645 mg, approximately 650 mg, approximately 655 mg, approximately 660 mg, approximately 665 mg, approximately 670 mg, approximately 675 mg, approximately 680 mg, approximately 685 mg, approximately 690 mg, approximately 695 mg, approximately 700 mg, approximately 705 mg, approximately 710 mg, approximately 715 mg, approximately 720 mg, approximately 725 mg, approximately 730 mg, approximately 735 mg, approximately 740 mg, approximately 745 mg, approximately 750 mg, approximately 755 mg, approximately 760 mg, approximately 765 mg, approximately 770 mg, approximately 775 mg, approximately 780 mg, approximately 785 mg, approximately 790 mg, approximately 795 mg, approximately 800 mg, approximately 805 mg, approximately 810 mg, approximately 815 mg, approximately 820 mg, approximately 825 mg, approximately 830 mg, approximately 835 mg, approximately 840 mg, approximately 845 mg, approximately 850 mg, approximately 855 mg, approximately 860 mg, approximately 865 mg, approximately 870 mg, approximately 875 mg, approximately 880 mg, approximately 885 mg, approximately 890 mg, approximately 895 mg, approximately 900 mg, approximately 905 mg, approximately 910 mg, approximately 915 mg, approximately 920 mg, approximately 925 mg, approximately 930 mg, approximately 935 mg, approximately 940 mg, approximately 945 mg, approximately 950 mg, approximately 955 mg, approximately 960 mg, approximately 965 mg, approximately 970 mg, approximately 975 mg, approximately 980 mg, approximately 985 mg, approximately 990 mg, approximately 995 mg or approximately 1000 mg per administration.

The anti-CDH6 antibody-drug conjugate used in the present invention can preferably be administered at a dose of 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, 500 mg, 505 mg, 510 mg, 515 mg, 520 mg, 525 mg, 530 mg, 535 mg, 540 mg, 545 mg, 550 mg, 555 mg, 560 mg, 565 mg, 570 mg, 575 mg, 580 mg, 585 mg, 590 mg, 595 mg, 600 mg, 605 mg, 610 mg, 615 mg, 620 mg, 625 mg, 630 mg, 635 mg, 640 mg, 645 mg, 650 mg, 655 mg, 660 mg, 665 mg, 670 mg, 675 mg, 680 mg, 685 mg, 690 mg, 695 mg, 700 mg, 705 mg, 710 mg, 715 mg, 720 mg, 725 mg, 730 mg, 735 mg, 740 mg, 745 mg, 750 mg, 755 mg, 760 mg, 765 mg, 770 mg, 775 mg, 780 mg, 785 mg, 790 mg, 795 mg, 800 mg, 805 mg, 810 mg, 815 mg, 820 mg, 825 mg, 830 mg, 835 mg, 840 mg, 845 mg, 850 mg, 855 mg, 860 mg, 865 mg, 870 mg, 875 mg, 880 mg, 885 mg, 890 mg, 895 mg, 900 mg, 905 mg, 910 mg, 915 mg, 920 mg, 925 mg, 930 mg, 935 mg, 940 mg, 945 mg, 950 mg, 955 mg, 960 mg, 965 mg, 970 mg, 975 mg, 980 mg, 985 mg, 990 mg, 995 mg or 1000 mg per administration.

The dosage regimen of the anti-CDH6 antibody-drug conjugate used in the present invention may be, for example, 0.1 mg/kg (at intervals of 3 weeks; hereinafter, referred to as "q3w"), 0.2 mg/kg (q3w), 0.3 mg/kg (q3w), 0.4 mg/kg (q3w), 0.5 mg/kg (q3w), 0.6 mg/kg (q3w), 0.7 mg/kg (q3w), 0.8 mg/kg (q3w), 0.9 mg/kg (q3w), 1.0 mg/kg (q3w), 1.1 mg/kg (q3w), 1.2 mg/kg (q3w), 1.3 mg/kg (q3w), 1.4 mg/kg (q3w), 1.5 mg/kg (q3w), 1.6 mg/kg (q3w), 1.7 mg/kg (q3w), 1.8 mg/kg (q3w), 1.9 mg/kg (q3w), 2.0 mg/kg (q3w), 2.1 mg/kg (q3w), 2.2 mg/kg (q3w), 2.3 mg/kg (q3w), 2.4 mg/kg (q3w), 2.5 mg/kg (q3w), 2.6 mg/kg (q3w), 2.7 mg/kg (q3w), 2.8 mg/kg (q3w), 2.9 mg/kg (q3w), 3.0 mg/kg (q3w), 3.1 mg/kg (q3w), 3.2 mg/kg (q3w), 3.3 mg/kg (q3w), 3.4 mg/kg (q3w), 3.5 mg/kg (q3w), 3.6 mg/kg (q3w), 3.7 mg/kg (q3w), 3.8 mg/kg (q3w), 3.9 mg/kg (q3w), 4.0 mg/kg (q3w), 4.1 mg/kg (q3w), 4.2 mg/kg (q3w), 4.3 mg/kg (q3w), 4.4 mg/kg (q3w), 4.5 mg/kg (q3w), 4.6 mg/kg (q3w), 4.7 mg/kg (q3w), 4.8 mg/kg (q3w), 4.9 mg/kg (q3w), 5.0 mg/kg (q3w), 5.1 mg/kg (q3w), 5.2 mg/kg (q3w), 5.3 mg/kg (q3w), 5.4 mg/kg (q3w), 5.5 mg/kg (q3w), 5.6 mg/kg (q3w), 5.7 mg/kg (q3w), 5.8 mg/kg (q3w), 5.9 mg/kg (q3w), 6.0 mg/kg (q3w), 6.1 mg/kg (q3w), 6.2 mg/kg (q3w), 6.3 mg/kg (q3w), 6.4 mg/kg (q3w), 6.5 mg/kg (q3w), 6.6 mg/kg (q3w), 6.7 mg/kg (q3w), 6.8 mg/kg (q3w), 6.9 mg/kg (q3w), 7.0 mg/kg (q3w), 7.1 mg/kg (q3w), 7.2 mg/kg (q3w), 7.3 mg/kg (q3w), 7.4 mg/kg (q3w), 7.5 mg/kg (q3w), 7.6 mg/kg (q3w), 7.7 mg/kg (q3w), 7.8 mg/kg (q3w), 7.9 mg/kg (q3w), 8.0 mg/kg (q3w), 8.1 mg/kg (q3w), 8.2 mg/kg (q3w), 8.3 mg/kg (q3w), 8.4 mg/kg (q3w), 8.5 mg/kg (q3w), 8.6 mg/kg (q3w), 8.7 mg/kg (q3w), 8.8 mg/kg (q3w), 8.9 mg/kg (q3w), 9.0 mg/kg (q3w), 9.1 mg/kg (q3w), 9.2 mg/kg (q3w), 9.3 mg/kg (q3w), 9.4 mg/kg (q3w), 9.5 mg/kg (q3w), 9.6 mg/kg (q3w), 9.7 mg/kg (q3w), 9.8 mg/kg (q3w), 9.9 mg/kg (q3w), 10.0 mg/kg (q3w), 10.1 mg/kg (q3w), 10.2 mg/kg (q3w), 10.3 mg/kg (q3w), 10.4 mg/kg (q3w), 10.5 mg/kg (q3w), 10.6 mg/kg (q3w), 10.7 mg/kg (q3w), 10.8 mg/kg (q3w), 10.9 mg/kg (q3w), 11.0 mg/kg (q3w), 11.1 mg/kg (q3w), 11.2 mg/kg (q3w), 11.3 mg/kg (q3w), 11.4 mg/kg (q3w), 11.5 mg/kg (q3w), 11.6 mg/kg (q3w), 11.7 mg/kg (q3w), 11.8 mg/kg (q3w), 11.9 mg/kg (q3w) or 12.0 mg/kg (q3w).

The dosage regimen of the anti-CDH6 antibody-drug conjugate used in the present invention is preferably 1.6 mg/kg (q3w), 3.2 mg/kg (q3w), 4.8 mg/kg (q3w), 5.4 mg/kg (q3w), 5.6 mg/kg (q3w), 6.4 mg/kg (q3w), 8.0 mg/kg (q3w) or 9.6 mg/kg (q3w), more preferably 3.2 mg/kg (q3w), 4.8 mg/kg (q3w), 5.4 mg/kg (q3w), 5.6 mg/kg (q3w), 6.4 mg/kg (q3w) or 8.0 mg/kg (q3w), and still more preferably 4.8 mg/kg (q3w), 5.4 mg/kg (q3w), 5.6 mg/kg (q3w), 6.4 mg/kg (q3w) or 8.0 mg/kg

(q3w).

The dosage regimen of the anti-CDH6 antibody-drug conjugate used in the present invention is preferably 200 mg (q3w), 205 mg (q3w), 210 mg (q3w), 215 mg (q3w), 220 mg (q3w), 225 mg (q3w), 230 mg (q3w), 235 mg (q3w), 240 mg (q3w), 245 mg (q3w), 250 mg (q3w), 255 mg (q3w), 260 mg (q3w), 265 mg (q3w), 270 mg (q3w), 275 mg (q3w), 280 mg (q3w), 285 mg (q3w), 290 mg (q3w), 295 mg (q3w), 300 mg (q3w), 305 mg (q3w), 310 mg (q3w), 315 mg (q3w), 320 mg (q3w), 325 mg (q3w), 330 mg (q3w), 335 mg (q3w), 340 mg (q3w), 345 mg (q3w), 350 mg (q3w), 355 mg (q3w), 360 mg (q3w), 365 mg (q3w), 370 mg (q3w), 375 mg (q3w), 380 mg (q3w), 385 mg (q3w), 390 mg (q3w), 395 mg (q3w), 400 mg (q3w), 405 mg (q3w), 410 mg (q3w), 415 mg (q3w), 420 mg (q3w), 425 mg (q3w), 430 mg (q3w), 435 mg (q3w), 440 mg (q3w), 445 mg (q3w), 450 mg (q3w), 455 mg (q3w), 460 mg (q3w), 465 mg (q3w), 470 mg (q3w), 475 mg (q3w), 480 mg (q3w), 485 mg (q3w), 490 mg (q3w), 495 mg (q3w), 500 mg (q3w), 505 mg (q3w), 510 mg (q3w), 515 mg (q3w), 520 mg (q3w), 525 mg (q3w), 530 mg (q3w), 535 mg (q3w), 540 mg (q3w), 545 mg (q3w), 550 mg (q3w), 555 mg (q3w), 560 mg (q3w), 565 mg (q3w), 570 mg (q3w), 575 mg (q3w), 580 mg (q3w), 585 mg (q3w), 590 mg (q3w), 595 mg (q3w), 600 mg (q3w), 605 mg (q3w), 610 mg (q3w), 615 mg (q3w), 620 mg (q3w), 625 mg (q3w), 630 mg (q3w), 635 mg (q3w), 640 mg (q3w), 645 mg (q3w), 650 mg (q3w), 655 mg (q3w), 660 mg (q3w), 665 mg (q3w), 670 mg (q3w), 675 mg (q3w), 680 mg (q3w), 685 mg (q3w), 690 mg (q3w), 695 mg (q3w), 700 mg (q3w), 705 mg (q3w), 710 mg (q3w), 715 mg (q3w), 720 mg (q3w), 725 mg (q3w), 730 mg (q3w), 735 mg (q3w), 740 mg (q3w), 745 mg (q3w), 750 mg (q3w), 755 mg (q3w), 760 mg (q3w), 765 mg (q3w), 770 mg (q3w), 775 mg (q3w), 780 mg (q3w), 785 mg (q3w), 790 mg (q3w), 795 mg (q3w), 800 mg (q3w), 805 mg (q3w), 810 mg (q3w), 815 mg (q3w), 820 mg (q3w), 825 mg (q3w), 830 mg (q3w), 835 mg (q3w), 840 mg (q3w), 845 mg (q3w), 850 mg (q3w), 855 mg (q3w), 860 mg (q3w), 865 mg (q3w), 870 mg (q3w), 875 mg (q3w), 880 mg (q3w), 885 mg (q3w), 890 mg (q3w), 895 mg (q3w), 900 mg (q3w), 905 mg (q3w), 910 mg (q3w), 915 mg (q3w), 920 mg (q3w), 925 mg (q3w), 930 mg (q3w), 935 mg (q3w), 940 mg (q3w), 945 mg (q3w), 950 mg (q3w), 955 mg (q3w), 960 mg (q3w), 965 mg (q3w), 970 mg (q3w), 975 mg (q3w), 980 mg (q3w), 985 mg (q3w), 990 mg (q3w), 995 mg (q3w) or 1000 mg (q3w).

The HIF-2α inhibitor according to the present invention can be administered orally to a human at intervals of once or twice daily to once or twice per 7 days, and can preferably be administered orally at intervals of once daily or twice daily. The HIF-2α inhibitor used in the present invention can be administered orally at a dose of 0.1 mg to 3000 mg per administration, and can preferably be administered orally at a dose of 2.5 mg to 600 mg per administration. The HIF-2α inhibitor according to the present invention can be administered to a human by intravenous drip at intervals of 1 to 180 days, and can preferably be administered by intravenous drip once a week, once every 2 weeks, once every 3 weeks, or once every 4 weeks. The HIF-2α inhibitor used in the present invention can be administered by intravenous drip at a dose of 0.1 mg to 3000 mg per administration, and can preferably be administered by intravenous drip at a dose of 10 mg to 100 mg per administration.

When the HIF-2α inhibitor used in the present invention is belzutifan or a pharmaceutically acceptable salt thereof, examples of the method of administration include, but are not limited to, the following dosages and administrations. For example, belzutifan can be administered at a dose of 5 to 1000 mg per administration at intervals of once, twice, three times, four times or five times daily.

As another dosage and administration, belzutifan can be administered orally at a dose of 20 to 240 mg per administration at intervals of once daily.

As another dosage and administration, belzutifan can be administered orally at a dose of 20, 40, 80, 120, 160 or 240 mg (preferably 40, 80 or 120 mg) per administration at intervals of once daily.

As another dosage and administration, belzutifan can be administered orally at a dose of 120 mg per administration at intervals of once daily.

As another dosage and administration, belzutifan can be administered orally at a dose of 80 mg per administration at intervals of once daily.

As another dosage and administration, belzutifan can be administered orally at a dose of 40 mg per administration at intervals of once daily.

The pharmaceutical composition and the method of treatment of the present invention may further comprise a therapeutic agent for cancer other than the anti-CDH6 antibody-drug conjugate according to the present invention and the HIF-2α inhibitor. The pharmaceutical composition and the method of treatment of the present invention can also be administered in combination with an additional therapeutic agent for cancer, and can thereby enhance an antitumor effect. The additional therapeutic agent for cancer used for such a purpose may be administered to an individual at the same time (at approximately the same time), separately, or continuously, together with the pharmaceutical composition of the present invention. Otherwise, the additional therapeutic agent and the pharmaceutical composition may each be administered to the subject at different administration intervals. Such a therapeutic agent for cancer is not limited as long as the agent has an antitumor activity. Examples thereof can include at least one selected from the group consisting of irinotecan (CPT-11), cisplatin, carboplatin, oxaliplatin, fluorouracil (5-FU), gemcitabine, capecitabine, doxorubicin, epirubicin, cyclophosphamide, mitomycin C, tegafur/gimeracil/oteracil, panitumumab, bevacizumab, ramucirumab, regorafenib, trifluridine/tipiracil, gefitinib, erlotinib, afatinib, methotrexate, pemetrexed, tamoxifen, toremifene, fulvestrant, leuprorelin, goserelin,

letrozole, anastrozole, progesterone formulations, and lapatinib.

The pharmaceutical composition and the method of treatment of the present invention may be used in combination with radiotherapy. For example, a cancer patient receives radiotherapy before and/or after or at the same time (at approximately the same time) as treatment with the pharmaceutical composition of the present invention.

The pharmaceutical composition and the method of treatment of the present invention may be used as adjunctive chemotherapy combined with surgery. The pharmaceutical composition of the present invention may be administered for the purpose of decreasing a tumor size before surgery (neoadjuvant chemotherapy), or may be administered for the purpose of preventing tumor recurrence after surgery (adjuvant chemotherapy).

The pharmaceutical composition and the method of treatment of the present invention may be used as maintenance therapy. For example, after initial chemotherapy, treatment is continued for the purpose of preventing recurrence.

The present invention includes a method of treatment of a disease, comprising administering the anti-CDH6 antibody-drug conjugate of the present application and a HIF-2α inhibitor in combination to a subject in need of the treatment. The present invention includes the anti-CDH6 antibody-drug conjugate of the present application for use in combination with a HIF-2α inhibitor for treating a disease. The present invention includes use of the anti-CDH6 antibody-drug conjugate of the present application combined with a HIF-2α inhibitor for the manufacture of a medicament for treating a disease. The present invention includes a pharmaceutical product comprising the anti-CDH6 antibody-drug conjugate of the present application and a HIF-2α inhibitor. The present invention includes a combination medicament comprising the anti-CDH6 antibody-drug conjugate of the present application and a HIF-2α inhibitor. The present invention includes a pharmaceutical combination comprising the anti-CDH6 antibody-drug conjugate of the present application and a HIF-2α inhibitor. The present invention includes use of the anti-CDH6 antibody-drug conjugate of the present application combined with a HIF-2α inhibitor for treating a disease. The present invention includes a medicament comprising the anti-CDH6 antibody-drug conjugate of the present application and a HIF-2α inhibitor.

[Examples]

Hereinafter, the present invention will be specifically described in the following examples. However, these examples are not intended to limit the scope of the present invention. Furthermore, these examples should not be construed in a limited manner by any means.

Production Example 1: Production of anti-CDH6 antibody-drug conjugate

In accordance with the production method described in International Publication No. WO 2018/212136 with use of a humanized anti-CDH6 antibody (antibody comprising a heavy chain consisting of the amino acid sequence shown in SEQ ID NO: 29 and a light chain consisting of the amino acid sequence shown in SEQ ID NO: 38), an antibody-drug conjugate in which a drug-linker represented by the formula:

[Formula 15]

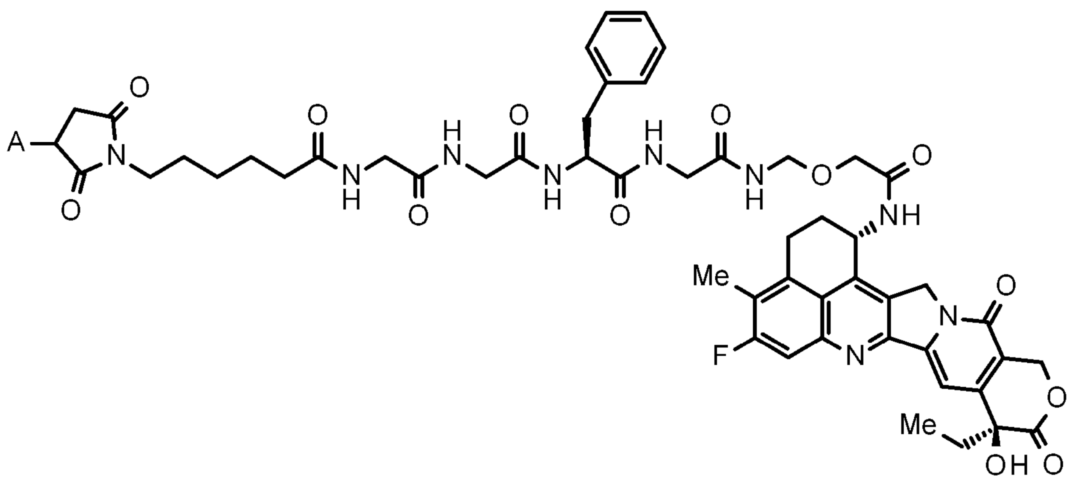

wherein A represents a connecting position to an antibody, is conjugated to the anti-CDH6 antibody via a thioether bond (hereinafter, referred to as the "anti-CDH6 antibody-drug conjugate (1)") was produced. The DAR of the anti-CDH6 antibody-drug conjugate (1) was 7.9.

Production Example 2: Preparation of compound (B)

A compound (B) represented by the formula:

[Formula 16]

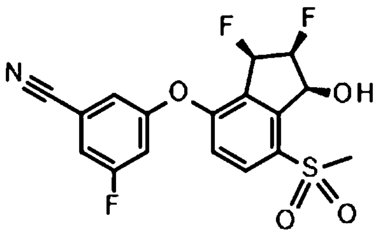

(belzutifan) was produced in accordance with the production method described in the literature J. Med. Chem. 2019, 62, 6876-6893.

Example 1: Antitumor test (1)

Mouse: Six-week-old female BALB/c nude mice (The Jackson Laboratory Japan, Inc.) were subjected to an experiment.

Measurement and calculation expression: The long diameter and short diameter of each tumor were measured twice a week using electronic digital calipers (CD-15CX, Mitutoyo Corp.), and the volume of the tumor was then calculated. The following expression was used in the calculation.

Tumor volume ($mm^3$) = 1/2 $\times$ Long diameter (mm) $\times$ [Short diameter (mm)]$^2$

The anti-CDH6 antibody-drug conjugate (1) was diluted with ABS buffer (10 mM acetate buffer (pH 5.5), 5% sorbitol), and the dilution was intravenously administered at a dose of 10 mL/kg to the tail of each mouse. Belzutifan was suspended in 10% dimethyl sulfoxide, 40% polyethylene glycol 300, 5% Tween-80, and 45% physiological saline, and the suspension was administered orally at a dose of 10 mL/kg.

Human kidney cancer cell line 786-O cells purchased from ATCC (American Type Culture Collection) were suspended in Matrigel Matrix, and the cell suspension was subcutaneously inoculated at a dose of 4 $\times$ $10^6$ cells to the right flank region of each female nude mouse. 31 days after inoculation, the mice were randomly grouped (Day 0). The anti-CDH6 antibody-drug conjugate (1) was administered at a dose of 1 mg/kg on Day 0. Belzutifan was administered at a dose of 0.3 mg/kg once daily and five times a week for 3 weeks. Their respective single administration groups and combined administration group, and an ABS buffer administration group as a control group, were established.

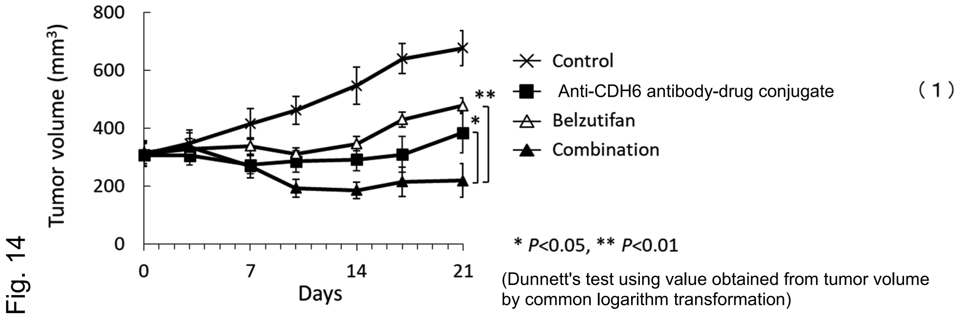

The result of combined use of the anti-CDH6 antibody-drug conjugate (1) and belzutifan is shown in Figure 14. In Figure 14, the abscissa depicts the number of days after the start of administration, and the ordinate depicts tumor volume.

Tumor growth inhibition (TGI) is calculated according to the following expression.

TGI (%) = [1 - (Average tumor volume of the drug administration group / Average tumor volume of the control group)] $\times$ 100 [Expression 2]

The tumor growth inhibition (TGI) on the date of response evaluation of single administration of belzutifan (Day 21) was 29%. The TGI by the single administration of the anti-CDH6 antibody-drug conjugate (1) was 43%. On the other hand, the combined administration of the anti-CDH6 antibody-drug conjugate (1) and belzutifan was found to have a significantly better tumor growth suppressing effect than that of the single administration of the anti-CDH6 antibody-drug conjugate (1) and the single administration of belzutifan (P = 0.0325 and P = 0.0028, respectively; calculated by Dunnett's test using a value obtained from the tumor volume by common logarithm transformation). TGI by the combined administration (68%) was higher than that of the respective single administrations, demonstrating that the combined use enhances an antitumor effect.

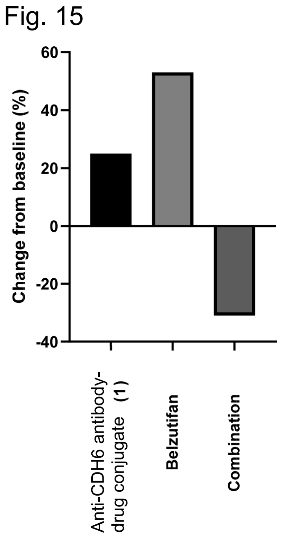

Change from baseline (%) (percentage change of average tumor volume on Day 21 from an average tumor volume on Day 0 as a baseline) was calculated according to the following expression, and is shown in Figure 15.

[Expression 3]

$$\text{Change from baseline (\%)} = \frac{\text{Average tumor volume on Day 21} - \text{Average tumor volume on Day 0}}{\text{Average tumor volume on Day 0}} \times 100$$

In addition, the change from baseline (%) of each group is described in Table 1. As shown in Figure 15 and Table 1, the change from baseline (%) was a negative value in the combined administration group, demonstrating a strong combinatorial effect with regression.

[Table 1]

| Compound | Change from baseline (%) |
|---|---|
| Anti-CDH6 antibody-drug conjugate (1) | 25 |
| Belzutifan | 53 |
| Anti-CDH6 antibody-drug conjugate (1) + belzutifan | -31 |

None of the single administration groups and the combined administration group exhibited any particular notable finding such as severe weight loss. Hence, the combination of the anti-CDH6 antibody-drug conjugate (1) and belzutifan increased efficacy without increasing toxicity.

[Industrial Applicability]

From the above-described experimental results, the antibody-drug conjugate according to the present invention has been found to exhibit an excellent antitumor effect by administering the antibody-drug conjugate and a HIF-2α inhibitor in combination.

[Free Text of Sequence Listing]

SEQ ID NO: 1: Amino acid sequence of human CDH6 EC3
SEQ ID NO: 2: Amino acid sequence of chG019 CDRH1
SEQ ID NO: 3: Amino acid sequence of chG019 CDRH2
SEQ ID NO: 4: Amino acid sequence of chG019 CDRH3
SEQ ID NO: 5: Amino acid sequence of chG019 CDRL1
SEQ ID NO: 6: Amino acid sequence of chG019 CDRL2
SEQ ID NO: 7: Amino acid sequence of chG019 CDRL3
SEQ ID NO: 8: Amino acid sequence of rG019 CDRH2
SEQ ID NO: 9: Amino acid sequence of rG055 CDRH1
SEQ ID NO: 10: Amino acid sequence of rG055 CDRH2
SEQ ID NO: 11: Amino acid sequence of rG055 CDRH3
SEQ ID NO: 12: Amino acid sequence of rG055 CDRL1
SEQ ID NO: 13: Amino acid sequence of rG055 CDRL2
SEQ ID NO: 14: Amino acid sequence of rG055 CDRL3
SEQ ID NO: 15: Amino acid sequence of rG056 CDRH1
SEQ ID NO: 16: Amino acid sequence of rG056 CDRH2
SEQ ID NO: 17: Amino acid sequence of rG056 CDRH3
SEQ ID NO: 18: Amino acid sequence of rG056 CDRL1
SEQ ID NO: 19: Amino acid sequence of rG056 CDRL2
SEQ ID NO: 20: Amino acid sequence of rG056 CDRL3
SEQ ID NO: 21: Amino acid sequence of rG061 CDRH1
SEQ ID NO: 22: Amino acid sequence of rG061 CDRH2
SEQ ID NO: 23: Amino acid sequence of rG061 CDRH3

SEQ ID NO: 24: Amino acid sequence of rG061 CDRL1
SEQ ID NO: 25: Amino acid sequence of rG061 CDRL2
SEQ ID NO: 26: Amino acid sequence of rG061 CDRL3
SEQ ID NO: 27: Full-length amino acid sequence of a hH01 heavy chain
SEQ ID NO: 28: Amino acid sequence of a hH01 heavy chain variable region
SEQ ID NO: 29: Full-length amino acid sequence of the hH01 heavy chain except for a signal sequence
SEQ ID NO: 30: Full-length amino acid sequence of a hH02 heavy chain
SEQ ID NO: 31: Amino acid sequence of a hH02 heavy chain variable region
SEQ ID NO: 32: Full-length amino acid sequence of the hH02 heavy chain except for a signal sequence
SEQ ID NO: 33: Full-length amino acid sequence of a hH04 heavy chain
SEQ ID NO: 34: Amino acid sequence of a hH04 heavy chain variable region
SEQ ID NO: 35: Full-length amino acid sequence of the hH04 heavy chain except for a signal sequence
SEQ ID NO: 36: Full-length amino acid sequence of a hL02 light chain
SEQ ID NO: 37: Amino acid sequence of a hL02 light chain variable region
SEQ ID NO: 38: Full-length amino acid sequence of the hL02 light chain except for a signal sequence
SEQ ID NO: 39: Full-length amino acid sequence of a hL03 light chain
SEQ ID NO: 40: Amino acid sequence of a hL03 light chain variable region
SEQ ID NO: 41: Full-length amino acid sequence of the hL03 light chain except for a signal sequence

## Claims

1. A pharmaceutical composition comprising an anti-CDH6 antibody-drug conjugate, wherein the anti-CDH6 antibody-drug conjugate and a HIF-2α inhibitor are administered in combination, and
the anti-CDH6 antibody-drug conjugate is an antibody-drug conjugate in which a drug-linker represented by the formula:

[Formula 1]

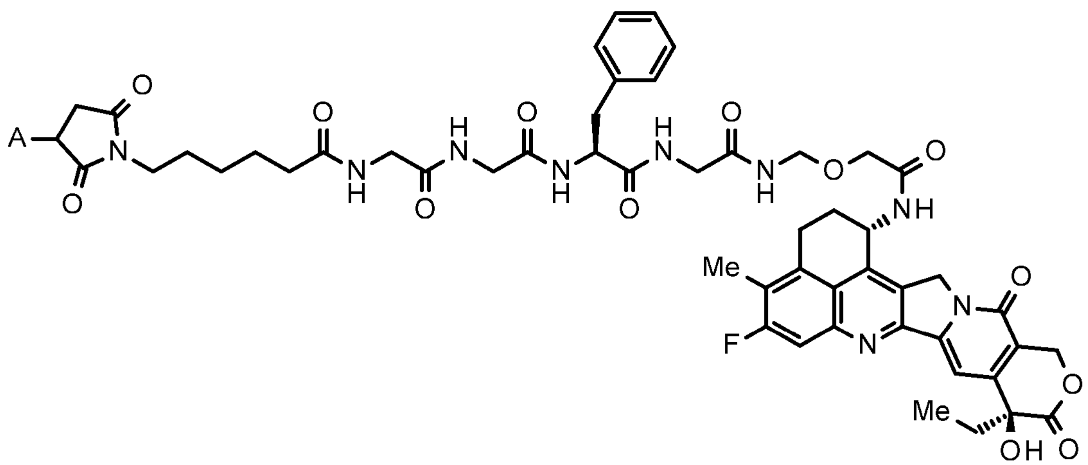

wherein A represents a connecting position to an anti-CDH6 antibody or a functional fragment of the antibody, is conjugated to the anti-CDH6 antibody or the functional fragment of the antibody via a thioether bond.

2. The pharmaceutical composition according to claim 1, wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

3. The pharmaceutical composition according to claim 1 or 2, wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

4. The pharmaceutical composition according to any one of claims 1 to 3, wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.

5. The pharmaceutical composition according to any one of claims 1 to 4, wherein the anti-CDH6 antibody or the functional fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.

6. The pharmaceutical composition according to any one of claims 1 to 5, wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus, or a functional fragment of the antibody.

7. The pharmaceutical composition according to claim 6, wherein the anti-CDH6 antibody or the functional fragment of the antibody is an antibody in which one or two amino acids are deleted from the carboxyl terminus of a heavy chain thereof, or a functional fragment of the antibody.

8. The pharmaceutical composition according to claim 7, wherein the anti-CDH6 antibody or the functional fragment of the antibody is an antibody in which one amino acid is deleted from the carboxyl terminus of each of the two heavy chains thereof, or a functional fragment of the antibody.

9. The pharmaceutical composition according to any one of claims 6 to 8, wherein the deleted amino acid is lysine.

10. The pharmaceutical composition according to any one of claims 6 to 9, wherein the anti-CDH6 antibody or the functional fragment of the antibody is an antibody in which a proline residue at the carboxyl terminus of a heavy chain thereof is further amidated, or a functional fragment of the antibody.

11. The pharmaceutical composition according to any one of claims 1 to 10, wherein the average number of units of the drug-linker structure conjugated per antibody in the anti-CDH6 antibody-drug conjugate is in the range of from 1 to 10.

12. The pharmaceutical composition according to any one of claims 1 to 10, wherein the average number of units of the drug-linker structure conjugated per antibody in the anti-CDH6 antibody-drug conjugate is in the range of from 7 to 8.

13. The pharmaceutical composition according to any one of claims 1 to 12, wherein the HIF-2$\alpha$ inhibitor is belzutifan, NKT2152, DFF332, AB521, or BPI-452080, or a pharmaceutically acceptable salt thereof.

14. The pharmaceutical composition according to any one of claims 1 to 12, wherein the HIF-2$\alpha$ inhibitor is belzutifan or a pharmaceutically acceptable salt thereof.

15. The pharmaceutical composition according to any one of claims 1 to 14, wherein the anti-CDH6 antibody-drug conjugate and the HIF-2$\alpha$ inhibitor are separately contained in different formulations, and are administered at the same time or at different times.

16. The pharmaceutical composition according to any one of claims 1 to 14, wherein the anti-CDH6 antibody-drug conjugate and the HIF-2$\alpha$ inhibitor are contained in a single formulation for administration.

17. The pharmaceutical composition according to any one of claims 1 to 16, wherein the pharmaceutical composition is for use in treating cancer.

18. The pharmaceutical composition according to any one of claims 1 to 16, wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell

lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

**19.** The pharmaceutical composition according to any one of claims 1 to 16, wherein the pharmaceutical composition is for use in treating at least one selected from the group consisting of renal cell carcinoma, renal clear cell carcinoma, and papillary renal cell carcinoma.

**20.** A pharmaceutical composition comprising an anti-CDH6 antibody-drug conjugate, wherein the anti-CDH6 antibody-drug conjugate and a HIF-2α inhibitor are administered in combination, and
the anti-CDH6 antibody-drug conjugate is an antibody-drug conjugate represented by the formula:

[Formula 2]

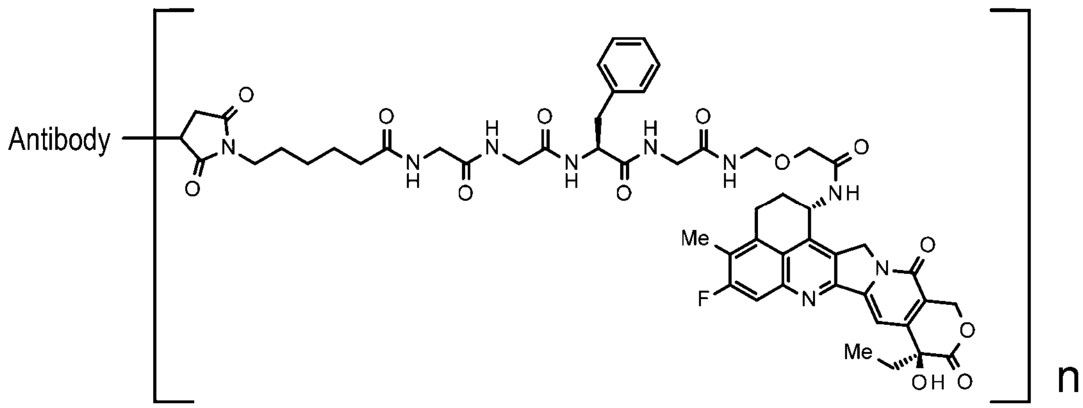

wherein Antibody is an anti-CDH6 antibody, a drug-linker is conjugated to the antibody via a thioether bond, and n represents an average number of units of the drug-linker conjugated per antibody.

**21.** The pharmaceutical composition according to claim 20, wherein the anti-CDH6 antibody is an antibody comprising a heavy chain consisting of the amino acid sequence shown in SEQ ID NO: 29 and a light chain consisting of the amino acid sequence shown in SEQ ID NO: 38.

**22.** The pharmaceutical composition according to claim 20 or 21, wherein a lysine residue is deleted from the carboxyl terminus of a heavy chain of the anti-CDH6 antibody.

**23.** The pharmaceutical composition according to any one of claims 20 to 22, wherein the average number of units of the drug-linker conjugated per antibody in the anti-CDH6 antibody-drug conjugate is in the range of from 7 to 8.

**24.** The pharmaceutical composition according to any one of claims 20 to 23, wherein the HIF-2α inhibitor is belzutifan, NKT2152, DFF332, AB521, or BPI-452080, or a pharmaceutically acceptable salt thereof.

**25.** The pharmaceutical composition according to any one of claims 20 to 23, wherein the HIF-2α inhibitor is belzutifan or a pharmaceutically acceptable salt thereof.

**26.** The pharmaceutical composition according to any one of claims 20 to 25, wherein the anti-CDH6 antibody-drug conjugate and the HIF-2α inhibitor are separately contained in different formulations, and are administered at the same time or at different times.

**27.** The pharmaceutical composition according to any one of claims 20 to 25, wherein the anti-CDH6 antibody-drug conjugate and the HIF-2α inhibitor are contained in a single formulation for administration.

**28.** The pharmaceutical composition according to any one of claims 20 to 27, wherein the pharmaceutical composition is for use in treating cancer.

**29.** The pharmaceutical composition according to any one of claims 20 to 27, wherein the pharmaceutical composition is for use in treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

**30.** The pharmaceutical composition according to any one of claims 20 to 27, wherein the pharmaceutical composition is for use in treating at least one selected from the group consisting of renal cell carcinoma, renal clear cell carcinoma, and papillary renal cell carcinoma.

**31.** The pharmaceutical composition according to any one of claims 1 to 30, wherein the anti-CDH6 antibody-drug conjugate is raludotatug deruxtecan (DS-6000a).

**32.** A method of treatment, comprising administering an anti-CDH6 antibody-drug conjugate and a HIF-2$\alpha$ inhibitor in combination to a subject in need of the treatment, wherein the antibody-drug conjugate is an antibody-drug conjugate in which a drug-linker represented by the formula:

[Formula 3]

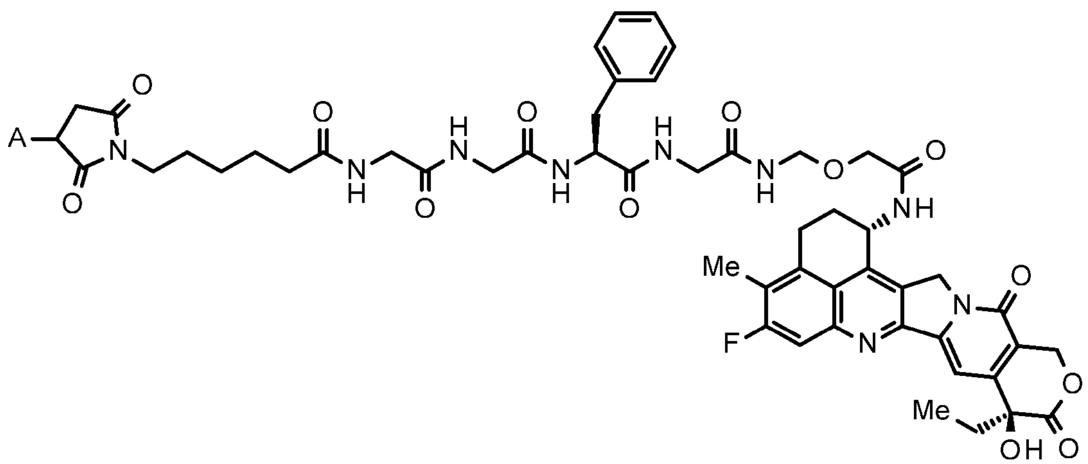

wherein A represents a connecting position to an anti-CDH6 antibody or a functional fragment of the antibody, is conjugated to the anti-CDH6 antibody or the functional fragment of the antibody via a thioether bond.

**33.** The method of treatment according to claim 32, wherein the anti-CDH6 antibody or the functional fragment of the antibody is an antibody which specifically binds to the amino acid sequence shown in SEQ ID NO: 1 and has an internalization ability that permits cellular uptake, or a functional fragment of the antibody.

**34.** The method of treatment according to claim 32 or 33, wherein the anti-CDH6 antibody or the functional fragment of the antibody is an antibody comprising CDRH1 consisting of the amino acid sequence shown in SEQ ID NO: 2, CDRH2 consisting of the amino acid sequence shown in SEQ ID NO: 3, CDRH3 consisting of the amino acid sequence shown in SEQ ID NO: 4, CDRL1 consisting of the amino acid sequence shown in SEQ ID NO: 5, CDRL2 consisting of the amino acid sequence shown in SEQ ID NO: 6, and CDRL3 consisting of the amino acid sequence shown in SEQ ID NO: 7, or a functional fragment of the antibody.

**35.** The method of treatment according to any one of claims 32 to 34, wherein the anti-CDH6 antibody or the functional fragment of the antibody is an antibody comprising a heavy chain variable region consisting of the amino acid sequence shown in SEQ ID NO: 28 and a light chain variable region consisting of the amino acid sequence shown in SEQ ID NO: 37, or a functional fragment of the antibody.

**36.** The method of treatment according to any one of claims 32 to 35, wherein the anti-CDH6 antibody or the functional

fragment of the antibody is an antibody comprising a heavy chain consisting of the amino acid sequence shown in SEQ ID NO: 29 and a light chain consisting of the amino acid sequence shown in SEQ ID NO: 38, or a functional fragment of the antibody.

**37.** The method of treatment according to any one of claims 32 to 36, wherein the anti-CDH6 antibody or the functional fragment of the antibody is an antibody in which the heavy chain or the light chain has undergone one or two or more modifications selected from the group consisting of N-linked glycosylation, O-linked glycosylation, N-terminal processing, C-terminal processing, deamidation, isomerization of aspartic acid, oxidation of methionine, addition of a methionine residue to the N-terminus, amidation of a proline residue, conversion of N-terminal glutamine or N-terminal glutamic acid to pyroglutamic acid, and a deletion of one or two amino acids from the carboxyl terminus, or a functional fragment of the antibody.

**38.** The method of treatment according to claim 37, wherein the anti-CDH6 antibody or the functional fragment of the antibody is an antibody in which one or two amino acids are deleted from the carboxyl terminus of a heavy chain thereof, or a functional fragment of the antibody.

**39.** The method of treatment according to claim 38, wherein the anti-CDH6 antibody or the functional fragment of the antibody is an antibody in which one amino acid is deleted from the carboxyl terminus of each of the two heavy chains thereof, or a functional fragment of the antibody.

**40.** The method of treatment according to any one of claims 37 to 39, wherein the deleted amino acid is lysine.

**41.** The method of treatment according to any one of claims 37 to 40, wherein the anti-CDH6 antibody or the functional fragment of the antibody is an antibody in which a proline residue at the carboxyl terminus of a heavy chain thereof is further amidated, or a functional fragment of the antibody.

**42.** The method of treatment according to any one of claims 32 to 41, wherein the average number of units of the drug-linker structure conjugated per antibody in the anti-CDH6 antibody-drug conjugate is in the range of from 1 to 10.

**43.** The method of treatment according to any one of claims 32 to 41, wherein the average number of units of the drug-linker structure conjugated per antibody in the anti-CDH6 antibody-drug conjugate is in the range of from 7 to 8.

**44.** The method of treatment according to any one of claims 32 to 43, wherein the HIF-2$\alpha$ inhibitor is belzutifan, NKT2152, DFF332, AB521, or BPI-452080, or a pharmaceutically acceptable salt thereof.

**45.** The method of treatment according to any one of claims 32 to 43, wherein the HIF-2$\alpha$ inhibitor is belzutifan or a pharmaceutically acceptable salt thereof.

**46.** The method of treatment according to any one of claims 32 to 45, wherein the anti-CDH6 antibody-drug conjugate and the HIF-2$\alpha$ inhibitor are separately contained in different formulations, and are administered at the same time or at different times.

**47.** The method of treatment according to any one of claims 32 to 45, wherein the anti-CDH6 antibody-drug conjugate and the HIF-2$\alpha$ inhibitor are contained in a single formulation for administration.

**48.** The method of treatment according to any one of claims 32 to 47, wherein the method of treatment is for treating cancer.

**49.** The method of treatment according to any one of claims 32 to 47, wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

**50.** The method of treatment according to any one of claims 32 to 47, wherein the method of treatment is for treating at

least one selected from the group consisting of renal cell carcinoma, renal clear cell carcinoma, and papillary renal cell carcinoma.

**51.** A method of treatment, comprising administering an anti-CDH6 antibody-drug conjugate and a HIF-2α inhibitor in combination to a subject in need of the treatment, wherein the anti-CDH6 antibody-drug conjugate is an antibody-drug conjugate represented by the formula:

[Formula 4]

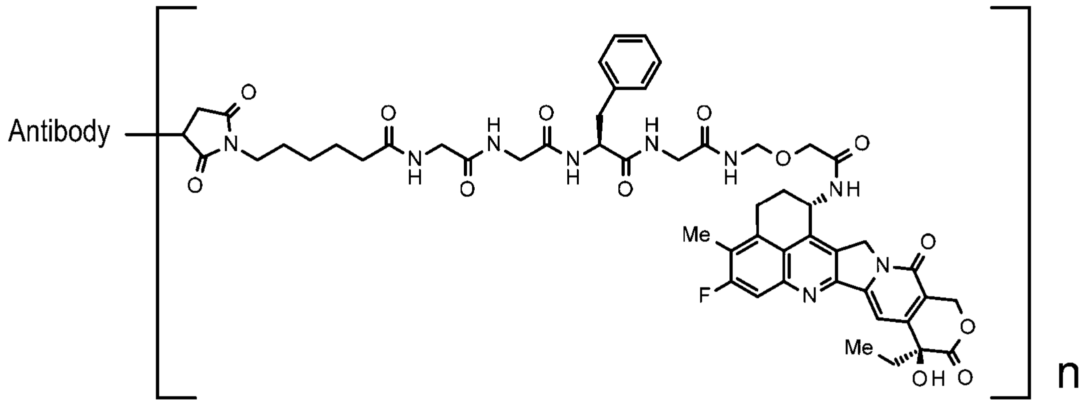

wherein Antibody is an anti-CDH6 antibody, a drug-linker is conjugated to the antibody via a thioether bond, and n represents an average number of units of the drug-linker conjugated per antibody.

**52.** The method of treatment according to claim 51, wherein the anti-CDH6 antibody is an antibody comprising a heavy chain consisting of the amino acid sequence shown in SEQ ID NO: 29 and a light chain consisting of the amino acid sequence shown in SEQ ID NO: 38.

**53.** The method of treatment according to claim 51 or 52, wherein a lysine residue is deleted from the carboxyl terminus of a heavy chain of the anti-CDH6 antibody.

**54.** The method of treatment according to any one of claims 51 to 53, wherein the average number of units of the drug-linker conjugated per antibody in the anti-CDH6 antibody-drug conjugate is in the range of from 7 to 8.

**55.** The method of treatment according to any one of claims 51 to 54, wherein the HIF-2α inhibitor is belzutifan, NKT2152, DFF332, AB521, or BPI-452080, or a pharmaceutically acceptable salt thereof.

**56.** The method of treatment according to any one of claims 51 to 54, wherein the HIF-2α inhibitor is belzutifan or a pharmaceutically acceptable salt thereof.

**57.** The method of treatment according to any one of claims 51 to 56, wherein the anti-CDH6 antibody-drug conjugate and the HIF-2α inhibitor are separately contained in different formulations, and are administered at the same time or at different times.

**58.** The method of treatment according to any one of claims 51 to 56, wherein the anti-CDH6 antibody-drug conjugate and the HIF-2α inhibitor are contained in a single formulation for administration.

**59.** The method of treatment according to any one of claims 51 to 58, wherein the method of treatment is for treating cancer.

**60.** The method of treatment according to any one of claims 51 to 58, wherein the method of treatment is for treating at least one selected from the group consisting of renal cell carcinoma, renal clear cell carcinoma, papillary renal cell carcinoma, ovarian cancer, ovarian serous adenocarcinoma, ovarian clear cell carcinoma, endometrioid ovarian cancer, ovarian mucinous tumor, thyroid cancer, bile duct cancer, lung cancer, non-small cell lung cancer, cervical cancer, brain tumor, head and neck cancer, sarcoma, osteosarcoma, small-cell lung cancer, glioblastoma, mesothelioma, uterine cancer, pancreatic cancer, Wilms' tumor, neuroblastoma, colorectal cancer, stomach cancer, endometrial cancer, uterine body cancer, nasopharyngeal cancer, prostate cancer, and cancer related to von Hippel-Lindau disease.

**61.** The method of treatment according to any one of claims 51 to 58, wherein the method of treatment is for treating at least one selected from the group consisting of renal cell carcinoma, renal clear cell carcinoma, and papillary renal cell carcinoma.

**62.** The method of treatment according to any one of claims 32 to 61, wherein the anti-CDH6 antibody-drug conjugate is raludotatug deruxtecan (DS-6000a).

# Fig. 1

SEQ ID NO: 1: Amino acid sequence of human CDH6 EC3

P Q S T Y Q F K T P E S S P P G T P I G R I K A S D A D V G E N A E I E Y S
I T D G E G L D M F D V I T D Q E T Q E G I I T V K K L L D F E K K K V Y T
L K V E A S N P Y V E P R F L Y L G P F K D S A T V R I V V E D V D E P P V
F

# Fig. 2

SEQ ID NO: 2 Amino acid sequence of anti-CDH6 antibody heavy chain CDRH1 (amino acid sequence of chG019 CDRH1)

G Y T F T R N F M H

# Fig. 3

SEQ ID NO: 3: Amino acid sequence of anti-CDH6 antibody heavy chain CDRH2 (amino acid sequence of chG019 CDRH2)

W I Y P G D G E T E

# Fig. 4

SEQ ID NO: 4: Amino acid sequence of anti-CDH6 antibody heavy chain CDRH3 (amino acid sequence of chG019 CDRH3)

G V Y G G F A G G Y F D F

# Fig. 5

SEQ ID NO: 5: Amino acid sequence of anti-CDH6 antibody light chain CDRL1 (amino acid sequence of chG019 CDRL1)

K A S Q N I Y K N L A

# Fig. 6

SEQ ID NO: 6: Amino acid sequence of anti-CDH6 antibody light chain CDRL2 (amino acid sequence of chG019 CDRL2)

D A N T L Q T

# Fig. 7

SEQ ID NO: 7: Amino acid sequence of anti-CDH6 antibody light chain CDRL3 (amino acid sequence of chG019 CDRL3)

Q Q Y Y S G W A

# Fig. 8

SEQ ID NO: 27: Amino acid sequence of anti-CDH6 antibody heavy chain (full-length amino acid sequence of hH01 heavy chain)

M K H L W F F L L L V A A P R W V L S E V Q L V Q S G A E V K K P G A S V K
V S C K A S G Y T F T R N F M H W V R Q A P G Q G L E W M G W I Y P G D G E
T E Y A Q K F Q G R V T I T A D T S T S T A Y M E L S S L R S E D T A V Y Y
C A R G V Y G G F A G G Y F D F W G Q G T L V T V S S A S T K G P S V F P L
A P S S K S T S G G T A A L G C L V K D Y F P E P V T V S W N S G A L T S G
V H T F P A V L Q S S G L Y S L S S V V T V P S S S L G T Q T Y I C N V N H
K P S N T K V D K R V E P K S C D K T H T C P P C P A P E L L G G P S V F L
F P P K P K D T L M I S R T P E V T C V V V D V S H E D P E V K F N W Y V D
G V E V H N A K T K P R E E Q Y N S T Y R V V S V L T V L H Q D W L N G K E
Y K C K V S N K A L P A P I E K T I S K A K G Q P R E P Q V Y T L P P S R E
E M T K N Q V S L T C L V K G F Y P S D I A V E W E S N G Q P E N N Y K T T
P P V L D S D G S F F L Y S K L T V D K S R W Q Q G N V F S C S V M H E A L
H N H Y T Q K S L S L S P G K

| Signal sequence (1-19) | variable region (20-141) | constant region (142-471) |
| --- | --- | --- |

# Fig. 9

SEQ ID NO: 28: Amino acid sequence of anti-CDH6 antibody heavy chain variable region (amino acid sequence of hH01 heavy chain variable region)

E V Q L V Q S G A E V K K P G A S V K V S C K A S G Y T F T R N F M H W V R
Q A P G Q G L E W M G W I Y P G D G E T E Y A Q K F Q G R V T I T A D T S T
S T A Y M E L S S L R S E D T A V Y Y C A R G V Y G G F A G G Y F D F W G Q
G T L V T V S S

# Fig. 10

SEQ ID NO: 29: Amino acid sequence of mature anti-CDH6 antibody heavy chain (except for signal sequence) (full-length amino acid sequence of hH01 heavy chain except for signal sequence)

E V Q L V Q S G A E V K K P G A S V K V S C K A S G Y T F T R N F M H W V R
Q A P G Q G L E W M G W I Y P G D G E T E Y A Q K F Q G R V T I T A D T S T
S T A Y M E L S S L R S E D T A V Y Y C A R G V Y G G F A G G Y F D F W G Q
G T L V T V S S A S T K G P S V F P L A P S S K S T S G G T A A L G C L V K
D Y F P E P V T V S W N S G A L T S G V H T F P A V L Q S S G L Y S L S S V
V T V P S S S L G T Q T Y I C N V N H K P S N T K V D K R V E P K S C D K T
H T C P P C P A P E L L G G P S V F L F P P K P K D T L M I S R T P E V T C
V V V D V S H E D P E V K F N W Y V D G V E V H N A K T K P R E E Q Y N S T
Y R V V S V L T V L H Q D W L N G K E Y K C K V S N K A L P A P I E K T I S
K A K G Q P R E P Q V Y T L P P S R E E M T K N Q V S L T C L V K G F Y P S
D I A V E W E S N G Q P E N N Y K T T P P V L D S D G S F F L Y S K L T V D
K S R W Q Q G N V F S C S V M H E A L H N H Y T Q K S L S L S P G K

# Fig. 11

SEQ ID NO: 36: Amino acid sequence of anti-CDH6 antibody light chain (full-length amino acid sequence of hL02 light chain)

M V L Q T Q V F I S L L L W I S G A Y G D I Q M T Q S P S S L S A S V G D R
V T I T C K A S Q N I Y K N L A W Y Q Q K P G K A P K L L I Y D A N T L Q T
G V P S R F S G S G S G S D F T L T I S S L Q P E D F A T Y F C Q Q Y Y S G
W A F G Q G T K V E I K R T V A A P S V F I F P P S D E Q L K S G T A S V V
C L L N N F Y P R E A K V Q W K V D N A L Q S G N S Q E S V T E Q D S K D S
T Y S L S S T L T L S K A D Y E K H K V Y A C E V T H Q G L S S P V T K S F
N R G E C

| constant region (1-20) | variable region (21-128) | Signal sequence (129-233) |
|---|---|---|

# Fig. 12

SEQ ID NO: 37: Amino acid sequence of anti-CDH6 antibody light chain variable region (amino acid sequence of hL02 light chain variable region)

D I Q M T Q S P S S L S A S V G D R V T I T C K A S Q N I Y K N L A W Y Q Q
K P G K A P K L L I Y D A N T L Q T G V P S R F S G S G S G S D F T L T I S
S L Q P E D F A T Y F C Q Q Y Y S G W A F G Q G T K V E I K R T

# Fig. 13

SEQ ID NO: 38: Amino acid sequence of mature anti-CDH6 antibody light chain (except for signal sequence) (full-length amino acid sequence of hL02 light chain except for signal sequence)

D I Q M T Q S P S S L S A S V G D R V T I T C K A S Q N I Y K N L A W Y Q Q
K P G K A P K L L I Y D A N T L Q T G V P S R F S G S G S G S D F T L T I S
S L Q P E D F A T Y F C Q Q Y Y S G W A F G Q G T K V E I K R T V A A P S V
F I F P P S D E Q L K S G T A S V V C L L N N F Y P R E A K V Q W K V D N A
L Q S G N S Q E S V T E Q D S K D S T Y S L S S T L T L S K A D Y E K H K V
Y A C E V T H Q G L S S P V T K S F N R G E C

Fig. 14

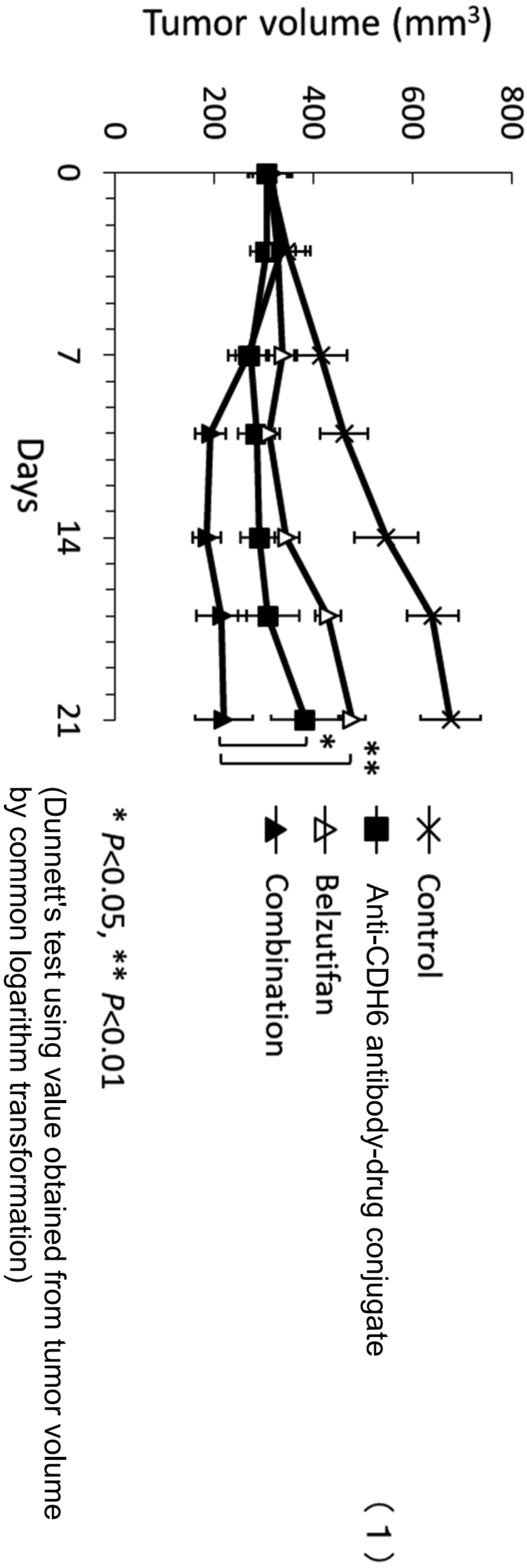
Tumor volume (mm3)
0
200
400
600
800
0
7
14
21
Days
*
**
Control
Anti-CDH6 antibody-drug conjugate (1)
Belzutifan
Combination
* P<0.05, ** P<0.01
(Dunnett's test using value obtained from tumor volume by common logarithm transformation)

Fig. 15

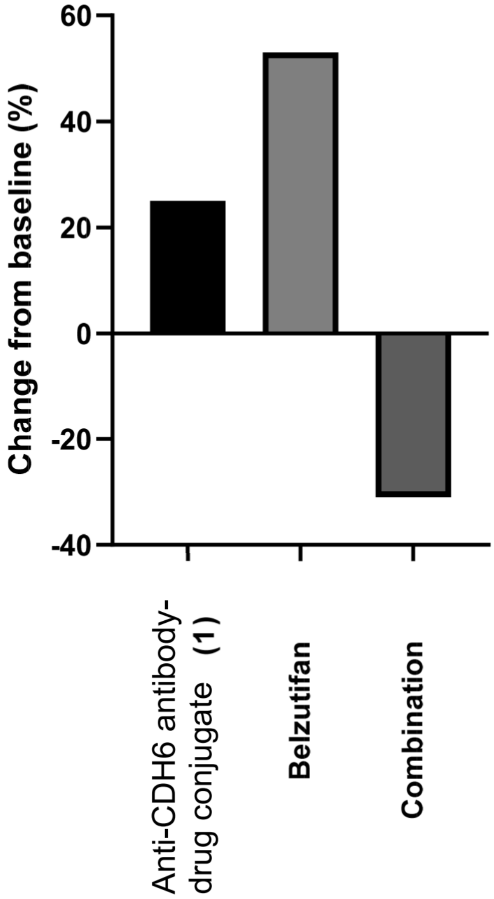
Change from baseline (%)
60
40
20
0
-20
-40
Anti-CDH6 antibody-drug conjugate (1)
Belzutifan
Combination

**INTERNATIONAL SEARCH REPORT**

International application No.
**PCT/JP2024/012972**

**A. CLASSIFICATION OF SUBJECT MATTER**

***A61K 47/68***(2017.01)i; ***A61K 31/277***(2006.01)i; ***A61K 31/4745***(2006.01)i; ***A61K 39/395***(2006.01)i; ***A61K 45/00***(2006.01)i; ***A61P 1/00***(2006.01)i; ***A61P 1/16***(2006.01)i; ***A61P 1/18***(2006.01)i; ***A61P 5/14***(2006.01)i; ***A61P 11/00***(2006.01)i; ***A61P 13/08***(2006.01)i; ***A61P 13/12***(2006.01)i; ***A61P 15/00***(2006.01)i; ***A61P 17/00***(2006.01)i; ***A61P 19/08***(2006.01)i; ***A61P 25/00***(2006.01)i; ***A61P 35/00***(2006.01)i; ***C07K 1/107***(2006.01)i; ***C07K 7/06***(2006.01)i; ***C07K 16/28***(2006.01)i; ***C12N 15/13***(2006.01)i

FI: A61K47/68; A61P17/00; A61K31/277; A61P35/00; A61P13/12; A61P15/00; A61P5/14; A61P1/16; A61P11/00; A61P25/00; A61P19/08; A61K45/00; A61P1/18; A61P1/00; A61P13/08; C07K16/28; C07K1/107; C07K7/06; C12N15/13; A61K39/395 D ZNA; A61K39/395 N; A61K31/4745

According to International Patent Classification (IPC) or to both national classification and IPC

**B. FIELDS SEARCHED**

Minimum documentation searched (classification system followed by classification symbols)

A61K47/68; A61K31/277; A61K31/4745; A61K39/395; A61K45/00; A61P1/00; A61P1/16; A61P1/18; A61P5/14; A61P11/00; A61P13/08; A61P13/12; A61P15/00; A61P17/00; A61P19/08; A61P25/00; A61P35/00; C07K1/107; C07K7/06; C07K16/28; C12N15/13

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2020/130125 A1 (DAIICHI SANKYO COMPANY, LIMITED) 25 June 2020 (2020-06-25)<br>claims, examples | 1-62 |
| Y | WO 2020/122034 A1 (DAIICHI SANKYO COMPANY, LIMITED) 18 June 2020 (2020-06-18)<br>claims, examples | 1-62 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No. **PCT/JP2024/012972**

**C. DOCUMENTS CONSIDERED TO BE RELEVANT**

| Category* | Citation of document, with indication, where appropriate, of the relevant passages | Relevant to claim No. |
|---|---|---|
| Y | WO 2020/031936 A1 (DAIICHI SANKYO COMPANY, LIMITED) 13 February 2020 (2020-02-13) claims, examples | 1-62 |
| Y | WO 2018/212136 A1 (DAIICHI SANKYO COMPANY, LIMITED) 22 November 2018 (2018-11-22) claims, examples | 1-62 |
| Y | WO 2021/262563 A1 (MERCK SHARP & DOHME CORP.) 30 December 2021 (2021-12-30) claims, examples | 1-62 |
| Y | WO 2020/097336 A1 (BETH ISRAEL DEACONESS MEDICAL CENTER) 14 May 2020 (2020-05-14) claims | 1-62 |

Form PCT/ISA/210 (second sheet) (July 2022)

**INTERNATIONAL SEARCH REPORT**

International application No.
**PCT/JP2024/012972**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/012972**

| Patent document cited in search report | Publication date (day/month/year) | Patent family member(s) | Publication date (day/month/year) |
|---|---|---|---|
| WO 2020/130125 A1 | 25 June 2020 | US 2022/0040324 A1<br>claims, examples<br>EP 3903828 A1<br>CN 113195000 A<br>KR 10-2021-0107069 A | |
| WO 2020/122034 A1 | 18 June 2020 | US 2022/0023436 A1<br>claims, examples<br>EP 3909580 A1<br>CN 113271942 A<br>KR 10-2021-0102341 A | |
| WO 2020/031936 A1 | 13 February 2020 | US 2021/0290775 A1<br>claims, examples<br>EP 3834843 A1<br>CN 112512587 A<br>KR 10-2021-0042120 A | |
| WO 2018/212136 A1 | 22 November 2018 | US 2020/0171163 A1<br>claims, examples<br>EP 3626825 A1<br>CN 110651045 A<br>KR 10-2020-0006061 A | |
| WO 2021/262563 A1 | 30 December 2021 | JP 2023-531675 A<br>US 2023/0210838 A1<br>EP 4168008 A1<br>KR 10-2023-0026493 A<br>CN 115803629 A | |
| WO 2020/097336 A1 | 14 May 2020 | JP 2022-507294 A<br>US 2022/0119545 A1<br>EP 3876988 A1<br>CN 113382750 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018212136 A
- WO 2023102875 A
- WO 2023104188 A
- WO 9007861 A
- US 5821337 A
- WO 9954342 A
- WO 0061739 A
- WO 0231140 A
- WO 2007133855 A
- WO 2013120066 A
- WO 2014057687 A
- WO 2015098099 A
- WO 2015115091 A
- WO 2015155998 A
- WO 2019044947 A
- WO 2018135501 A


### Non-patent literature cited in the description


- **KEITH et al.** *Nat. Rev. Cancer.*, 2011, vol. 12 (1), 9-22
- **MARTINEZ-SAEZ et al.** *Crit Rev Oncol Hematol.*, 2017, vol. 111, 117-123
- **XU et al.** *J. Med. Chem.*, 2019, vol. 62 (15), 6876-6893
- **SHIMOYAMA Y et al.** *Cancer Research*, 15 May 1995, vol. 55, 2206-2211
- **OGITANI Y. et al.** *Clinical Cancer Research*, 29 March 2016, vol. 22 (20), 5097-5108
- **SUZUKI H et al.** *Molecular Cancer Therapeutics*, 2024, vol. 23 (3), 257-271
- *Cell Death and Differentiation*, 2008, vol. 15, 751-761
- *Molecular Biology of the Cell*, December 2004, vol. 15, 5268-5282
- *Bio Techniques*, January 2000, vol. 28, 162-165
- **KOHLER** ; **MILSTEIN**. *Nature*, 1975, vol. 256, 495-497
- Monoclonal Antibodies. Plenum Press, 1980, 365-367
- *Proc. Natl. Acad. Sci. U.S.A.*, 1984, vol. 81, 6851-6855
- *Nature*, 1986, vol. 321, 522-525
- **TOMIZUKA, K. et al.** *Nature Genetics*, 1997, vol. 16, 133-143
- **KUROIWA, Y. et al.** *Nucl. Acids Res.*, 1998, vol. 26, 3447-3448
- **YOSHIDA, H. et al.** Animal Cell Technology: Basic and Applied Aspects. Kluwer Academic Publishers, 1999, vol. 10, 69-73
- **TOMIZUKA, K. et al.** *Proc. Natl. Acad. Sci. USA*, 2000, vol. 97, 722-727
- **WORMSTONE, I. M. et al.** *Investigative Ophthalmology & Visual Science.*, 2002, vol. 43 (7), 2301-2308
- **CARMEN, S. et al.** *Briefings in Functional Genomics and Proteomics*, 2002, vol. 1 (2), 189-203
- **SIRIWARDENA, D. et al.** *Ophthalmology*, 2002, vol. 109 (3), 427-431
- **LARKIN MA** ; **BLACKSHIELDS G** ; **BROWN NP** ; **CHENNA R** ; **MCGETTIGAN PA** ; **MCWILLIAM H** ; **VALENTIN F** ; **WALLACE IM** ; **WILM A** ; **LOPEZ R**. Clustal W and Clustal X version 2.0. *Bioinformatics.*, 2007, vol. 23 (21), 2947-2948
- *Journal of Chromatography A*, 1995, vol. 705, 129-134
- *Analytical Biochemistry*, 2007, vol. 360, 75-83
- **HERMANSON, G.T**. Bioconjugate Techniques. Academic Press, 1996, 456-493
- Patients With Advanced Clear Cell Renal Cell Carcinoma. National Library of Medicine (National Institutes of Health), 15 November 2021
- Advanced/Relapsed Renal Cancer & Other Malignancies. National Library of Medicine (National Institutes of Health), 20 May 2021
- Renal Cell Carcinoma and Other Solid Tumors (ARC-20). National Library of Medicine (National Institutes of Health, 10 September 2022
- **WANG et al.** *Cancer Res*, 2023, vol. 83 (7), 494
- *J. Med. Chem.*, 2019, vol. 62, 6876-6893